(19) Europäisches Patentamt
European Patent Office
Office européen des brevets

(11) EP 4 485 474 B1

(12) EUROPÄISCHE PATENTSCHRIFT

(45) Veröffentlichungstag und Bekanntmachung des Hinweises auf die Patenterteilung:
**08.04.2026 Patentblatt 2026/15**

(21) Anmeldenummer: **24178988.2**

(22) Anmeldetag: **30.05.2024**

(51) Internationale Patentklassifikation (IPC):
***G16H 30/40*** *(2018.01)* ***A61B 6/00*** *(2024.01)*
***G06N 3/045*** *(2023.01)* ***A61B 6/50*** *(2024.01)*
***G06N 3/088*** *(2023.01)* ***G06N 3/09*** *(2023.01)*
***G06N 3/096*** *(2023.01)* ***A61B 8/08*** *(2006.01)*
***A61K 49/10*** *(2006.01)* ***A61B 8/00*** *(2006.01)*
***G06V 10/774*** *(2022.01)* ***G06V 10/82*** *(2022.01)*
***G06N 3/0464*** *(2023.01)* ***G06N 3/0475*** *(2023.01)*
***G06N 3/094*** *(2023.01)* *G16H 50/70 (2018.01)*
*A61B 5/055 (2006.01)* *A61B 6/03 (2006.01)*

(52) Gemeinsame Patentklassifikation (CPC):
**G16H 30/40; A61B 6/481; A61K 49/105; A61K 49/106; A61K 49/108; G06N 3/045; G06N 3/088; G06N 3/09; G06N 3/096; G06V 10/774; G06V 10/82;** A61B 5/055; A61B 6/032; A61B 8/481; G06N 3/0464; (Forts.)

(54) **ERZEUGEN VON KÜNSTLICHEN KONTRASTVERSTÄRKTEN RADIOLOGISCHEN AUFNAHMEN**

GENERATION OF ARTIFICIAL CONTRAST ENHANCED RADIOLOGICAL RECORDINGS

GÉNÉRATION D’ENREGISTREMENTS RADIOLOGIQUES ARTIFICIELS À CONTRASTE AMÉLIORÉ

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **05.06.2023 EP 23177301**
**13.07.2023 EP 23185296**

(43) Veröffentlichungstag der Anmeldung:
**01.01.2025 Patentblatt 2025/01**

(73) Patentinhaber: **Bayer Aktiengesellschaft**
**51373 Leverkusen (DE)**

(72) Erfinder:
- **Lenga, Matthias**
  **51373 Leverkusen (DE)**
- **Baltruschat, Ivo Matteo**
  **51373 Leverkusen (DE)**
- **Janbakhshi, Parvaneh**
  **51373 Leverkusen (DE)**
- **Kreis, Felix Karl**
  **51373 Leverkusen (DE)**

(74) Vertreter: **BIP Patents**
**c/o Bayer Intellectual Property GmbH**
**Alfred-Nobel-Straße 50**
**40789 Monheim am Rhein (DE)**

(56) Entgegenhaltungen:
**EP-A1- 4 044 109** **WO-A1-2016/193190**
**WO-A1-2020/030618** **WO-A1-2022/194777**
**WO-A1-2023/062196** **WO-A1-2023/062202**
**US-A1- 2019 108 634** **US-A1- 2021 241 458**

EP 4 485 474 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
G06N 3/0475; G06N 3/094; G06V 2201/03;
G16H 50/70

**Beschreibung**

TECHNISCHES GEBIET

Die vorliegende Offenbarung befasst sich mit dem technischen Gebiet der Erzeugung von künstlichen kontrastverstärkten radiologischen Aufnahmen.

EINLEITUNG

WO2019/074938A1 offenbart ein Verfahren zur Reduzierung der Menge an Kontrastmittel bei der Erzeugung von radiologischen Aufnahmen mit Hilfe eines künstlichen neuronalen Netzes.

In dem offenbarten Verfahren wird in einem ersten Schritt ein Trainingsdatensatz erzeugt. Der Trainingsdatensatz umfasst für jede Person einer Vielzahl an Personen i) eine native radiologische Aufnahme (*zero-contrast image*), ii) eine radiologische Aufnahme nach der Applikation einer geringen Menge an Kontrastmittel (*low-contrast image*) und iii) eine radiologische Aufnahme nach der Applikation einer Standardmenge an Kontrastmittel (*full-contrast image*). Die Standardmenge ist die vom Hersteller und/oder Vertreiber des Kontrastmittels empfohlene und/oder die von einer Zulassungsbehörde zugelassene und/oder die in einem Beipackzettel zum Kontrastmittel aufgeführte Menge.

In einem zweiten Schritt wird ein künstliches neuronales Netz trainiert, für jede Person des Trainingsdatensatzes auf Basis der nativen Aufnahme und der Aufnahme nach Applikation einer geringeren Menge an Kontrastmittel als der Standardmenge eine künstliche radiologische Aufnahme vorherzusagen, die einen Aufnahmebereich nach der Applikation der Standardmenge an Kontrastmittel zeigt. Die gemessene radiologische Aufnahme nach der Applikation einer Standardmenge an Kontrastmittel dient beim Training jeweils als Referenz (*ground truth*).

In einem dritten Schritt kann das trainierte künstliche neuronale Netz verwendet werden, für eine neue Person auf Basis einer nativen Aufnahme und einer radiologischen Aufnahme nach der Applikation einer geringeren Menge an Kontrastmittel als der Standardmenge eine künstliche radiologische Aufnahme vorherzusagen, die den aufgenommenen Bereich so zeigt wie er aussehen würde, wenn die Standardmenge an Kontrastmittel appliziert worden wäre.

Das in WO2019/074938A1 offenbarte Verfahren weist Nachteile auf.

Das in WO2019/074938A1 beschriebene künstliche neuronale Netz ist eine Blackbox, d.h., es ist nicht nachvollziehbar, was das künstliche neuronale Netz genau lernt, wenn es trainiert wird. Es ist unklar, inwieweit das künstliche neuronale Netz Vorhersagen auf Basis von Daten treffen kann, die nicht beim Trainieren verwendet wurden. Natürlich ist es möglich, anhand von weiteren Daten eine Validierung des künstlichen neuronalen Netzes durchzuführen. Zum einen müssen solche Validierungsdaten neben den Trainingsdaten aber beschafft (erzeugt) werden, zum anderen werden verfügbare Validierungsdaten nicht alle Situationen, die bei einer späteren Nutzung des trainierten künstlichen neuronalen Netzes zur Vorhersage eintreten können, abdecken können. Das bedeutet, dass stets eine Unsicherheit verbleibt, ob das trainierte künstliche neuronale Netz für alle eingegebenen Daten sinnvolle/korrekte Vorhersagen erzeugen kann.

In vielen Ländern ist eine Genehmigung erforderlich, um ein trainiertes künstliches neuronales Netz, wie es in WO2019/074938A1 beschrieben ist, zur Diagnose von Krankheiten bei menschlichen Patienten einzusetzen. Der beschriebene Umstand, dass ein trainiertes künstliches neuronales Netz eine Blackbox darstellt, bei der eine Unsicherheit besteht, ob das trainierte künstliche neuronale Netz stets sinnvolle/korrekte Vorhersagen erzeugt, erschwert den Genehmigungsprozess.

Die Patentanmeldung US2019/0108634A1 offenbart ein Verfahren, bei dem aus niedrig dosierten Scans mittels eines Deep-Learning-Ansatzes voll-dosierte Bildvolumina erzeugt werden, wodurch die Kontrastmittelbelastung für Patienten reduziert wird. Hierzu werden die Bilder zunächst vorverarbeitet, unter anderem durch Ko-Registrierung, Normalisierung und optionales Maskieren relevanter Regionen, um standardisierte Bildausschnitte für das Training eines neuronalen Netzes zu erstellen. Das Netzwerk, typischerweise ein Encoder-Decoder-CNN mit Residualverbindungen, lernt, das Rauschen zu reduzieren und das Kontrastsignal vorherzusagen; während des Trainings werden die Vollkontrastbilder als Referenz genutzt, und die Methode ist auf unterschiedliche Bildgebungsmodalitäten und Netzwerkarchitekturen anpassbar.

ZUSAMMENFASSUNG

Diesem und weiteren Problemen widmet sich die vorliegende Offenbarung.

Ein erster Gegenstand der vorliegenden Offenbarung ist ein computer-implementiertes Verfahren zum Erzeugen einer synthetischen kontrastverstärkten radiologischen Aufnahme, umfassend die Schritte:

- Bereitstellen eines trainierten Modells des maschinellen Lernens,

  ◦ wobei das trainierte Modell des maschinellen Lernens anhand von Trainingsdaten trainiert wurde,

  ◦ wobei die Trainingsdaten für jedes Referenzobjekt einer Vielzahl an Referenzobjekten (i) eine erste Referenz-Repräsentation eines Referenzbereichs des Referenzobjekts und eine zweite Referenz-Repräsentation des Referenzbereichs des Referenzobjekts als Eingabedaten und (ii) eine dritte Referenz-Repräsentation des

Referenzbereichs des Referenzobjekts als Zieldaten umfassen,

◦ wobei die erste Referenz-Repräsentation den Referenzbereich ohne Kontrastmittel oder nach Applikation einer ersten Menge eines Kontrastmittels repräsentiert,

◦ wobei die zweite Referenz-Repräsentation den Referenzbereich nach Applikation einer zweiten Menge des Kontrastmittels repräsentiert, wobei die zweite Menge größer als die erste Menge ist,

◦ wobei die dritte Referenz-Repräsentation den Referenzbereich nach Applikation einer dritten Menge des Kontrastmittels repräsentiert, wobei die dritte Menge verschieden von der zweiten Menge, vorzugsweise größer als die zweite Menge ist,

◦ wobei das trainierte Modell des maschinellen Lernens zwei Teilmodelle umfasst: ein erstes Teilmodell und ein zweites Teilmodell, wobei das erste Teilmodell konfiguriert ist und trainiert wurde, auf Basis der ersten Referenz-Repräsentation und der zweiten Referenz-Repräsentation eines Referenzobjekts mindestens einen Modellparameter für das zweite Teilmodell zu ermitteln, wobei das zweite Teilmodell konfiguriert ist, auf Basis der ersten Referenz-Repräsentation und der zweiten Referenz-Repräsentation und des von dem ersten Teilmodell ermittelten mindestens einen Modellparameters eine synthetische dritte Referenz-Repräsentation zu erzeugen,

- Bereitstellen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
- Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation und der zweiten Repräsentation dem trainierten Modell des maschinellen Lernens,
- Empfangen einer synthetischen dritten Repräsentation des Untersuchungsbereichs des Untersuchungsobjekts von dem trainierten Modell des maschinellen Lernens, wobei die synthetische dritte Repräsentation den Referenzbereich nach Applikation einer dritten Menge des Kontrastmittels repräsentiert, wobei die dritte Menge verschieden von der zweiten Menge, vorzugsweise größer als die zweite Menge ist,
- Ausgeben und/oder Speichern der synthetischen dritten Repräsentation und/oder Übermitteln der synthetischen dritten Repräsentation an ein separates Computersystem

wobei das zweite Teilmodell konfiguriert ist,

- die erste Repräsentation des Untersuchungsbereichs des Untersuchungsobjekts von der zweiten Repräsentation des Untersuchungsbereichs des Untersuchungsobjekts zu subtrahieren, wobei eine Differenz gebildet wird,
- die Differenz mit einem Verstärkungsfaktor zu multiplizieren, wobei der Verstärkungsfaktor eine positive oder negative reelle Zahl ist, wobei der mindestens eine von dem ersten Teilmodell ermittelte Modellparameter den Verstärkungsfaktor umfasst,
- die mit dem Verstärkungsfaktor multiplizierte Differenz auf die erste Repräsentation oder die zweite Repräsentation zu addieren.

Ein weiterer Gegenstand der vorliegenden Offenbarung ist ein Computersystem umfassend:

einen Prozessor; und

einen Speicher, der ein Computerprogramm speichert, das so konfiguriert ist, dass es, wenn es vom Prozessor ausgeführt wird, eine Operation durchführt, wobei die Operation umfasst:

- Bereitstellen eines trainierten Modells des maschinellen Lernens,

  ◦ wobei das trainierte Modell des maschinellen Lernens anhand von Trainingsdaten trainiert wurde,

  ◦ wobei die Trainingsdaten für jedes Referenzobjekt einer Vielzahl an Referenzobjekten (i) eine erste Referenz-Repräsentation eines Referenzbereichs des Referenzobjekts und eine zweite Referenz-Repräsentation des Referenzbereichs des Referenzobjekts als Eingabedaten und (ii) eine dritte Referenz-Repräsentation des Referenzbereichs des Referenzobjekts als Zieldaten umfassen,

  ◦ wobei die erste Referenz-Repräsentation

den Referenzbereich ohne Kontrastmittel oder nach Applikation einer ersten Menge eines Kontrastmittels repräsentiert,

◦ wobei die zweite Referenz-Repräsentation den Referenzbereich nach Applikation einer zweiten Menge des Kontrastmittels repräsentiert, wobei die zweite Menge größer als die erste Menge ist,

◦ wobei die dritte Referenz-Repräsentation den Referenzbereich nach Applikation einer dritten Menge des Kontrastmittels repräsentiert, wobei die dritte Menge verschieden von der zweiten Menge, vorzugsweise größer als die zweite Menge ist,

◦ wobei das trainierte Modell des maschinellen Lernens zwei Teilmodelle umfasst: ein erstes Teilmodell und ein zweites Teilmodell, wobei das erste Teilmodell konfiguriert ist und trainiert wurde, auf Basis der ersten Referenz-Repräsentation und der zweiten Referenz-Repräsentation eines Referenzobjekts mindestens einen Modellparameter für das zweite Teilmodell zu ermitteln, wobei das zweite Teilmodell konfiguriert ist, auf Basis der ersten Referenz-Repräsentation und der zweiten Referenz-Repräsentation und des von dem ersten Teilmodell ermittelten mindestens einen Modellparameters eine synthetische dritte Referenz-Repräsentation zu erzeugen,

- Bereitstellen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
- Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation und der zweiten Repräsentation dem trainierten Modell des maschinellen Lernens,
- Empfangen einer synthetischen dritten Repräsentation des Untersuchungsbereichs des Untersuchungsobjekts von dem trainierten Modell des maschinellen Lernens, wobei die synthetische dritte Repräsentation den Referenzbereich nach Applikation einer dritten Menge des Kontrastmittels repräsentiert, wobei die dritte Menge verschieden von der zweiten Menge, vorzugsweise größer als die zweite Menge ist,
- Ausgeben und/oder Speichern der synthetischen dritten Repräsentation und/oder Übermitteln der synthetischen dritten Repräsentation an ein separates Computersystem

wobei das zweite Teilmodell konfiguriert ist,

- die erste Repräsentation des Untersuchungsbereichs des Untersuchungsobjekts von der zweiten Repräsentation des Untersuchungsbereichs des Untersuchungsobjekts zu subtrahieren, wobei eine Differenz gebildet wird,
- die Differenz mit einem Verstärkungsfaktor zu multiplizieren, wobei der Verstärkungsfaktor eine positive oder negative reelle Zahl ist, wobei der mindestens eine von dem ersten Teilmodell ermittelte Modellparameter den Verstärkungsfaktor umfasst,
- die mit dem Verstärkungsfaktor multiplizierte Differenz auf die erste Repräsentation oder die zweite Repräsentation zu addieren.

Ein weiterer Gegenstand der vorliegenden Offenbarung ist ein Computerprogramm, das in einen Arbeitsspeicher eines Computersystems geladen werden kann und dort das Computersystem dazu veranlasst, folgende Schritte ausführen:

- Bereitstellen eines trainierten Modells des maschinellen Lernens,

  ◦ wobei das trainierte Modell des maschinellen Lernens anhand von Trainingsdaten trainiert wurde,

  ◦ wobei die Trainingsdaten für jedes Referenzobjekt einer Vielzahl an Referenzobjekten (i) eine erste Referenz-Repräsentation eines Referenzbereichs des Referenzobjekts und eine zweite Referenz-Repräsentation des Referenzbereichs des Referenzobjekts als Eingabedaten und (ii) eine dritte Referenz-Repräsentation des Referenzbereichs des Referenzobjekts als Zieldaten umfassen,

  ◦ wobei die erste Referenz-Repräsentation den Referenzbereich ohne Kontrastmittel oder nach Applikation einer ersten Menge eines Kontrastmittels repräsentiert,

  ◦ wobei die zweite Referenz-Repräsentation den Referenzbereich nach Applikation einer

zweiten Menge des Kontrastmittels repräsentiert, wobei die zweite Menge größer als die erste Menge ist,

◦ wobei die dritte Referenz-Repräsentation den Referenzbereich nach Applikation einer dritten Menge des Kontrastmittels repräsentiert, wobei die dritte Menge verschieden von der zweiten Menge, vorzugsweise größer als die zweite Menge ist,

◦ wobei das trainierte Modell des maschinellen Lernens zwei Teilmodelle umfasst: ein erstes Teilmodell und ein zweites Teilmodell, wobei das erste Teilmodell konfiguriert ist und trainiert wurde, auf Basis der ersten Referenz-Repräsentation und der zweiten Referenz-Repräsentation eines Referenzobjekts mindestens einen Modellparameter für das zweite Teilmodell zu ermitteln, wobei das zweite Teilmodell konfiguriert ist, auf Basis der ersten Referenz-Repräsentation und der zweiten Referenz-Repräsentation und des von dem ersten Teilmodell ermittelten mindestens einen Modellparameters eine synthetische dritte Referenz-Repräsentation zu erzeugen,

- Bereitstellen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,

- Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,

- Zuführen der ersten Repräsentation und der zweiten Repräsentation dem trainierten Modell des maschinellen Lernens,

- Empfangen einer synthetischen dritten Repräsentation des Untersuchungsbereichs des Untersuchungsobjekts von dem trainierten Modell des maschinellen Lernens, wobei die synthetische dritte Repräsentation den Referenzbereich nach Applikation einer dritten Menge des Kontrastmittels repräsentiert, wobei die dritte Menge verschieden von der zweiten Menge, vorzugsweise größer als die zweite Menge ist,

- Ausgeben und/oder Speichern der synthetischen dritten Repräsentation und/oder Übermitteln der synthetischen dritten Repräsentation an ein separates Computersystem

wobei das zweite Teilmodell konfiguriert ist,

- die erste Repräsentation des Untersuchungsbereichs des Untersuchungsobjekts von der zweiten Repräsentation des Untersuchungsbereichs des Untersuchungsobjekts zu subtrahieren, wobei eine Differenz gebildet wird,

- die Differenz mit einem Verstärkungsfaktor zu multiplizieren, wobei der Verstärkungsfaktor eine positive oder negative reelle Zahl ist, wobei der mindestens eine von dem ersten Teilmodell ermittelte Modellparameter den Verstärkungsfaktor umfasst,

- die mit dem Verstärkungsfaktor multiplizierte Differenz auf die erste Repräsentation oder die zweite Repräsentation zu addieren.

Ein weiterer Gegenstand der vorliegenden Offenbarung ist eine Verwendung eines Kontrastmittels in einem radiologischen Untersuchungsverfahren, wobei das radiologische Untersuchungsverfahren umfasst:

- Bereitstellen eines trainierten Modells des maschinellen Lernens,

  ◦ wobei das trainierte Modell des maschinellen Lernens anhand von Trainingsdaten trainiert wurde,

  ◦ wobei die Trainingsdaten für jedes Referenzobjekt einer Vielzahl an Referenzobjekten (i) eine erste Referenz-Repräsentation eines Referenzbereichs des Referenzobjekts und eine zweite Referenz-Repräsentation des Referenzbereichs des Referenzobjekts als Eingabedaten und (ii) eine dritte Referenz-Repräsentation des Referenzbereichs des Referenzobjekts als Zieldaten umfassen,

  ◦ wobei die erste Referenz-Repräsentation den Referenzbereich ohne Kontrastmittel oder nach Applikation einer ersten Menge des Kontrastmittels repräsentiert,

  ◦ wobei die zweite Referenz-Repräsentation den Referenzbereich nach Applikation einer zweiten Menge des Kontrastmittels repräsentiert, wobei die zweite Menge größer als die erste Menge ist,

  ◦ wobei die dritte Referenz-Repräsentation den Referenzbereich nach Applikation einer dritten Menge des Kontrastmittels repräsentiert, wobei die dritte Menge verschieden von der zweiten Menge, vorzugsweise größer als die zweite Menge ist,

◦ wobei das trainierte Modell des maschinellen Lernens zwei Teilmodelle umfasst: ein erstes Teilmodell und ein zweites Teilmodell, wobei das erste Teilmodell konfiguriert ist und trainiert wurde, auf Basis der ersten Referenz-Repräsentation und der zweiten Referenz-Repräsentation eines Referenzobjekts mindestens einen Modellparameter für das zweite Teilmodell zu ermitteln, wobei das zweite Teilmodell konfiguriert ist, auf Basis der ersten Referenz-Repräsentation und der zweiten Referenz-Repräsentation und des von dem ersten Teilmodell ermittelten mindestens einen Modellparameters eine synthetische dritte Referenz-Repräsentation zu erzeugen,

- Bereitstellen einer ersten Repräsentation, wobei die erste Repräsentation einen Untersuchungsbereich eines Untersuchungsobjekts ohne Kontrastmittel oder nach Applikation einer ersten Menge des Kontrastmittels repräsentiert,
- Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation und der zweiten Repräsentation dem trainierten Modell des maschinellen Lernens,
- Empfangen einer synthetischen dritten Repräsentation des Untersuchungsbereichs des Untersuchungsobjekts von dem trainierten Modell des maschinellen Lernens, wobei die synthetische dritte Repräsentation den Referenzbereich nach Applikation einer dritten Menge des Kontrastmittels repräsentiert, wobei die dritte Menge verschieden von der zweiten Menge, vorzugsweise größer als die zweite Menge ist,
- Ausgeben und/oder Speichern der synthetischen dritten Repräsentation und/oder Übermitteln der synthetischen dritten Repräsentation an ein separates Computersystem

wobei das zweite Teilmodell konfiguriert ist,

- die erste Repräsentation des Untersuchungsbereichs des Untersuchungsobjekts von der zweiten Repräsentation des Untersuchungsbereichs des Untersuchungsobjekts zu subtrahieren, wobei eine Differenz gebildet wird,
- die Differenz mit einem Verstärkungsfaktor zu multiplizieren, wobei der Verstärkungsfaktor eine positive oder negative reelle Zahl ist, wobei der mindestens eine von dem ersten Teilmodell ermittelte Modellparameter den Verstärkungsfaktor umfasst,
- die mit dem Verstärkungsfaktor multiplizierte Differenz auf die erste Repräsentation oder die zweite Repräsentation zu addieren.

Ein weiterer Gegenstand der vorliegenden Offenbarung ist ein Kit umfassend ein Computerprogrammprodukt und ein Kontrastmittel, wobei das Computerprogrammprodukt ein Computerprogramm umfasst, das in einen Arbeitsspeicher eines Computersystems geladen werden kann und dort das Computersystem dazu veranlasst, folgende Schritte ausführen:

- Bereitstellen eines trainierten Modells des maschinellen Lernens,

  ◦ wobei das trainierte Modell des maschinellen Lernens anhand von Trainingsdaten trainiert wurde,

  ◦ wobei die Trainingsdaten für jedes Referenzobjekt einer Vielzahl an Referenzobjekten (i) eine erste Referenz-Repräsentation eines Referenzbereichs des Referenzobjekts und eine zweite Referenz-Repräsentation des Referenzbereichs des Referenzobjekts als Eingabedaten und (ii) eine dritte Referenz-Repräsentation des Referenzbereichs des Referenzobjekts als Zieldaten umfassen,

  ◦ wobei die erste Referenz-Repräsentation den Referenzbereich ohne Kontrastmittel oder nach Applikation einer ersten Menge des Kontrastmittels repräsentiert,

  ◦ wobei die zweite Referenz-Repräsentation den Referenzbereich nach Applikation einer zweiten Menge des Kontrastmittels repräsentiert, wobei die zweite Menge größer als die erste Menge ist,

  ◦ wobei die dritte Referenz-Repräsentation den Referenzbereich nach Applikation einer dritten Menge des Kontrastmittels repräsentiert, wobei die dritte Menge verschieden von der zweiten Menge, vorzugsweise größer als die zweite Menge ist,

  ◦ wobei das trainierte Modell des maschinellen Lernens zwei Teilmodelle umfasst: ein erstes Teilmodell und ein zweites Teilmodell, wobei das erste Teilmodell konfiguriert ist und trainiert wurde, auf Basis der ersten Referenz-Repräsentation und der zweiten Referenz-Repräsentation eines Referenzobjekts mindestens einen

Modellparameter für das zweite Teilmodell zu ermitteln, wobei das zweite Teilmodell konfiguriert ist, auf Basis der ersten Referenz-Repräsentation und der zweiten Referenz-Repräsentation und des von dem ersten Teilmodell ermittelten mindestens einen Modellparameters eine synthetische dritte Referenz-Repräsentation zu erzeugen,

- Bereitstellen einer ersten Repräsentation, wobei die erste Repräsentation einen Untersuchungsbereich eines Untersuchungsobjekts ohne Kontrastmittel oder nach Applikation einer ersten Menge des Kontrastmittels repräsentiert,
- Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation und der zweiten Repräsentation dem trainierten Modell des maschinellen Lernens,
- Empfangen einer synthetischen dritten Repräsentation des Untersuchungsbereichs des Untersuchungsobjekts von dem trainierten Modell des maschinellen Lernens, wobei die synthetische dritte Repräsentation den Referenzbereich nach Applikation einer dritten Menge des Kontrastmittels repräsentiert, wobei die dritte Menge verschieden von der zweiten Menge, vorzugsweise größer als die zweite Menge ist,
- Ausgeben und/oder Speichern der synthetischen dritten Repräsentation und/oder Übermitteln der synthetischen dritten Repräsentation an ein separates Computersystem

wobei das zweite Teilmodell konfiguriert ist,

- die erste Repräsentation des Untersuchungsbereichs des Untersuchungsobjekts von der zweiten Repräsentation des Untersuchungsbereichs des Untersuchungsobjekts zu subtrahieren, wobei eine Differenz gebildet wird,
- die Differenz mit einem Verstärkungsfaktor zu multiplizieren, wobei der Verstärkungsfaktor eine positive oder negative reelle Zahl ist, wobei der mindestens eine von dem ersten Teilmodell ermittelte Modellparameter den Verstärkungsfaktor umfasst,
- die mit dem Verstärkungsfaktor multiplizierte Differenz auf die erste Repräsentation oder die zweite Repräsentation zu addieren.

DETAILLIERTE BESCHREIBUNG

Die Gegenstände der vorliegenden Offenbarung werden nachstehend näher erläutert, ohne zwischen den Gegenständen (Verfahren, Computersystem, Computerprogramm(produkt), Verwendung, Kit) zu unterscheiden. Die nachfolgenden Erläuterungen sollen vielmehr für alle Gegenstände in analoger Weise gelten, unabhängig davon, in welchem Kontext (Verfahren, Computersystem, Computerprogramm(produkt), Verwendung, Kit) sie erfolgen.

Wenn in der vorliegenden Beschreibung oder in den Patentansprüchen Schritte in einer Reihenfolge genannt sind, bedeutet dies nicht zwingend, dass diese Offenbarung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen dar.

Die Erfindung wird an einigen Stellen in Bezug auf Zeichnungen näher erläutert. Dabei sind in den Zeichnungen konkrete Ausführungsformen mit konkreten Merkmalen und Merkmalskombinationen dargestellt, die in erster Linie der Veranschaulichung dienen; die Erfindung soll nicht so verstanden werden, dass sie auf die in den Zeichnungen dargestellten Merkmale und Merkmalskombinationen beschränkt ist. Ferner sollen Aussagen, die bei der Beschreibung der Zeichnungen in Bezug auf Merkmale und Merkmalskombinationen getroffen werden, allgemein gelten, das heißt auch auf andere Ausführungsformen übertragbar und nicht auf die gezeigten Ausführungsformen beschränkt sein.

Die vorliegende Offenbarung beschreibt Mittel, mit denen auf Basis einer ersten Repräsentation eines Untersuchungsbereichs eines Untersuchungsobjekts und einer zweiten Repräsentation des Untersuchungsbereichs des Untersuchungsobjekts eine dritte Repräsentation des Untersuchungsbereichs des Untersuchungsobjekts vorhergesagt werden kann.

Eine solche vorhergesagte dritte Repräsentation wird in dieser Offenbarung auch als synthetische dritte Repräsentation bezeichnet. Die Vorhersage einer dritten Repräsentation wird in dieser Offenbarung auch als Erzeugung einer synthetischen dritten Repräsentation bezeichnet.

Der Begriff "synthetisch" bedeutet, dass die synthetische Repräsentation nicht das (unmittelbare) Ergebnis einer physischen Messung an einem realen Untersuchungsobjekt ist, sondern dass das Bild durch ein Modell des maschinellen Lernens erzeugt (berechnet) worden ist. Ein Synonym für den Begriff "synthetisch" ist der Begriff "künstlich". Eine synthetische Repräsentation kann aber auf gemessenen Repräsentationen basieren, d.h. das Modell des maschinellen Lernens kann die

synthetische Repräsentation auf Basis von gemessenen Repräsentationen erzeugen.

Das "Untersuchungsobjekt" ist üblicherweise ein Lebewesen, vorzugsweise ein Säugetier, ganz besonders bevorzugt ein Mensch.

Der "Untersuchungsbereich" ist ein Teil des Untersuchungsobjekts, zum Beispiel ein Organ oder ein Teil eines Organs oder mehrere Organe oder ein anderer Teil des Untersuchungsobjekts.

Der Untersuchungsbereich kann beispielsweise eine Leber, eine Niere, ein Herz, eine Lunge, ein Gehirn, ein Magen, eine Blase, eine Prostatadrüse, ein Darm oder ein Teil der genannten Teile oder ein anderer Teil des Körpers eines Säugetiers (z.B. eines Menschen) sein.

In einer Ausführungsform umfasst der Untersuchungsbereich eine Leber oder einen Teil einer Leber oder der Untersuchungsbereich ist eine Leber oder ein Teil einer Leber eines Säugetiers, vorzugsweise eines Menschen.

In einer weiteren Ausführungsform umfasst der Untersuchungsbereich ein Gehirn oder einen Teil eines Gehirns oder der Untersuchungsbereich ist ein Gehirn oder ein Teil eines Gehirns eines Säugetiers, vorzugsweise eines Menschen.

In einer weiteren Ausführungsform umfasst der Untersuchungsbereich ein Herz oder ein Teil eines Herzes oder der Untersuchungsbereich ist ein Herz oder ein Teil eines Herzes eines Säugetiers, vorzugsweise eines Menschen.

In einer weiteren Ausführungsform umfasst der Untersuchungsbereich einen Thorax oder einen Teil eines Thorax oder der Untersuchungsbereich ist ein Thorax oder ein Teil eines Thorax eines Säugetiers, vorzugsweise eines Menschen.

In einer weiteren Ausführungsform umfasst der Untersuchungsbereich einen Magen oder einen Teil eines Magens oder der Untersuchungsbereich ist ein Magen oder ein Teil eines Magens eines Säugetiers, vorzugsweise eines Menschen.

In einer weiteren Ausführungsform umfasst der Untersuchungsbereich eine Bauchspeicheldrüse oder einen Teil einer Bauchspeicheldrüse oder der Untersuchungsbereich ist eine Bauchspeicheldrüse oder ein Teil einer Bauchspeicheldrüse eines Säugetiers, vorzugsweise eines Menschen.

In einer weiteren Ausführungsform umfasst der Untersuchungsbereich eine Niere oder einen Teil einer Niere oder der Untersuchungsbereich ist eine Niere oder ein Teil einer Niere eines Säugetiers, vorzugsweise eines Menschen.

In einer weiteren Ausführungsform umfasst der Untersuchungsbereich einen oder beide Lungenflügel oder einen Teil eines Lungenflügels Säugetiers, vorzugsweise eines Menschen.

In einer weiteren Ausführungsform umfasst der Untersuchungsbereich eine Brust oder einen Teil einer Brust oder der Untersuchungsbereich ist eine Brust oder ein Teil einer Brust eines weiblichen Säugetiers, vorzugsweise eines weiblichen Menschen.

In einer weiteren Ausführungsform umfasst der Untersuchungsbereich eine Prostata oder einen Teil einer Prostata oder der Untersuchungsbereich ist eine Prostata oder ein Teil einer Prostata eines männlichen Säugetiers, vorzugsweise eines männlichen Menschen.

Der Untersuchungsbereich, auch Aufnahmevolumen (engl.: *field of view*, FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme festgelegt. Selbstverständlich kann der Untersuchungsbereich alternativ oder zusätzlich auch automatisch, beispielsweise auf Grundlage eines ausgewählten Protokolls, festgelegt werden.

Der Untersuchungsbereich wird einer radiologischen Untersuchung unterzogen.

Die "Radiologie" ist das Teilgebiet der Medizin, das sich mit der Anwendung elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlung wie Gammastrahlung oder Elektronen zum Einsatz. Da ein wesentlicher Einsatzzweck die Bildgebung ist, werden auch andere bildgebende Verfahren wie die Sonographie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Offenbarung umfasst damit insbesondere die folgenden Untersuchungsmethoden: Computertomographie, Magnetresonanztomographie, Sonographie.

In einer Ausführungsform der vorliegenden Offenbarung handelt es sich bei der radiologischen Untersuchung um eine magnetresonanztomographische Untersuchung.

In einer weiteren Ausführungsform handelt es sich bei der radiologischen Untersuchung um eine computertomographische Untersuchung

In einer weiteren Ausführungsform handelt es sich bei der radiologischen Untersuchung um eine Ultraschalluntersuchung.

Die erste Repräsentation und die zweite Repräsentation sind das Ergebnis einer solchen radiologischen Untersuchung. Die erste und die zweite Repräsentation sind üblicherweise gemessene radiologische Aufnahmen oder werden auf Basis solcher gemessenen radiologischen Aufnahmen erzeugt. Die erste und/oder die zweite Repräsentation können beispielsweise eine MRT-Aufnahme, eine CT-Aufnahme und/oder eine Ultraschallaufnahme sein.

Die erste Repräsentation repräsentiert den Untersuchungsbereich des Untersuchungsobjekts ohne Kontrastmittel oder nach Applikation einer ersten Menge

eines Kontrastmittels. Vorzugsweise repräsentiert die erste Repräsentation den Untersuchungsbereich ohne Kontrastmittel (native Repräsentation).

Die zweite Repräsentation repräsentiert den Untersuchungsbereich des Untersuchungsobjekts nach Applikation einer zweiten Menge des Kontrastmittels. Die zweite Menge ist größer als die erste Menge (wobei die erste Menge wie beschrieben auch Null sein kann).

Der Ausdruck "nach Applikation einer zweiten Menge des Kontrastmittels" soll nicht so verstanden werden, dass sich die erste Menge und die zweite Menge in dem Untersuchungsbereich addieren. Der Ausdruck "die Repräsentation repräsentiert den Untersuchungsbereich nach Applikation einer (ersten oder zweiten) Menge" soll also eher bedeuten: "die Repräsentation repräsentiert den Untersuchungsbereich mit einer (ersten oder zweiten) Menge" oder "die Repräsentation repräsentiert den Untersuchungsbereich umfassend eine (erste oder zweite) Menge". Dies gilt analog auch für die dritte Menge des Kontrastmittels.

Die vorhergesagte dritte Repräsentation repräsentiert den Untersuchungsbereich des Untersuchungsobjekts nach Applikation einer dritten Menge des Kontrastmittels. Die dritte Menge ist verschieden von, vorzugsweise größer als die zweite Menge.

"Kontrastmittel" sind Substanzen oder Gemische von Substanzen, die die Darstellung von Strukturen und Funktionen des Körpers bei radiologischen Untersuchungen verbessern.

In der Computertomographie werden meist iodhaltige Lösungen als Kontrastmittel eingesetzt. In der Magnetresonanztomographie (MRT) werden üblicherweise superparamagnetische Substanzen (z.B. Eisenoxidnanopartikel, superparamagnetische Eisen-Platin-Partikel (SIPPs)) oder paramagnetische Substanzen (z.B. Gadolinium-Chelate, Mangan-Chelate, Hafnium-Chelate) als Kontrastmittel verwendet. Im Fall der Sonographie werden üblicherweise Flüssigkeiten, die gasgefüllte Mikrobläschen (*microbubbles*) enthalten, intravenös verabreicht. Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wp-content/uploads/2017/10/contrast-agents-tutorial.pdf, M. R. Nouh et al.: Radiographie and magnetic resonances contrast agents: Essentials and tips for safe practices, World J Radiol. 2017 Sep 28; 9(9): 339-349; L. C. Abonyi et al.: Intravascular Contrast Media in Radiography: Historical Development & Review of Risk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

MRT-Kontrastmittel entfalten in einer MRT-Untersuchung ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: *superparamagnetic iron oxide*). Beispiele für paramagnetische Kontrastmittel sind Gadolinium-Chelate wie Gadopentetat-Dimeglumin (Handelsname: Magnevist® u.a.), Gadotersäure (Dotarem®, Dotagita®, Cyclolux®), Gadodiamid (Omniscan®), Gadoteridol (ProHance®), Gadobutrol (Gadovist®), Gadopiclenol (Elucirem, Vueway) und Gadoxetsäure (Primovist®/Eovist®).

In einer Ausführungsform der vorliegenden Offenbarung handelt es sich bei der radiologischen Untersuchung um eine MRT-Untersuchung, bei der ein MRT-Kontrastmittel eingesetzt wird.

In einer weiteren Ausführungsform handelt es sich bei der radiologischen Untersuchung um eine CT-Untersuchung, bei der ein CT-Kontrastmittel eingesetzt wird.

In einer weiteren Ausführungsform handelt es sich bei der radiologischen Untersuchung um eine CT-Untersuchung, bei der ein MRT-Kontrastmittel eingesetzt wird.

In einer Ausführungsform ist sowohl die erste Menge als auch die zweite Menge des Kontrastmittels kleiner als die Standardmenge.

In einer weiteren Ausführungsform entspricht die zweite Menge des Kontrastmittels der Standardmenge.

In einer weiteren Ausführungsform ist die erste Menge des Kontrastmittels gleich Null und die zweite Menge des Kontrastmittels ist kleiner als die Standardmenge.

In einer weiteren Ausführungsform ist die erste Menge des Kontrastmittels gleich Null und die zweite Menge des Kontrastmittels entspricht der Standardmenge.

Die Standardmenge ist üblicherweise die vom Hersteller und/oder Vertreiber des Kontrastmittels empfohlene und/oder die von einer Zulassungsbehörde zugelassene und/oder die in einem Beipackzettel zum Kontrastmittel aufgeführte Menge.

So beträgt die Standardmenge von Primovist® beispielsweise 0,025 mmol Gd-EOB-DTPA Dinatrium / kg Körpergewicht.

In einer Ausführungsform der vorliegenden Offenbarung handelt es sich bei dem Kontrastmittel um ein Mittel, das Gadolinium(III) 2-[4,7,10-tris(carboxymethyl)-1,4,7,10-tetrazacyclododec-1-yl]essigsäure (auch als Gadolinium-DOTA oder Gadotersäure bezeichnet) umfasst.

In einer weiteren Ausführungsform handelt es sich bei dem Kontrastmittel um ein Mittel, das Gadolinium(III) Ethoxybenzyl-diethylenetriaminepentaessigsäure (Gd-EOB-DTPA) umfasst; vorzugsweise umfasst das Kontrastmittel das Dinatriumsalz der Gadolinium(III)-ethoxybenzyl-diethylenetriaminpentaessigsäure (auch als Gadoxetsäure bezeichnet).

In einer Ausführungsform der vorliegenden Offenbarung handelt es sich bei dem Kontrastmittel um ein Mittel, das Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat (auch als Gadopiclenol bezeichnet) umfasst (siehe z.B. WO2007/042504 sowie WO2020/030618 und/oder WO2022/013454).

In einer Ausführungsform der vorliegenden Offenbarung handelt es sich bei dem Kontrastmittel um ein Mittel, das Dihydrogen[(±)-4-carboxy-5,8,11-tris(carboxymethyl)-1-phenyl-2-oxa-5,8,11-triazatridecan-13-oato(5-)]gadolinat(2-) (auch als Gadobensäure bezeichnet) umfasst.

In einer Ausführungsform der vorliegenden Offenbarung handelt es sich bei dem Kontrastmittel um ein Mittel, das Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat (auch als Gadoquatrane bezeichnet) umfasst (siehe z.B. J. Lohrke et al.: Preclinical Profile of Gadoquatrane: A Novel Tetramerie, Macrocyclic High Relaxivity Gadolinium-Based Contrast Agent. Invest Radiol., 2022, 1, 57(10): 629-638; WO2016193190).

In einer Ausführungsform der vorliegenden Offenbarung handelt es sich bei dem Kontrastmittel um ein Mittel, das einen $Gd^{3+}$-Komplex einer Verbindung der Formel (I)

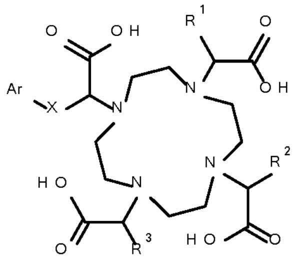

(I) ,

umfasst, wobei

Ar eine Gruppe ausgewählt aus

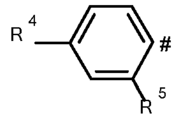

und

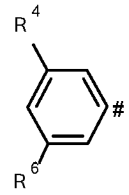

darstellt,

wobei # die Anknüpfung zu X darstellt,

X eine Gruppe darstellt, die aus

$CH_2$, $(CH_2)_2$, $(CH_2)_3$, $(CH_2)_4$ und $*-(CH_2)_2-O-CH_2-^{\#}$ ausgewählt wird,

wobei * die Anknüpfung zu Ar darstellt und $^{\#}$ die Anknüpfung zum Essigsäurerest darstellt,

$R^1$, $R^2$ and $R^3$ unabhängig voneinander ein Wasserstoffatom oder eine Gruppe ausgewählt aus $C_1$-$C_3$-Alkyl, $-CH_2OH$, $-(CH_2)_2OH$ und $-CH_2OCH_3$ darstellen,

$R^4$ eine Gruppe ausgewählt aus $C_2$-$C_4$-Alkoxy, $(H_3C-CH_2)-O-(CH_2)_2-O-$, $(H_3C-CH_2)-O-(CH_2)_2-O-(CH_2)_2-O-$ und $(H_3C-CH_2)-O-(CH_2)_2-O-(CH_2)_2-O-(CH_2)_2-O-$ darstellt,

$R^5$ ein Wasserstoffatom darstellt,

und

$R^6$ ein Wasserstoffatom darstellt,

oder ein Stereoisomer, Tautomer, Hydrat, Solvat oder Salz davon, oder eine Mischung davon.

In einer Ausführungsform der vorliegenden Offenbarung handelt es sich bei dem Kontrastmittel um ein Mittel, das einen $Gd^{3+}$-Komplex einer Verbindung der Formel (II)

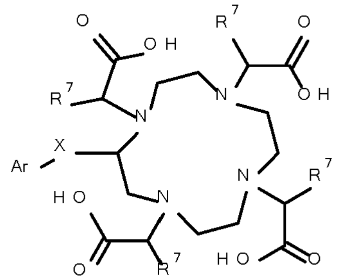

(II) ,

umfasst, wobei

Ar eine Gruppe ausgewählt aus

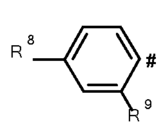

and

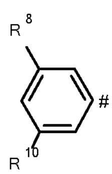

darstellt,

wobei # die Anknüpfung zu X darstellt,

X eine Gruppe darstellt, die aus $CH_2$, $(CH_2)_2$, $(CH_2)_3$, $(CH_2)_4$ and $*-(CH_2)_2-O-CH_2-^{\#}$ ausgewählt wird, wobei * die Anknüpfung zu Ar darstellt und # die Anknüpfung zum Essigsäurerest darstellt,

$R^7$ ein Wasserstoffatom oder eine Gruppe ausgewählt aus $C_1$-$C_3$-Alkyl, $-CH_2OH$, $-(CH_2)_2OH$ und $-CH_2OCH_3$ darstellt;

$R^8$ eine Gruppe ausgewählt aus $C_2$-$C_4$-Alkoxy, $(H_3C-CH_2O)-(CH_2)_2-O-$, $(H_3C-CH_2O)-(CH_2)_2-O-(CH_2)_2-O-$ und $(H_3C-CH_2O)-(CH_2)_2-O-(CH_2)_2-O-(CH_2)_2-O-$ darstellt;

$R^9$ und $R^{10}$ unabhängig voneinander ein Wasserstoffatom darstellen;

oder ein Stereoisomer, Tautomer, Hydrat, Solvat oder Salz davon, oder eine Mischung davon.

Der Begriff "$C_1$-$C_3$-Alkyl" bedeutet eine lineare oder verzweigte, gesättigte, einwertige Kohlenwasserstoffgruppe mit 1, 2 oder 3 Kohlenstoffatomen, z.B. Methyl, Ethyl, n-Propyl und Isopropyl. Der Begriff "$C_2$-$C_4$-Alkyl" bedeutet eine lineare oder verzweigte, gesättigte, einwertige Kohlenwasserstoffgruppe mit 2, 3 oder 4 Kohlenstoffatomen.

Der Begriff "$C_2$-$C_4$-Alkoxy" bedeutet eine lineare oder verzweigte, gesättigte, einwertige Gruppe der Formel ($C_2$-$C_4$-Alkyl)-O-, in der der Begriff "$C_2$-$C_4$-Alkyl" wie oben definiert ist, z. B. ein Methoxy, Ethoxy, n-Propoxy oder Isopropoxygruppe.

In einer Ausführungsform der vorliegenden Offenbarung handelt es sich bei dem Kontrastmittel um ein Mittel, das Gadolinium 2,2',2"-(10-{1-carboxy-2-[2-(4-ethoxyphenyl)ethoxy]ethyl}-1,4,7,10-tetraazacyclododecan-1,4,7-triyl)triacetat umfasst (siehe z.B. WO2022/194777, Beispiel 1).

In einer Ausführungsform der vorliegenden Offenbarung handelt es sich bei dem Kontrastmittel um ein Mittel, das Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 2).

In einer Ausführungsform der vorliegenden Offenbarung handelt es sich bei dem Kontrastmittel um ein Mittel, das Gadolinium 2,2',2"-{10-[(1R)-1-carboxy-2-{4-[2-(2-ethoxyethoxy)ethoxy]phenyl} ethyl]-1,4,7,10-tetraazacyclododecan-1,4,7-triyl}triacetat umfasst (siehe z.B. WO2022/194777, Beispiel 4).

In einer Ausführungsform der vorliegenden Offenbarung handelt es sich bei dem Kontrastmittel um ein Mittel, das Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat) umfasst (siehe z.B. WO2022/194777, Beispiel 15).

In einer Ausführungsform der vorliegenden Offenbarung handelt es sich bei dem Kontrastmittel um ein Mittel, das Gadolinium 2,2',2"-{10-[(1S)-4-(4-butoxyphenyl)-1-carboxybutyl]-1,4,7,10-tetraazacyclododecan-1,4,7-triyl}triacetat umfasst (siehe z.B. WO2022/194777, Beispiel 31).

In einer Ausführungsform der vorliegenden Offenbarung handelt es sich bei dem Kontrastmittel um ein Mittel, das Gadolinium-2,2',2"-{(2S)-10-(carboxymethyl)-2-[4-(2-ethoxyethoxy)benzyl]-1,4,7,10-tetraazacyclododecane-1,4,7-triyl}triacetat umfasst.

In einer Ausführungsform der vorliegenden Offenbarung handelt es sich bei dem Kontrastmittel um ein Mittel, das Gadolinium-2,2',2"-[10-(carboxymethyl)-2-(4-ethoxybenzyl)-1,4,7,10-tetraazacyclododecane-1,4,7-triyl]triacetat umfasst.

In einer Ausführungsform der vorliegenden Offenbarung handelt es sich bei dem Kontrastmittel um ein Mittel, das Gadolinium(III) 5,8-bis(carboxylatomethyl)-2-[2-(methylamino)-2-oxoethyl]-10-oxo-2,5,8,11-tetraazadodecan-1-carboxylat-Hydrat (auch als Gadodiamid bezeichnet) umfasst.

In einer Ausführungsform der vorliegenden Offenbarung handelt es sich bei dem Kontrastmittel um ein Mittel, das Gadolinium(III) 2-[4-(2-hydroxypropyl)-7,10-bis(2-oxido-2-oxoethyl)-1,4,7,10-tetrazacyclododec-1-yl]acetat (auch als Gadoteridol bezeichnet) umfasst.

In einer Ausführungsform der vorliegenden Offenbarung handelt es sich bei dem Kontrastmittel um ein Mittel, das Gadolinium(III) 2,2',2"-(10-((2R,3S)-1,3,4-trihydroxybutan-2-yl)-1,4,7,10-tetraazacyclododecan-1,4,7-triyl)triacetat (auch als Gadobutrol oder Gd-DO3A-butrol bezeichnet) umfasst.

Eine Repräsentation eines Untersuchungsbereichs im Sinne der vorliegenden Offenbarung ist vorzugsweise eine radiologische Aufnahme des Untersuchungsbereichs.

Eine Repräsentation eines Untersuchungsbereichs (sowie eine Referenz-Repräsentation eines Referenzbereichs) im Sinne der vorliegenden Offenbarung kann eine Repräsentation im Ortsraum (Bildraum), eine Repräsentation im Frequenzraum, eine Repräsentation im Projektionsraum oder eine Repräsentation in einem anderen Raum sein.

In einer Repräsentation im Ortsraum, in dieser Beschreibung auch als Ortsraumdarstellung oder Ortsraum-Repräsentation bezeichnet, wird der Untersuchungsbereich üblicherweise durch eine Vielzahl an Bildelementen (z.B. Pixel oder Voxel oder Doxel) repräsentiert, die beispielsweise rasterförmig angeordnet sein können, wobei jedes Bildelement einen Teil des Untersuchungsbereichs repräsentiert, wobei jedem Bildelement ein Farbwert oder Grauwert zugeordnet sein kann. Der Farbwert oder Grauwert repräsentiert eine Signalintensität, z.B. die Abschwächung von Röntgenstrahlen. Ein in der Radiologie weit verbreitetes Format zur Speicherung und Verarbeitung von Repräsentationen im

Ortsraum ist das DICOM-Format. DICOM (Digital Imaging and Communications in Medicine) ist ein offener Standard zur Speicherung und zum Austausch von Informationen im medizinischen Bilddatenmanagement.

In einer Repräsentation im Frequenzraum, in dieser Beschreibung auch als Frequenzraumdarstellung oder Frequenzraum-Repräsentation bezeichnet, wird der Untersuchungsbereich durch eine Überlagerung von Grundschwingungen repräsentiert. Beispielsweise kann der Untersuchungsbereich durch eine Summe von Sinus- und Kosinus-Funktionen mit verschiedenen Amplituden, Frequenzen und Phasen repräsentiert werden. Die Amplituden und Phasen können als Funktion der Frequenzen beispielsweise in einer zwei- oder dreidimensionalen Darstellung aufgetragen werden. Üblicherweise wird die niedrigste Frequenz (Ursprung) in das Zentrum gelegt. Je weiter man sich von diesem Zentrum entfernt, desto höher sind die Frequenzen. Jeder Frequenz kann eine Amplitude, mit der die Frequenz in der Frequenzraumdarstellung vertreten ist, und eine Phase, die angibt, inwieweit die jeweilige Schwingung gegenüber einer Sinus- oder Kosinus-Schwingung verschoben ist, zugeordnet werden.

Eine Repräsentation im Ortsraum lässt sich beispielsweise durch eine Fourier-Transformation in eine Repräsentation im Frequenzraum überführen (transformieren). Umgekehrt lässt sich eine Repräsentation im Frequenzraum beispielsweise durch eine inverse Fourier-Transformation in eine Repräsentation im Ortsraum überführen (transformieren).

Details über Ortsraumdarstellungen und Frequenzraumdarstellungen und ihre jeweilige Umwandlung ineinander sind in zahlreichen Publikationen beschrieben, siehe z.B.: https://see.stanford.edu/materials/lsoftaee261/book-fall-07.pdf.

Eine Repräsentation eines Untersuchungsbereichs im Projektionsraum ist üblicherweise das Ergebnis einer computertomographischen Untersuchung vor einer Bildrekonstruktion. Mit anderen Worten: die bei der computertomographischen Untersuchung anfallenden Rohdaten können als eine Projektionsraumdarstellung aufgefasst werden. Im Rahmen einer Computertomographie wird die Intensität bzw. die Schwächung der Röntgenstrahlung beim Durchgang durch das Untersuchungsobjekt gemessen. Daraus lassen sich Projektionswerte errechnen. In einem zweiten Schritt wird die durch die Projektion kodierte Objektinformation durch eine computergestützte Rekonstruktion in ein Bild (Ortsraumdarstellung) transformiert. Die Rekonstruktion kann mit der Radon-Transformation erfolgen. Die Radon-Transformation beschreibt die Verknüpfung zwischen dem unbekannten Untersuchungsobjekt und seinen zugehörigen Projektionen.

Details über die Transformation von Projektionsdaten in eine Ortsraumdarstellung sind in zahlreichen Publikationen beschrieben, siehe z.B. K. Fang: The Radon Transformation and Its Application in Tomography, Journal of Physics Conference Series 1903(1):012066.

Eine Repräsentation des Untersuchungsbereichs kann auch eine Repräsentation im Hough-Raum sein. Zur Erkennung von geometrischen Objekten in einem Bild wird nach einer Kantendetektion durch die so genannte Hough-Transformation ein Dualraum erschaffen, in den für jeden Punkt im Bild, der auf einer Kante liegt, alle möglichen Parameter des geometrischen Objekts eingetragen werden. Jeder Punkt im Dualraum entspricht damit einem geometrischen Objekt im Bildraum. Bei einer Geraden kann das z.B. die Steigung und der y-Achsen-Abschnitt der Geraden sein, bei einem Kreis der Mittelpunkt und Radius des Kreises. Details über die Hough-Transformation sind in der Literatur zu finden (siehe z.B.: A.S. Hassanein et al.: A Survey on Hough Transform, Theory, Techniques and Applications, arXiv:1502.02160v1).

Es gibt weitere Räume, in denen Repräsentationen des Untersuchungsbereichs vorliegen können. Der Einfachheit halber und der besseren Verständlichkeit halber wird die Erfindung in weiten Teilen der Beschreibung auf Basis von Ortsraum-Repräsentationen beschrieben. Dies soll jedoch nicht als Einschränkung verstanden werden. Der Fachmann der Bildanalyse weiß, wie er die entsprechenden Teile der Beschreibung auf andere Repräsentationen als Ortsraum-Repräsentationen anwenden kann.

Die Erzeugung der synthetischen dritten Repräsentation (d.h. die Vorhersage der dritten Repräsentation) erfolgt mit Hilfe eines trainierten Modells des maschinellen Lernens.

Ein "Modell des maschinellen Lernens" kann als eine computerimplementierte Datenverarbeitungsarchitektur verstanden werden. Ein solches Modell kann Eingabedaten empfangen und Ausgabedaten auf der Grundlage dieser Eingabedaten und Modellparametern liefern. Ein solches Modell kann durch Training eine Beziehung zwischen den Eingabedaten und den Ausgabedaten erlernen. Beim Training können Modellparameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modellparameter werden so verändert, dass die Abweichungen zwischen den Ausgabedaten und den Zieldaten auf ein (definiertes) Minimum reduziert werden. Zur Modifizierung der Modellparameter im Hinblick auf eine Reduzierung der Abweichungen kann ein Optimierungsverfahren wie beispielsweise ein Gradientenverfahren verwendet werden.

Die Abweichungen können mit Hilfe einer Fehlerfunktion (engl.: *loss function*) quantifiziert werden. Eine solche Fehlerfunktion kann verwendet werden, um einen Fehler (engl.: *loss*) für ein gegebenes Paar von Ausgabedaten und Zieldaten zu berechnen. Das Ziel des Trainingsprozesses kann darin bestehen, die Parameter des Modells des maschinellen Lernens so zu verändern (anzupassen), dass der Fehler für alle Paare des Trainingsdatensatzes auf ein (definiertes) Minimum reduziert wird.

Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Fehler bedeuten, dass ein oder mehrere Modellparameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder eine andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

Das hier beschriebene Modell wird als "Modell des maschinellen Lernens" verstanden, weil es mindestens einen Bestandteil umfasst, der zum Beispiel in einem überwachten Lernverfahren trainiert werden kann. Bestandteile des hier beschriebenen Modells werden in dieser Beschreibung auch als Teilmodelle bezeichnet. Solche Teilmodelle können separat voneinander eingesetzt werden und/oder in dem (Gesamt-)Modell so miteinander verbunden sein, dass die Ausgabe eines Teilmodells direkt dem nachfolgenden Teilmodell zugeführt wird. Mit anderen Worten: Teilmodelle können nach außen als separate Einheiten erkennbar sein und/oder so miteinander verbunden sein, dass sie nach außen als ein einheitliches Modell wahrgenommen werden.

Das trainierte Modell des maschinellen Lernens, das zur Erzeugung einer synthetischen dritten Repräsentation eingesetzt wird, umfasst mindestens zwei Teilmodelle, ein erstes Teilmodell und ein zweites Teilmodell. Das trainierte Modell des maschinellen Lernens kann neben dem ersten Teilmodell und dem zweiten Teilmodell ein oder mehrere weitere Teilmodelle umfassen.

Das erste Teilmodell ist ein Modell des maschinellen Lernens. In einem Trainingsverfahren wird das erste Teilmodell auf Basis von Trainingsdaten trainiert. Das erste Teilmodell ist konfiguriert und wird trainiert, mindestens einen Modellparameter für das zweite Teilmodell zu ermitteln (vorherzusagen).

Das zweite Teilmodell ist ein mechanistisches (deterministisches) Modell. Mechanistische Modelle beruhen auf grundlegenden Prinzipien und bekannten Beziehungen innerhalb eines Systems. Sie werden oft aus wissenschaftlichen Theorien und bereichsspezifischem Wissen abgeleitet. Mechanistische Modelle beschreiben die zugrundeliegenden Mechanismen eines Systems mit Hilfe mathematischer Gleichungen oder physikalischer Gesetze. Sie zielen darauf ab, das Verhalten des Systems auf der Grundlage eines Verständnisses seiner Komponenten und Wechselwirkungen zu simulieren.

Modelle des maschinellen Lernens sind hingegen datengesteuert und lernen Muster und Beziehungen aus Eingabedaten, ohne dass die Beziehungen explizit programmiert werden.

Während also mechanistische Modelle auf grundlegenden Prinzipien beruhen und darauf abzielen, die zugrundeliegenden Systemmechanismen darzustellen, lernen Modelle des maschinellen Lernens Muster und Beziehungen direkt aus Daten, ohne diese Beziehungen explizit zu programmieren.

Das mechanistische Modell beruht also auf physikalischen Gesetzen. In radiologischen Untersuchungen ist ein Signal, das von einem Kontrastmittel hervorgerufen wird, üblicherweise abhängig von der Menge (z.B. der Konzentration) des Kontrastmittels in dem Untersuchungsbereich. Die Signalstärke kann beispielsweise über einen definierten Konzentrationsbereich linear oder in Form einer anderen Abhängigkeit von der Konzentration des Kontrastmittels in dem Untersuchungsbereich abhängen. Die funktionelle Abhängigkeit der Signalstärke von der Konzentration kann man sich zu Nutze machen, um ein mechanistisches Modell zu erstellen.

Das mechanistische Modell umfasst mindestens einen Modellparameter, der die Signalintensitätsverteilung in der synthetischen dritten Repräsentation zumindest anteilig mitbestimmt. Der mindestens eine Modellparameter kann beispielsweise die Anhängigkeit der Signalstärke von der Konzentration an Kontrastmittel in dem Untersuchungsbereich repräsentieren. Der mindestens eine Modellparameter kann auch ein oder mehrere Parameter eines Filters umfassen, der auf eine Repräsentation des Untersuchungsbereichs angewendet wird.

Dies sei nachfolgend anhand von zwei Beispielen näher erläutert, ohne die Erfindung auf diese Beispiele beschränken zu wollen.

Subtrahiert man eine erste Ortsraum-Repräsentation eines Untersuchungsbereichs eines Untersuchungsobjekts, die den Untersuchungsbereich ohne Kontrastmittel repräsentiert (native Repräsentation), von einer zweiten Ortsraum-Repräsentation des Untersuchungsbereichs des Untersuchungsobjekts, die den Untersuchungsbereich nach Applikation einer zweiten,

von Null verschiedenen Menge eines Kontrastmittel repräsentiert, so ist das Ergebnis eine Ortsraum-Repräsentation des Untersuchungsbereichs, bei der die Signalintensitätsverteilung nur durch das Kontrastmittel bestimmt wird (Kontrastmittelverteilungs-Repräsentation). Bei einer solchen Subtraktion werden üblicherweise die Farbwerte oder Grauwerte korrespondierender Bildelemente voneinander subtrahiert. Korrespondierende Bildelemente sind solche Bildelemente, die denselben Teilbereich des Untersuchungsbereichs repräsentieren.

Addiert man diese Kontrastmittelverteilungs-Repräsentation zu der ersten (nativen) Ortsraum-Repräsentation des Untersuchungsbereichs, ergibt sich wieder die zweite Ortsraum-Repräsentation des Untersuchungsbereichs. Auch bei einer solchen Addition werden üblicherweise die Farbwerte oder Grauwerte korrespondierender Bildelemente addiert.

Addiert man die Kontrastmittelverteilungs-Repräsentation nur anteilig (z.B. nach Multiplizieren mit dem Faktor 0,5 oder einem anderen Faktor zwischen 0 und 1) zu der ersten (nativen) Ortsraum-Repräsentation des Untersuchungsbereichs, so erhält man eine Ortsraum-Repräsentation des Untersuchungsbereichs, bei der die durch Kontrastmittel hervorgerufene Signalintensitätsverteilung größer ist als bei der ersten (nativen) Ortsraum-Repräsentation und kleiner ist als bei der zweiten Ortsraum-Repräsentation.

Multipliziert man die Kontrastmittelverteilungs-Repräsentation mit einem Faktor, der größer als 1 ist, und addiert das Ergebnis zu der ersten (nativen) Ortsraum-Repräsentation des Untersuchungsbereichs, so erhält man (ggf. nach einer Normierung) eine Ortsraum-Repräsentation des Untersuchungsbereichs, bei der die durch Kontrastmittel hervorgerufene Signalintensitätsverteilung größer ist als bei der zweiten Ortsraum-Repräsentation. Ein solche Multiplikation wird üblicherweise wie im Fall der oben beschriebenen Subtraktion und Addition durch Multiplizieren der Farbwerte oder Grauwerte aller Bildelemente mit dem Faktor durchgeführt.

Mit anderen Worten: die Kontrastmittelverteilungs-Repräsentation kann nach Multiplikation mit einem Verstärkungsfaktor $\alpha$ zu der ersten oder zweiten Repräsentation addiert werden, um die Kontrastverstärkung von Teilbereichen des Untersuchungsbereichs, die Kontrastmittel enthalten, gegenüber anderen Teilbereichen ohne Kontrastmittel zu verstärken (oder auch zu vermindern). Die Signalintensitäten von Teilbereichen, die mehr Kontrastmittel als andere Teilbereiche enthalten, können gegenüber den Signalintensitäten dieser anderen Teilbereiche verstärkt werden.

Es sind auch negative Werte von $\alpha$ möglich, die z.B. so gewählt werden können, dass Bereiche des Untersuchungsbereichs, die eine Kontrastmittel-induzierte Signalverstärkung in der messtechnisch erzeugten Repräsentation erfahren, in der synthetischen dritten Repräsentation komplett dunkel (schwarz) sind.

Der Verstärkungsfaktor $\alpha$ ist also eine positive oder negative reelle Zahl, mit der die Kontrastverstärkung variiert werden kann; durch Variation des Verstärkungsfaktors $\alpha$ kann der Kontrast zwischen Bereichen mit Kontrastmittel und Bereichen ohne Kontrastmittel also variiert werden.

Fig. 1 zeigt eine Ausführungsform des zweiten Teilmodells. Das zweite Teilmodell TM2 ist konfiguriert, auf Basis einer ersten Repräsentation R1 eines Untersuchungsbereichs eines Untersuchungsobjekts, einer zweiten Repräsentation R2 des Untersuchungsbereichs des Untersuchungsobjekts und des Verstärkungsfaktors $\alpha$ als Modellparameter eine synthetische dritte Repräsentation R3* des Untersuchungsbereichs des Untersuchungsobjekts zu erzeugen.

Das Untersuchungsobjekt ist im vorliegenden Beispiel ein Schwein und der Untersuchungsbereich umfasst die Leber des Schweins.

Die erste Repräsentation R1 ist eine magnetresonanztomografische Aufnahme, die den Untersuchungsbereich im Ortsraum ohne Kontrastmittel repräsentiert.

Die zweite Repräsentation R2 repräsentiert denselben Untersuchungsbereich desselben Untersuchungsobjekts wie die erste Repräsentation R1 im Ortsraum. Die zweite Repräsentation R2 ist ebenfalls eine magnetresonanztomografische Aufnahme.

Die zweite Repräsentation R2 repräsentiert den Untersuchungsbereich nach der Applikation einer zweiten Menge eines Kontrastmittels. Im vorliegenden Beispiel wurde dem Untersuchungsobjekt eine Menge von 25 $\mu$mol pro kg Körpergewicht eines hepatobiliären Kontrastmittels intravenös verabreicht. Die zweite Repräsentation R2 repräsentiert den Untersuchungsbereich in der so genannten arteriellen Phase (siehe z.B. DOI:10.1002/jmri.22200).

Ein hepatobiliäres Kontrastmittel zeichnet sich dadurch aus, dass es spezifisch von Leberzellen, den Hepatozyten, aufgenommen wird, sich im Funktionsgewebe (Parenchym) anreichert und den Kontrast in gesundem Lebergewebe verstärkt. Ein Beispiel eines hepatobiliären Kontrastmittels ist das Dinatriumsalz der Gadoxetsäure (Gd-EOB-DTPA Dinatrium), das in dem US-Patent No. 6,039,931A beschrieben ist, und unter den Markennamen Primovist® und Eovist® kommerziell erhältlich ist. Weitere hepatobiliäre Kontrastmittel sind u.a. in WO2022/194777 beschrieben.

Die erste Repräsentation R1 und die zweite Repräsentation R2 werden dem zweiten Teilmodel TM2 zugeführt. Auf Basis der ersten Repräsentation R1 und der zweiten Repräsentation R2 erzeugt das zweite Teilmodell TM2 eine synthetische dritte Repräsentation R3*. Synthetische Repräsentationen sind in dieser Offenbarung mit einem * gekennzeichnet.

In dem in Fig. 1 gezeigten Beispiel umfasst die Erzeugung der synthetischen dritten Repräsentation R3* ein Subtrahieren der ersten Repräsentation R1 von der zweiten Repräsentation R2. Die Differenz R2-R1 der ersten Repräsentation R1 von der zweiten Repräsentation R2 ist eine Kontrastmittelverteilungs-Repräsentation, wie sie oben beschrieben ist. Ferner umfasst das Erzeugen der synthetischen dritten Repräsentation R3* ein $\alpha$-faches Addieren der Differenz R2-R1 der ersten Repräsentation von der zweiten Repräsentation zu der ersten Repräsentation ($R3^* = \alpha \cdot (R2-R1) + R1$). Der Verstärkungsfaktor $\alpha$ wird von dem ersten Teilmodell TM1 bereitgestellt.

Falls beim Subtrahieren der ersten Repräsentation R1 von der zweiten Repräsentation R2 negative Grauwerte/Farbwerte entstehen, können diese negativen Werte auf Null (oder einen anderen Wert) gesetzt werden, um negative Werte zu vermeiden.

Die Differenz R2-R1 repräsentiert die durch die zweite Menge des Kontrastmittels im Untersuchungsbereich hervorgerufene Kontrastverstärkung (Signalintensitätsverteilung).

Die Differenz R2-R1 wird mit dem Verstärkungsfaktor $\alpha$ multipliziert und das Ergebnis der Multiplikation zu der ersten Repräsentation R1 addiert. Auf diese Weise wird die synthetische dritte Repräsentation R3* erzeugt. In dem in Fig. 1 gezeigten Beispiel beträgt der Verstärkungsfaktor $\alpha$=3, d.h. die Differenz R2-R1 wird dreimal zu der ersten Repräsentation R1 addiert.

Die dritte Repräsentation R3* kann einer Normalisierung unterzogen werden, das heißt, die Grauwerte/Farbwerte können mit einem Faktor multipliziert werden, so dass der Grauwert/Farbwert mit dem höchsten Wert beispielsweise durch den Grauton/Farbton "weiß" und der Grauwert/Farbwert mit dem geringsten Wert beispielsweise durch den Grauton/Farbton "schwarz" repräsentiert wird.

Das in Bezug zu Fig. 1 beschriebene mechanistische Modell basiert auf der Annahme, dass die durch Grauwerte oder Farbwerte repräsentierte Signalintensität in einer Repräsentation des Untersuchungsbereichs linear von der Menge des applizierten Kontrastmittels abhängt. Dies ist insbesondere bei vielen MRT-Untersuchungen der Fall. Die lineare Abhängigkeit erlaubt es, den Kontrast durch Variation des Verstärkungsfaktors $\alpha$ zu variieren. Ein Verstärkungsfaktor von $\alpha$ = 2 bedeutet also, dass die dritte Menge an Kontrastmittel der doppelten zweiten Menge entspricht.

Es sei angemerkt, dass statt einer linearen Abhängigkeit dem mechanistischen Modell auch eine andere Abhängigkeit zugrunde gelegt werden kann. Die Abhängigkeit kann empirisch ermittelt werden.

In dem in Fig. 1 gezeigten Beispiel besteht das zweite Teilmodell TM2 also aus mathematischen Operationen, die Subtraktionen, Multiplikationen und Additionen auf Basis der Grauwerte/Farbwerte der einzelnen Bildelemente (z.B. Pixel, Voxel) ausführen.

Die zuvor beschriebenen Operationen zur Erzeugung einer synthetischen dritten Repräsentation mit einer variablen Kontrastverstärkung können in analoger Weise auch in anderen Räumen auf Basis anderer Repräsentationen als Ortsraum-Repräsentationen durchgeführt werden, zum Beispiel im Frequenzraum auf Basis von Frequenzraum-Repräsentationen.

Fig. 2 zeigt schematisch eine weitere Ausführungsform eines zweiten Teilmodells.

Das in Fig. 2 dargestellte zweite Teilmodell TM2 ist konfiguriert, auf Basis einer ersten Repräsentation $R1^F$ eines Untersuchungsbereichs eines Untersuchungsobjekts im Frequenzraum und einer zweiten Repräsentation $R2^F$ des Untersuchungsbereichs des Untersuchungsobjekts im Frequenzraum eine synthetische dritte Repräsentation $R3^{*F}$ des Untersuchungsbereichs des Untersuchungsobjekts im Frequenzraum zu erzeugen.

Die Repräsentationen $R1^F$ und $R2^F$ des Untersuchungsbereichs des Untersuchungsobjekts im Frequenzraum können beispielsweise aus den entsprechenden Ortsraum-Repräsentationen $R1^I$ und $R2^I$ gewonnen werden.

Die erste Repräsentation $R1^I$ repräsentiert den Untersuchungsbereich im Ortsraum ohne Kontrastmittel oder nach der Applikation einer ersten Menge eines Kontrastmittels. Der in Fig. 2 gezeigte Untersuchungsbereich umfasst eine Leber eines Schweines. Die erste Repräsentation $R1^I$ ist eine magnetresonanztomographische Aufnahme.

Die erste Ortsraum-Repräsentation $R1^I$ lässt sich durch eine Transformation *T*, zum Beispiel eine Fourier-Transformation, in die erste Repräsentation $R1^F$ des Untersuchungsbereich im Frequenzraum umwandeln. Die erste Frequenzraum-Repräsentation $R1^F$ repräsentiert denselben Untersuchungsbereich desselben Untersuchungsobjekts wie die erste Ortsraum-Repräsentation $R1^I$, ebenfalls ohne Kontrastmittel oder nach der Applikation der ersten Menge des Kontrastmittels.

Die erste Frequenzraum-Repräsentation $R1^F$ lässt sich mittels einer Transformation $T^{-1}$ in die erste Ortsraum-Repräsentation $R1^I$ umwandeln, zum Beispiel durch eine inverse Fourier-Transformation. Die Transformation $T^{-1}$ ist die zur Transformation *T* inverse Transformation.

Die zweite Repräsentation $R2^I$ repräsentiert denselben Untersuchungsbereich desselben Untersuchungsobjekts wie die erste Repräsentation $R1^I$ im Ortsraum. Die zweite Ortsraum-Repräsentation $R2^I$ repräsentiert den Untersuchungsbereich nach der Applikation einer zweiten Menge des Kontrastmittels. Die zweite Menge ist größer als die erste Menge (wobei die erste Menge wie beschrieben auch Null sein kann). Die zweite Repräsentation $R2^I$ ist ebenfalls eine magnetresonanztomographische Aufnahme. Als Kontrastmittel wurde in dem in Fig. 2 dargestellten Beispiel das Dinatriumsalz der Gadoxetsäure (Gd-EOB-DTPA Dinatrium) als ein hepatobiliäres MRT-Kontrastmittel verwendet.

Die zweite Ortsraum-Repräsentation $R2^I$ lässt sich durch die Transformation *T* in die zweite Repräsentation $R2^F$ des Untersuchungsbereichs im Frequenzraum umwandeln. Die zweite Frequenzraum-Repräsentation $R2^F$ repräsentiert denselben Untersuchungsbereich desselben Untersuchungsobjekts wie die zweite

Ortsraum-Repräsentation $R2^I$, ebenfalls nach der Applikation der zweiten Menge des Kontrastmittels.

Die zweite Frequenzraum-Repräsentation $R2^F$ lässt sich mittels der Transformation $T^{-1}$ in die zweite Ortsraum-Repräsentation $R2^I$ umwandeln.

In dem in Fig. 2 gezeigten Beispiel werden die erste Frequenzraum-Repräsentation $R1^F$ und die zweite Frequenzraum-Repräsentation $R2^F$ dem zweiten Teilmodell TM2 zugeführt. Das zweite Teilmodell TM2 erzeugt auf Basis der ersten Frequenzraum-Repräsentation $R1^F$ und der zweiten Frequenzraum-Repräsentation $R2^F$ die synthetische dritte Frequenzraum-Repräsentation $R3^{*F}$. Die synthetische dritte Frequenzraum-Repräsentation $R3^{*F}$ kann durch eine Transformation $T^{-1}$ (z.B. eine inverse Fourier-Transformation) in eine synthetische dritte Ortsraum-Repräsentation $R3^{*I}$ überführt werden.

Das in Fig. 2 gezeigte zweite Teilmodell TM2 umfasst nicht die Transformation *T*, die die erste Ortsraum-Repräsentation $R1^I$ in die erste Frequenzraum-Repräsentation $R1^F$ überführt und die zweite Ortsraum-Repräsentation $R2^I$ in die zweite Frequenzraum-Repräsentation $R2^F$ überführt. Ebenso umfasst das in Fig. 2 gezeigte zweite Teilmodell TM2 nicht die Transformation $T^{-1}$, die die synthetische dritte Frequenzraum-Repräsentation $R3^{*F}$ in die synthetische dritte Ortsraum-Repräsentation $R3^{*I}$ überführt. Es ist aber denkbar, dass die Transformation *T*und/oder die Transformation $T^{-1}$ Bestandteil(e) des zweiten Teilmodells TM2 sind, d.h. es ist denkbar, dass das zweite Teilmodell TM2 die Transformation *T* und/oder die Transformation $T^{-1}$ ausführt.

Mittels des zweiten Teilmodells TM2 wird die erste Frequenzraum-Repräsentation $R1^F$ von der zweiten Frequenzraum-Repräsentation $R2^F$ subtrahiert ($R2^F - R1^F$). Das Ergebnis ist eine Repräsentation der durch Kontrastmittel im Untersuchungsbereich hervorgerufenen Signalintensitätsverteilung im Frequenzraum.

Die Differenz $R2^F - R1^F$ wird mit einer Gewichtsfunktion WF multipliziert, die niedrige Frequenzen höher gewichtet als hohe Frequenzen. Im vorliegenden Fall werden die Amplituden der Grundschwingungen mit einem umso größeren Gewichtsfaktor multipliziert desto kleiner die Frequenz ist. Dieser Schritt ist ein optionaler Schritt, der ausgeführt werden kann, um das Signal-RauschVerhältnis in der synthetischen dritten Repräsentation zu erhöhen, insbesondere dann, wenn der Verstärkungsfaktor $\alpha$ größere Werte (z.B. Werte größer als 3, 4 oder 5) annimmt. Das Ergebnis dieser frequenzabhängigen Gewichtung ist die gewichtete Repräsentation $(R2^F\text{-}R1^F)^W$.

Kontrastinformationen werden in einer Frequenzraumdarstellung durch niedrige Frequenzen repräsentiert, während die höheren Frequenzen Informationen zu Feinstrukturen repräsentieren. Durch eine solche Gewichtung werden somit diejenigen Frequenzen, die einen höheren Beitrag zum Kontrast leisten, mit einem höheren Gewicht versehen werden als diejenigen Frequenzen, die einen geringeren Beitrag zum Kontrast leisten. Bildrauschen ist typischerweise in der Frequenzdarstellung gleichverteilt. Die frequenzabhängige Gewichtsfunktion hat die Wirkung eines Filters. Der Filter erhöht das Signalzu-Rauschen-Verhältnis, da die spektrale Rauschdichte für hohe Frequenzen verringert wird.

Bevorzugte Gewichtsfunktionen sind Hann-Funktion (auch als Hann Window bezeichnet) und Poisson-Funktion (Poisson Window).

Beispiele für weitere Gewichtsfunktionen sind zum Beispiel zu finden unter https://de.wikipedia.org/wiki/Fensterfunktion#Beispiele_von_Fensterfunktionen; F.J. Harris et al.: On the Use of Windows for Harmonic Analysis with the Discrete Fourier Transform, Proceedings oft he IEEE, VoL. 66, N. 1, 1978; https://docs.scipy.org/doc/scipy/reference/signal.windows.html; K. M. M Prabhu: Window Functions and Their Applications in Signal Processing, CRC Press, 2014, 978-1-4665-1583-3).

Die gewichtete Differenz $(R2^F\text{-}R1^F)^W$ wird in einem nächsten Schritt mit einem Verstärkungsfaktor $\alpha$ multipliziert und zu der ersten Frequenzraum-Repräsentation $R1^F$ addiert. Das Ergebnis ist eine synthetische dritte Repräsentation $R3^{*F}=R1^F+\alpha\cdot(R2^F\text{-}R1^F)^W$ des Untersuchungsbereichs des Untersuchungsobjekts im Frequenzraum. Die synthetische dritte Frequenzraum-Repräsentation $R3^{*F}$ wird in einem weiteren Schritt durch die Transformation $T^{-1}$ (z.B. eine inverse Fouriertransformation) in die synthetische dritte Repräsentation $R3^{*I}$ des Untersuchungsbereichs des Untersuchungsobjekts im Ortsraum umgewandelt.

Die synthetische dritte Repräsentation $R3^{*I}$ repräsentiert den Untersuchungsbereich des Untersuchungsobjekts nach Applikation einer dritten Menge des Kontrastmittels. Die dritte Menge ist abhängig von dem Verstärkungsfaktor $\alpha$. Ist der Verstärkungsfaktor beispielsweise 3 und ist die durch die Grauwerte/Farbwerte repräsentierte Signalintensitätsverteilung linear von der Menge des Kontrastmittels abhängig, dann entspricht die dritte Menge dem Dreifachen der Differenz der ersten Menge von der zweiten Menge.

Wird ein zweites Teilmodell TM2, wie es in Fig. 1 dargestellt ist, verwendet, um die dritte Repräsentation zu erzeugen, dann wird durch einen Verstärkungsfaktor, der größer als 1 ist ($\alpha > 1$) nicht nur der Kontrast verstärkt, sondern es wird in demselben Umfang auch Rauschen verstärkt. Ein zweites Teilmodell TM2, wie es in Fig. 2 dargestellt ist, kann ein solches Rauschen durch die Gewichtung mit der Gewichtsfunktion im Frequenzraum zu einem gewissen Teil reduzieren.

Der Verstärkungsfaktor $\alpha$ kann ein Modellparameter des zweiten Teilmodells sein, der von dem ersten Teilmodell bereitgestellt (ermittelt, vorhergesagt) wird.

Ebenso können ein oder mehrere Parameter der Gewichtsfunkton Modellparameter des zweiten Teilmodells sein, die von dem ersten Teilmodell bereitgestellt (ermittelt, vorhergesagt) werden.

Ist die Gewichtsfunktion beispielsweise eine

zweidimensionale Gauß-Funktion, dann hat sie die Formel

$$wf(x,y) = \frac{1}{2\pi\sigma^2} e^{-\frac{x^2+y^2}{2\sigma^2}}$$

Dabei ist *wf* der frequenzabhängige Gewichtsfaktor, mit dem die Amplituden der Grundschwingungen der Frequenzraum-Repräsentation $R2^F$ - $R1^F$ multipliziert werden. *x* sind die die Frequenzen entlang der horizontalen Achse und *y* sind die Frequenzen entlang der vertikalen Achse. $\pi$ ist die Kreiszahl und $\sigma$ ist die Standardabweichung. Die Standardabweichung $\sigma$ kann ein Modellparameter des zweiten Teilmodells sein, der von dem ersten Teilmodell ermittelt (bereitgestellt) wird.

Für andere Gewichtsfunktionen wie beispielsweise die Hann-Funktion (Hann Window) und Poisson-Funktion (Poisson Window) gilt analog, dass auch deren Parameter, die die jeweilige Gewichtsfunktion charakterisieren, Modellparameter des zweiten Teilmodells sein können, die von dem ersten Teilmodell ermittelt (bereitgestellt) werden.

Der mindestens eine von dem ersten Teilmodell ermittelte Modellparameter kann beispielsweise einen Parameter oder mehrere Parameter der frequenzabhängigen Gewichtsfunktion umfassen, der/die bestimmt/bestimmen, mit welchem Gewichtsfaktor die Amplituden der einzelnen Frequenzen der Grundschwingungen multipliziert wird. Der mindestens eine von dem ersten Teilmodell ermittelte Modellparameter kann beispielsweise mindestens einen Parameter umfassen, der die Breite der Gewichtsfunktion (Fensterfunktion) bestimmt, die Steigung, mit der die Gewichtsfunktion mit zunehmender Frequenz absinkt und/oder andere Eigenschaften der Gewichtsfunktion.

Es ist auch denkbar, dass ein Modellparameter bestimmt, welche Gewichtsfunktion von dem zweiten Teilmodell verwendet wird, um eine frequenzabhängige Filterung vorzunehmen. Es ist denkbar, dass während des Trainingsverfahrens verschiedene Gewichtsfunktionen "ausprobiert" werden und das Modell des maschinellen Lernens trainiert wird, diejenige Gewichtsfunktion auszuwählen, die zu einer bestmöglichen Vorhersage der dritten Repräsentation führt.

Das erste Teilmodell kann trainiert werden, mindestens einen Modellparameter (wie beispielsweise den Verstärkungsfaktor $\alpha$, Parameter einer Gewichtsfunktion und/oder weitere/andere Modellparameter) zu ermitteln, der zu einer synthetischen dritten Repräsentation mit solchen Eigenschaften führt, die von Zieldaten bestimmt werden. Die Zieldaten müssen den mindestens einen Modellparameter also nicht selbst umfassen. Die Zieldaten können eine (gemessene) dritte Repräsentation umfassen. Das erste Modell kann trainiert werden, den mindestens einen Modellparameter so zu wählen, dass die synthetische dritte Repräsentation der (gemessenen) dritten Repräsentation (*ground truth*) möglichst nahekommt.

Dies ist beispielhaft und schematisch in Fig. 3 gezeigt.

Fig. 3 zeigt beispielhaft und schematisch das Trainieren eines Modells des maschinellen Lernens zum Erzeugen einer synthetischen Repräsentation eines Untersuchungsbereichs eines Untersuchungsobjekts.

Das Trainieren erfolgt anhand von Trainingsdaten TD. Die Trainingsdaten TD umfassen für jedes Referenzobjekt einer Vielzahl an Referenzobjekten (i) eine erste Referenz-Repräsentation RR1 eines Referenzbereichs des Referenzobjekts und eine zweite Referenz-Repräsentation RR2 des Referenzbereichs des Referenzobjekts als Eingabedaten und (ii) eine dritte Referenz-Repräsentation RR3 des Referenzbereichs des Referenzobjekts als Zieldaten. In Fig. 1 ist nur ein Datensatz eines Referenzobjekts gezeigt. Das Referenzobjekt ist ein Mensch und der Referenzbereich umfasst die Lunge des Menschen.

Der Begriff "Referenz" wird in dieser Beschreibung verwendet, um die Phase des Trainierens des Modells des maschinellen Lernens von der Phase des Verwendens des trainierten Modells zum Erzeugen einer synthetischen Repräsentation zu unterscheiden. Der Begriff "Referenz" hat ansonsten keinerlei einschränkende Bedeutung. Ein "Referenzobjekt" ist ein Objekt, von dem Daten (z.B. Referenz-Repräsentationen) zum Trainieren des Modells des maschinellen Lernens verwendet werden. Daten eines Untersuchungsobjekts werden hingegen verwendet, um das trainierte Modell zur Vorhersage zu nutzen. Der Begriff "(Referenz-)Repräsentation" bedeutet, dass die entsprechende Aussage sowohl für eine Repräsentation eines Untersuchungsobjekts als auch für eine Referenz-Repräsentation eines Referenzobjekts gilt. Ansonsten gelten alle Aussagen, die in dieser Beschreibung in Bezug auf ein Untersuchungsobjekt gemacht werden, in gleicher Weise auch für jedes Referenzobjekt und umgekehrt. Jedes Referenzobjekt ist, wie das Untersuchungsobjekt, üblicherweise ein Lebewesen, vorzugsweise ein Säugetier, ganz besonders bevorzugt ein Mensch. Der "Referenzbereich" ist ein Teil des Referenzobjekts. Der Referenzbereich entspricht üblicherweise (aber nicht notwendigerweise) dem Untersuchungsbereich des Untersuchungsobjekts. Mit anderen Worten: für den Fall, dass der Untersuchungsbereich ein Organ oder ein Teil eines Organs (z.B. die Leber oder ein Teil der Leber) des Untersuchungsobjekts ist, ist der Referenzbereich eines jeden Referenzobjekts vorzugsweise das entsprechende Organ oder der entsprechende Teil des Organs des jeweiligen Referenzobjekts. Ansonsten gelten alle Aussagen, die in dieser Beschreibung in Bezug auf einen Untersuchungsbereich gemacht werden, in gleicher Weise auch für den Referenzbereich und umgekehrt.

Die erste Referenz-Repräsentation RR1, die zweite Referenz-Repräsentation RR2 und die dritte Referenz-Repräsentation RR3 sind radiologische Aufnahmen; es können beispielsweise MRT-Aufnahmen und/oder CT-Aufnahmen und/oder Röntgenaufnahmen sein.

Die erste Referenz-Repräsentation RR1 repräsentiert den Referenzbereich des Referenzobjekts ohne Kontrastmittel oder nach Applikation einer ersten Menge eines Kontrastmittels. Die zweite Referenz-Repräsentation RR2 repräsentiert den Referenzbereich des Referenzobjekts nach Applikation einer zweiten Menge des Kontrastmittels. Die zweite Menge ist größer als die erste Menge (wobei die erste Menge wie beschrieben auch Null sein kann). Die dritte Referenz-Repräsentation RR3 repräsentiert den Referenzbereich des Referenzobjekts nach Applikation einer dritten Menge eines Kontrastmittels. Die dritte Menge ist verschieden von der zweiten Menge, vorzugsweise ist die dritte Menge größer als die zweite Menge.

Ist die zweite Menge kleiner als die Standardmenge, so kann die dritte Menge beispielsweise gleich der Standardmenge sein. Es ist aber auch möglich, dass die dritte Menge größer als die Standmenge ist.

Das Modell des maschinellen Lernens M umfasst zwei Teilmodelle TM1 und TM2. Das erste Teilmodell TM1 ist konfiguriert und wird trainiert, auf Basis der ersten Referenz-Repräsentation RR1 und der zweiten Referenz-Repräsentation RR2 (und auf Basis von Modellparametern des ersten Teilmodells TM1), mindestens einen Modellparameter MP für das zweite Teilmodell TM2 zu ermitteln (vorherzusagen). Das zweite Teilmodell TM2 ist konfiguriert, auf Basis der ersten Referenz-Repräsentation RR1 und/oder der zweiten Referenz-Repräsentation RR2 eine synthetische dritte Referenz-Repräsentation RR3* zu erzeugen.

Das erste Teilmodell TM1 kann beispielsweise ein künstliches neuronales Netzwerk sein (wie unten näher beschrieben) oder ein solches umfassen. Das zweite Teilmodell TM2 kann ein mechanistisches Modell sein, wie es in Bezug zu Fig. 1 oder Fig. 2 beschrieben wurde, oder ein anderes mechanistisches Modell sein. Das zweite Teilmodell TM2 kann ein Modell sein, das in EP22207079.9, EP22207080.7 und/oder EP23168725.2 offenbart ist.

Die erste Referenz-Repräsentation RR1 und die zweite Referenz-Repräsentation RR2 werden dem ersten Teilmodell TM1 zugeführt und das erste Teilmodell TM1 liefert den mindestens einen Modellparameter MP an das zweite Teilmodell TM2. Der mindestens eine Modellparameter MP kann beispielsweise der Verstärkungsfaktor $\alpha$ sein oder diesen umfassen. Der mindestens eine Modellparameter MP kann einen oder mehrere Parameter einer Gewichtsfunktion und/oder einer Filterfunktion sein oder einen oder mehrere solcher Parameter umfassen.

Es ist möglich, eine Co-Registrierung der ersten Referenz-Repräsentation RR1 und der zweiten Referenz-Repräsentation RR2 durchzuführen, bevor sie dem ersten Teilmodell TM1 zugeführt werden. Die "Co-Registrierung" (im Stand der Technik auch "Bildregistrierung" genannt) dient dazu, zwei oder mehrere Ortsraumdarstellungen desselben Untersuchungsbereichs bestmöglich in Übereinstimmung miteinander zu bringen. Dabei wird eine der Ortsraumdarstellungen als Referenzbild festgelegt, die andere wird Objektbild genannt. Um diese optimal an das Referenzbild anzupassen, wird eine ausgleichende Transformation berechnet.

Es ist auch möglich, eine Co-Registrierung von Repräsentationen im Frequenzraum durchzuführen, wobei hierbei zu beachten ist, dass sich eine Translation im Ortsraum als eine additive lineare Phasenrampe im Frequenzraum darstellt. Skalierung und Rotation hingegen bleiben bei der Fourier- und inversen Fourier-Transformation erhalten - eine Skalierung und Rotation im Frequenzraum ist auch eine Skalierung und Rotation im Ortsraum (siehe z.B.: S. Skare: Rigid Body Image Realignment in Image Space vs. k-Space, ISMRM SCIENTIFIC WORKSHOP on Motion Correction, 2014, https://cds.ismrm.org/protected/Motion_14/Program/Syllabus/Skare.pdf).

Das zweite Teilmodell MP2 nimmt den mindestens einen Modelparameter entgegen und erzeugt die synthetische dritte Referenz-Repräsentation RR3*. Die synthetische dritte Referenz-Repräsentation RR3* kann mit der dritten Referenz-Repräsentation RR3 der Zieldaten verglichen werden. In dem in Fig. 3 gezeigten Beispiel wird eine Fehlerfunktion LF verwendet, um Abweichungen zwischen der synthetischen dritten Referenz-Repräsentation RR3* und der dritten Referenz-Repräsentation RR3 zu quantifizieren. Die Abweichungen können verwendet werden, um Modellparameter des ersten Teilmodells TM1 in einem Optimierungsverfahren (z.B. einem Gradientenverfahren) so zu modifizieren, dass die Abweichungen minimiert werden.

Der beschriebene Vorgang wird für eine Vielzahl an Referenz-Repräsentation einer Vielzahl an Referenzobjekten ein- oder mehrfach durchgeführt. Das Training kann beendet werden, wenn der mittels der Fehlerfunktion errechnete Fehler ein vordefiniertes Minimum erreicht und/oder sich der Fehler durch Modifizieren von Modellparametern des ersten Teilmodells nicht mehr reduzieren lässt.

Aus Fig. 3 wird deutlich, dass zum Trainieren des Modells M des maschinellen Lernens der von dem ersten Teilmodell TM1 zu ermittelnde mindestens eine Modellparameter MP nicht bekannt sein muss. Mit anderen Worten: der mindestens eine Modellparameter MP muss kein Bestandteil der Trainingsdaten/Zieldaten sein. Stattdessen umfassen die Trainingsdaten die (gemessene) dritte Referenz-Repräsentation RR3 als Zieldaten (*ground truth*). Das erste Teilmodell TM1 schlägt mindestens einen Modellparameter MP vor, auf Basis dessen das zweite Teilmodell TM2 die synthetische dritte Referenz-Repräsentation RR3* erzeugt. Treten Abweichungen zwischen der synthetischen dritten Referenz-Repräsentation RR3* und der (gemessenen) dritten Referenz-Repräsentation RR3 auf, so werden diese mittels der Fehlerfunktion LF erkannt und quantifiziert. Modellparameter des ersten Teilmodells TM1 werden modifiziert, um die Abweichungen zu reduzieren/minimieren.

Aus Fig. 3 wird ferner deutlich, dass das zweite Teilmodell TM2 selbst nicht trainiert wird. Es wird lediglich das erste Teilmodell TM1 trainiert. Wenn es Abweichungen zwischen der synthetischen dritten Referenz-Repräsentation RR3* und der (gemessenen) dritten Referenz-Repräsentation RR3 gibt, werden lediglich Modellparameter des ersten Teilmodells TM1 in einem Optimierungsverfahren (z.B. einem Gradientenverfahren) modifiziert, um die Abweichungen zu reduzieren. Durch Modifizieren der Modellparameter des ersten Teilmodells TM1 ergibt sich üblicherweise auch eine Änderung des von dem ersten Teilmodell TM1 ermittelten mindestens einen Modellparameter MP. Der mindestens eine Modellparameter MP ist aber das Ergebnis einer Anpassung der Modellparameter des ersten Teilmodells TM1 während des Trainings und keine Trainingsmaßnahme.

Dennoch kann das zweite Teilmodell TM2 in das Training einbezogen werden: ist das zweite Teilmodell TM2 differenzierbar, dann kann das Modell M des maschinellen Lernens Ende-zu-Ende trainiert werden.

Der Vorteil der Aufteilung des Modells des maschinellen Lernens in mindestens zwei Teilmodelle besteht darin, dass das zweite Teilmodell auf einem mechanistischen Ansatz zur Erzeugung der synthetischen dritten Repräsentation basiert und damit nachvollziehbare Ergebnisse liefert. Die Erzeugung der synthetischen dritten Repräsentation bewegt sich in dem von dem zweiten Teilmodell vorgegebenen Rahmen. Es können keine synthetischen dritten Repräsentationen erzeugt werden, die nicht im Einklang mit dem mechanistischen Modell sind. Es wird nur eine im Vergleich zu einem künstlichen neuronalen Netz mit einer Vielzahl an Knoten und/oder Schichten geringe Zahl an Modellparametern modifiziert, um eine mögliche fehlerfreie Übereinstimmung zwischen der synthetischen dritten Repräsentation und der (gemessenen) dritten Repräsentation zu erreichen. Das erste Teilmodell ermittelt, wenn es trainiert wurde, diejenigen Modellparameter für das zweite Teilmodell, die zu einer optimalen Vorhersage führen, d.h. einer Vorhersage, bei der die synthetische Repräsentation einer gemessenen Repräsentation bestmöglich nahekommt.

Der von dem ersten Teilmodell ermittelte mindestens eine Modellparameter kann ausgegeben (z.B. auf einem Monitor angezeigt und/oder auf einem Drucker ausgedruckt) werden, damit ein Nutzer den mindestens einen ermittelten Modellparameter prüfen kann. Der Nutzer kann somit prüfen, ob der von dem ersten Teilmodell ermittelte mindestens eine Modellparameter innerhalb erwarteter Grenzen liegt und somit sinnvoll ist.

Eine solche Prüfung, ob der von dem ersten Teilmodell ermittelte mindestens eine Modellparameter innerhalb vordefinierter Grenzen liegt, kann auch automatisiert erfolgen. Automatisiert bedeutet ohne Zutun eines Menschen. Der von dem ersten Teilmodell ermittelte mindestens eine Modellparameter kann mit einem oder mehreren vordefinierten Grenzwerten verglichen werden. Liegt der mindestens eine Modellparameter oberhalb eines vordefinierten oberen Grenzwertes oder unterhalb eines vordefinierten unteren Grenzwertes kann eine Ausgabe erfolgen, dass der von dem ersten Teilmodell ermittelte mindestens eine Modellparameter außerhalb eines definierten Bereichs liegt und die synthetische dritte Repräsentation damit fehlerhaft sein kann.

Ein oder mehrere Grenzwerte können beispielsweise auf Basis physikalischer Gesetzmäßigkeiten und/oder statistischer Berechnungen und/oder empirisch festgelegt (vordefiniert) werden. Wenn beispielsweise die erste Referenz-Repräsentation den Untersuchungsbereich ohne Kontrastmittel, die zweite Referenz-Repräsentation den Untersuchungsbereich mit einer zweiten Menge an Kontrastmittel, die dritte Referenz-Repräsentation den Untersuchungsbereich mit einer dritten Menge an Kontrastmittel repräsentiert und die dritte Menge größer als die zweite Menge ist, dann muss der zuvor beschrieben Verstärkungsfaktor $\alpha$ größer als 1 sein. Umfasst der von dem ersten Teilmodell ermittelte mindestens eine Modellparameter einen solchen Verstärkungsfaktor und ist dieser kleiner als 1, so bewegt sich das zweite Teilmodell außerhalb physikalischer Gesetze und die von dem zweiten Teilmodell erzeugte synthetische dritte Referenz-Repräsentation kann fehlerhaft sein.

Wie in dieser Offenbarung beschrieben, ist das erste Teilmodell konfiguriert und wurde trainiert, auf Basis einer ersten (Referenz-)Repräsentation und einer zweiten (Referenz-)Repräsentation eines Untersuchungsbereichs (bzw. Referenzbereichs) eines Untersuchungsobjekts (bzw. Referenzobjekts) mindestens einen Modellparameter für das zweite Teilmodell zu ermitteln. In diesem Zusammenhang bedeutet der Begriff "auf Basis von", dass die erste (Referenz-)Repräsentation und die zweite (Referenz-)Repräsentation als Eingabedaten in das erste Teilmodell eingegeben werden und das erste Teilmodell in Reaktion auf diese Eingabe den mindestens einen Modellparameter bereitstellt (z.B. ausgibt), so dass das zweite Teilmodell diesen mindestens einen Modellparameter nutzen kann.

Das zweite Teilmodell ist konfiguriert, auf Basis der ersten (Referenz-)Repräsentation und/oder der zweiten (Referenz-)Repräsentation und des von dem ersten Teilmodell ermittelten mindestens einen Modellparameters eine synthetische dritte Referenz-Repräsentation zu erzeugen. Das bedeutet, dass die erste (Referenz-)Repräsentation und/oder der zweite (Referenz-) Repräsentation als Eingabedaten in das zweite Teilmodell eingegeben werden und das zweite Teilmodell in Reaktion auf diese Eingabe die synthetische dritte (Referenz-)Repräsentation erzeugt und bereitstellt (z.B. ausgibt). Dabei ist der von dem ersten Teilmodell ermittelte mindestens eine Modellparameter ein Modellparameter des zweiten Teilmodells, der einen Einfluss darauf hat, wie die synthetische dritte (Referenz-)Repräsentation erzeugt wird.

Das erste Teilmodell kann ein künstliches neuronales Netzwerk sein oder ein solches umfassen.

Ein "künstliches neuronales Netzwerk" umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen der ersten und der zweiten (Referenz-)Repräsentation. Es kann beispielsweise ein Eingangsneuron für jedes Pixel oder Voxel einer (Referenz-)Repräsentation geben, falls es sich bei der (Referenz-)Repräsentation um eine Ortsraumdarstellung in Form einer Rastergrafik handelt, oder ein Eingangsneuron für jede Frequenz, die in der (Referenz-)Repräsentation vorhanden ist, falls es sich bei der (Referenz-)Repräsentation um eine Frequenzraumdarstellung handelt. Es können zusätzliche Eingangsneuronen für zusätzliche Eingabedaten (z.B. Informationen zum Untersuchungsbereich/Referenzbereich, zum Untersuchungsobjekt/Referenzobjekt, zu Bedingungen, die bei der Erzeugung der (Referenz-)Repräsentation herrschten, Informationen zu dem Zustand, den die (Referenz-)Repräsentation repräsentiert, und/oder Informationen zu dem Zeitpunkt oder der Zeitspanne zu/in der die (Referenz-)Repräsentation erzeugt worden ist) vorhanden sein.

Die Ausgangsneuronen dienen dazu, den mindestens einen Modellparameter für das zweite Teilmodell auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Das künstliche neuronale Netzwerk kann ein Convolutional Neural Network (kurz: CNN) sein oder ein solches umfassen.

Ein Convolutional Neural Network ist in der Lage, Eingabedaten in Form einer Matrix zu verarbeiten. Dies ermöglicht es, als Matrix dargestellte digitale radiologische Aufnahmen (z.B. Breite x Höhe x Farbkanäle) als Eingabedaten zu verwenden. Ein normales neuronales Netzwerk z.B. in Form eines Multi-Layer-Perceptrons (MLP) benötigt dagegen einen Vektor als Eingabe, d.h. um eine radiologische Aufnahme als Eingabe zu verwenden, müssten die Pixel oder Voxel der radiologischen Aufnahme in einer langen Kette hintereinander ausgerollt werden. Dadurch sind normale neuronale Netzwerke z.B. nicht in der Lage, Objekte in einer radiologischen Aufnahme unabhängig von der Position des Objekts in der Aufnahme zu erkennen. Das gleiche Objekt an einer anderen Position in der Aufnahme hätte einen völlig anderen Eingabevektor.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von vollständig verbundenen Neuronen (Dense / Fully Connected Layer).

Das erste Teilmodell kann beispielsweise eine Architektur aufweisen, die auf der in Fig. 5 von WO2019/074938A1 dargestellten Architektur basiert: Eingabeschichten für die erste und die zweite (Referenz)-Repräsentation gefolgt von einer Reihe von Encoder-Schichten können verwendet werden, um die (Referenz-)Repräsentationen bzw. die darin enthaltenen Informationen in einem Merkmalsvektor zu komprimieren. Den Encoder-Schichten können Schichten aus vollständig verbundenen Neuronen (*fully connected layers*) folgen, denen schließlich eine Ausgabeschicht folgt. Die Schichten aus vollständig verbundenen Neuronen können z.B. in Form einer Regression aus dem Merkmalsvektor den mindestens einen Modellparameter berechnen. Die Ausgabeschicht kann so viele Ausgangsneuronen haben, wie Modellparameter von dem ersten Teilmodell für das zweite Teilmodell ermittelt (berechnet) werden.

In Fig. 3 ist ein Trainingsverfahren anhand von Referenz-Repräsentationen im Ortsraum gezeigt. Natürlich ist es ebenso möglich, das Training ganz oder teilweise anhand von Referenz-Repräsentationen im Frequenzraum durchzuführen.

Nach dem Trainieren kann das Modell des maschinellen Lernens der vorliegenden Offenbarung zur Vorhersage verwendet werden. Dies ist beispielhaft und schematisch in Fig. 4 gezeigt. In Fig. 4 ist ein Vorhersageverfahren anhand von Repräsentationen im Ortsraum gezeigt. Natürlich ist es ebenso möglich, die Vorhersage ganz oder teilweise anhand von Repräsentationen im Frequenzraum durchzuführen, insbesondere dann, wenn auch das entsprechende Trainingsverfahren ganz oder teilweise anhand von Referenz-Repräsentationen im Frequenzraum durchgeführt worden ist.

Fig. 4 zeigt beispielhaft und schematisch die Erzeugung einer synthetischen Repräsentation eines Untersuchungsbereichs eines Untersuchungsobjekts mit Hilfe eines trainierten Modells des maschinellen Lernens.

Das trainierte Modell $M^T$ des maschinellen Lernens kann wie in Bezug zu Fig. 3 beschrieben trainiert worden sind. Das hochgestellte $^T$ in dem Bezugszeichen $M^T$ für das Modell $M^T$ des maschinellen Lernens soll kennzeichnen, dass es sich um ein trainiertes Modell handelt. Dies gilt auch für das erste Teilmodell $TM1^T$.

Das trainierte Modell $M^T$ des maschinellen Lernens umfasst ein trainiertes erstes Teilmodell $TM1^T$ und ein zweites Teilmodell TM2. Das trainierte erste Teilmodell $TM1^T$ kann ein künstliches neuronales Netzwerk sein oder ein solches umfassen. Das zweite Teilmodell TM2 kann ein mechanistisches Modell sein, wie es in Bezug zu Fig. 1 oder Fig. 2 beschrieben wurde, oder ein anderes Modell sein. Das zweite Teilmodell TM2 kann ein Modell sein, das in EP22207079.9, EP22207080.7 und/oder EP23168725.2. offenbart ist.

In einem ersten Schritt werden eine erste Repräsentation R1 eines Untersuchungsbereichs eines Untersuchungsobjekts und eine zweite Repräsentation R2 des Untersuchungsbereichs des Untersuchungsobjekts empfangen.

Der Begriff "Empfangen" umfasst sowohl das Abrufen von Repräsentationen als auch das Entgegennehmen von Repräsentationen, die z.B. an das Computersystem der vorliegenden Offenbarung übermittelt werden. Die Repräsentationen können von einem Computertomographen, von einem Magnetresonanztomographen oder von einem Ultraschall-Scanner empfangen werden. Die Repräsentationen können aus einem oder mehreren Datenspeichern ausgelesen und/oder von einem separaten Computersystem übermittelt werden.

Die erste Repräsentation R1 und die zweite Repräsentation R2 werden dem trainierten ersten Teilmodell $TM1^T$ zugeführt.

Es ist möglich, eine Co-Registrierung der ersten Repräsentation R1 und der zweiten Repräsentation R2 durchzuführen, bevor sie dem ersten Teilmodell $TM1^T$ zugeführt werden.

Das Untersuchungsobjekt ist im vorliegenden Fall ein Mensch und der Untersuchungsbereich umfasst die Lunge des Menschen. Der Untersuchungsbereich entspricht in dem vorliegenden Beispiel also dem Referenzbereich beim Trainieren des Modells des maschinellen Lernens wie in Bezug zu Fig. 3 beschrieben.

Die erste Repräsentation R1 und die zweite Repräsentation R2 sind radiologische Aufnahmen; es können beispielsweise MRT-Aufnahmen und/oder CT-Aufnahmen und/oder Röntgenaufnahmen sein.

Die erste Repräsentation R1 repräsentiert den Untersuchungsbereich des Untersuchungsobjekts ohne Kontrastmittel oder nach Applikation einer ersten Menge eines Kontrastmittels. Die zweite Repräsentation R2 repräsentiert den Untersuchungsbereich des Untersuchungsobjekts nach Applikation einer zweiten Menge des Kontrastmittels. Die zweite Menge ist größer als die erste Menge (wobei die erste Menge wie beschrieben auch Null sein kann).

Das trainierte erste Teilmodell $TM1^T$ ist konfiguriert und wurde trainiert, auf Basis der ersten Repräsentation R1 und der zweiten Repräsentation R2 und auf Basis von Modellparametern mindestens einen Modellparameter MP für das zweite Teilmodell TM2 zu ermitteln. Der mindestens eine Modellparameter MP wird dem zweiten Teilmodell TM2 zugeführt. Das zweite Teilmodell TM2 ist konfiguriert, auf Basis der ersten Repräsentation R1 und/oder der zweiten Repräsentation R2 und auf Basis des mindestens einen Modellparameters MP eine synthetische dritte Repräsentation R3* zu erzeugen.

Die synthetisch dritte Repräsentation R3* repräsentiert den Untersuchungsbereich des Untersuchungsobjekts nach Applikation einer dritten Menge des Kontrastmittels. Die dritte Menge ist verschieden von der zweiten Menge, vorzugsweise ist die dritte Menge größer als die zweite Menge. Die dritte Menge wird durch das zweite Teilmodell TM2 und den mindestens einen Modellparameter MP festgelegt. Die dritte Menge hängt davon ab, wofür das trainierte Modell $M^T$ des maschinellen Lernens trainiert wurde.

Die synthetisch dritte Repräsentation R3* kann ausgegeben (z.B. auf einem Monitor angezeigt oder über einen Drucker ausgedruckt) und/oder in einem Datenspeicher gespeichert und/oder an ein separates Computersystem übermittelt werden.

Es ist auch möglich, das trainierte erste Teilmodell $TM1^T$ nach dem Training zu verwerfen und die erste Repräsentation R1 und die zweite Repräsentation R2 direkt dem zweiten Teilmodell TM2 zuzuführen, um die synthetische dritte Repräsentation R3* zu erzeugen. Dies gilt insbesondere dann, wenn stets die gleiche Kontrastverstärkung erzielt werden soll, die erste Menge, die zweite Menge und die dritte Menge des Kontrastmittels für alle Untersuchungsobjekte gleich sein sollen und der mindestens eine Modellparameter direkt oder indirekt die dritte Menge des Kontrastmittels angibt. In einem solchen Fall kann der nach dem Trainieren des Modells ($M^T$) des maschinellen Lernens ermittelte mindestens eine Modellparameter MP als fester Parameter in das zweite Teilmodell TM2 eingegeben werden und muss nicht für jeden Satz an neuen Eingabedaten neu ermittelt werden.

Wie bereits beschrieben, kann das Modell des maschinellen Lernens der vorliegenden Erfindung neben dem ersten Teilmodell und dem zweiten Teilmodell ein oder mehrere weitere Teilmodelle umfassen.

Um Rauschen und/oder andere unerwünschte Artefakte in der synthetischen dritten Repräsentation (weiter) zu reduzieren oder zu eliminieren, kann dem zweiten Teilmodell ein drittes Teilmodell nachgeschaltet sein. Das dritte Teilmodell kann der Korrektur der vom zweiten Teilmodell erzeugten synthetischen dritten Repräsentation dienen. Dabei kann der Begriff "Korrektur" das Reduzieren oder Eliminieren von Rauschen und/oder Artefakten bedeuten.

Dem dritten Teilmodell kann die von dem zweiten Teilmodell erzeugte synthetische dritte Repräsentation als Eingabedaten zugeführt werden und das dritte Teilmodell erzeugt auf Basis dieser Eingabedaten und auf Basis von Modellparametern eine korrigierte dritte Repräsentation. Neben der von dem zweiten Teilmodell erzeugten synthetischen dritten Repräsentationen können dem dritten Teilmodell noch weitere Daten als Eingabedaten zugeführt werden (siehe unten).

Das dritte Teilmodell kann ein Modell des maschinellen Lernens sein. Das dritte Teilmodell kann anhand von Trainingsdaten trainiert worden sein, auf Basis einer von dem zweiten Teilmodell erzeugten synthetischen dritten Repräsentation und Modellparametern eine korrigierte (z.B. modifizierte und/oder optimierte) dritte Repräsentation zu erzeugen.

Dies ist schematisch in Fig. 5 dargestellt.

Fig. 5 zeigt beispielhaft und schematisch das Training eines Modells des maschinellen Lernens der vorliegenden Offenbarung.

Das Modell M des maschinellen Lernens umfasst ein erstes Teilmodell TM1, ein zweites Teilmodell TM2 und ein drittes Teilmodell TM3.

Alle Aussagen, die zuvor in Bezug auf das in Fig. 3 gezeigte Trainingsverfahren getroffen wurden, gelten in gleicher Weise auch für das in Fig. 5 gezeigte Trainingsverfahren, mit folgenden Unterschieden:

- Die von dem zweiten Teilmodell TM2 erzeugte synthetische Referenz-Repräsentation RR3* wird dem dritten Teilmodell TM3 zugeführt. Zusätzlich können dem dritten Teilmodell TM3 auch die erste Referenz-Repräsentation RR1 und/oder die zweite Referenz-Repräsentation RR2 und/oder weitere Eingabedaten zugeführt werden.

- Das dritte Teilmodell TM3 ist konfiguriert und wird trainiert, auf Basis der zugeführten Eingabedaten und auf Basis von Modellparametern eine korrigierte dritte Referenz-Repräsentation $RR3^{*C}$ zu erzeugen.

- Eine Fehlerfunktion LF wird verwendet, um Abweichungen zwischen der korrigierten dritten Referenz-Repräsentation $RR3^{*C}$ und der (gemessenen) dritten Referenz-Repräsentation RR3 der Trainingsdaten zu quantifizieren. In einem Optimierungsverfahren (z.B. einem Gradientenverfahren) können die Abweichungen durch Modifizieren von Modellparametern des zweiten Teilmodells und/oder des dritten Teilmodell minimiert werden.

Das dritte Teilmodell kann beispielsweise ein künstliches neuronales Netzwerk sein oder ein solches umfassen. Das dritte Teilmodell kann ein Convolutional Neural Network sein oder ein solches umfassen.

Das dritte Teilmodell kann eine Autoencoder-Architektur aufweisen; z.B. kann das dritte Teilmodell eine Architektur wie das U-Net aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das dritte Teilmodell kann ein *Generative Adversarial Network* (GAN) sein (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das dritte Teilmodell kann insbesondere ein *Generative Adversarial Network* (GAN) für Superauflösung von Bildern (*image super-resolution* (SR)) sein (siehe z.B. C. Ledig et al.: Photo-Realistic Single Image Super-Resolution Using a Generative Adversarial Network, arXiv:1609.04802v5).

Das dritte Teilmodell kann ein Transfomer-Netzwerk sein (siehe z.B. D. Karimi et al.: Convolution-Free Medical Image Segmentation using Transformers, arXiv:2102.13645 [eess.IV]).

In dem in Fig. 5 gezeigten Trainingsverfahren werden das erste Teilmodell TM1 und das dritte Teilmodell TM3 gemeinsam/gleichzeitig trainiert. Das zweite Teilmodell TM2 wird nicht trainiert, da die Modellparameter MP des zweiten Teilmodells TM2 keine lernbaren Modellparameter sind, sondern von dem ersten Teilmodell TM1 ermittelt werden. Es ist aber auch möglich das erste Teilmodell TM1 und das dritte Teilmodell TM3 unabhängig voneinander zu trainieren. Es ist z.B. möglich, zunächst das erste Teilmodell TM1 wie in Bezug zu Fig. 3 beschrieben zu trainieren und die Modellparameter des ersten Teilmodells TM1 dann einzufrieren. In einem nachfolgenden Schritt wird dann das dritte Teilmodell TM3 trainiert. Im Anschluss an ein Training des dritten Teilmodells TM3 kann dann das Gesamtmodell M in einem Ende-zu-Ende-Trainingsverfahren trainiert werden.

Ist das in Fig. 5 gezeigte Modell M des maschinellen Lernens trainiert, kann es zur Erzeugung einer korrigierten synthetischen Repräsentation eines Untersuchungsbereichs eines Untersuchungsobjekt verwendet werden. Dies ist beispielhaft und schematisch in Fig. 6 dargestellt.

Fig. 6 zeigt beispielhaft und schematisch die Erzeugung einer synthetischen Repräsentation eines Untersuchungsbereichs eines Untersuchungsobjekts mit Hilfe eines trainierten Modells des maschinellen Lernens.

Das trainierte Modell $M^T$ des maschinellen Lernens kann wie in Bezug zu Fig. 5 beschrieben trainiert worden sind. Das hochgestellte $^T$ in dem Bezugszeichen $M^T$ für das Modell $M^T$ des maschinellen Lernens soll kennzeichnen, dass es sich um ein trainiertes Modell handelt. Dies gilt auch für das erste Teilmodell $TM1^T$ und das dritte Teilmodell $TM3^{T.}$

Die Aussagen, die oben in Bezug auf das in Fig. 4 gezeigte Verfahren getroffen wurde, treffen in gleicher Weise auch auf das in Fig. 6 gezeigte Verfahren zu, mit den folgenden Unterschieden:

- Die von dem zweiten Teilmodell TM2 erzeugte synthetische dritte Repräsentation R3* wird dem trainierten dritten Teilmodell $TM3^T$ zugeführt. Zusätzlich können dem trainierten dritten Teilmodell $TM3^T$ auch die erste Repräsentation R1 und/oder die zweite Repräsentation R2 des Untersuchungsbereichs des Untersuchungsobjekts und/oder weitere Eingabedaten zugeführt werden.

- Das trainierte dritte Teilmodell $TM3^T$ ist konfiguriert und wurde trainiert, auf Basis der zugeführten Eingabedaten und Modellparametern eine korrigierte dritte Repräsentation $R3^{*C}$ zu erzeugen.

- Die korrigierte dritte Repräsentation $R3^{*C}$ repräsentiert den Untersuchungsbereich des Untersuchungsobjekts nach Applikation der dritten Menge

des Kontrastmittels mit weniger Rauschen und/oder weniger Artefakten als die synthetische dritte Repräsentation R3*.

- Die korrigierte dritte Repräsentation R3*$^{C}$ kann ausgegeben (z.B. auf einem Monitor angezeigt oder über einen Drucker ausgedruckt) und/oder in einem Datenspeicher gespeichert und/oder an ein separates Computersystem übermittelt werden.

Es ist auch möglich, bei einem Modell des maschinellen Lernens der vorliegenden Offenbarung umfassend ein erstes Teilmodell und ein zweiten Teilmodell und optional ein drittes Teilmodell dem ersten Teilmodell ein initiales Teilmodell voranzustellen.

Es ist zum Beispiel möglich, dass das initiale Teilmodell eine Co-Regression der ersten (Referenz-) Repräsentation und der zweiten (Referenz-)Repräsentation vornimmt. Es ist zum Beispiel möglich, dass das initiale Teilmodell eine Normalisierung und/oder eine Segmentierung und/oder Maskierung und/oder eine andere/weitere Transformation/Modifikation der ersten (Referenz-)Repräsentation und der zweiten (Referenz-)Repräsentation vornimmt. Zum Beispiel kann das initiale Teilmodell eine Fourier-Transformation oder eine inverse Fourier-Transformation der ersten und/oder zweiten (Referenz-)Repräsentation durchführen.

Ein solches initiales Teilmodell kann beispielsweise ein künstliches neuronales Netz sein oder ein solches umfassen.

Sind alle Teilmodelle des Modells des maschinellen Lernens differenzierbar, dann kann das Modell des maschinellen Lernens in einem Ende-zu-Ende-Trainingsverfahren trainiert werden.

Fig. 7 zeigt eine Ausführungsform für das Trainieren des Modells des maschinellen Lernens in Form eines Ablaufschemas. Das Trainingsverfahren (100) umfasst die Schritte:

(110) Bereitstellen von Trainingsdaten, wobei die Trainingsdaten für jedes Referenzobjekt einer Vielzahl an Referenzobjekten (i) eine erste Referenz-Repräsentation eines Referenzbereichs des Referenzobjekts und eine zweite Referenz-Repräsentation des Referenzbereichs des Referenzobjekts als Eingabedaten und (ii) eine dritte Referenz-Repräsentation des Referenzbereichs des Referenzobjekts als Zieldaten umfassen,

◦ wobei die erste Referenz-Repräsentation den Referenzbereich ohne Kontrastmittel oder nach Applikation einer ersten Menge eines Kontrastmittels repräsentiert,

◦ wobei die zweite Referenz-Repräsentation den Referenzbereich nach Applikation einer zweiten Menge des Kontrastmittels repräsentiert, wobei die zweite Menge größer als die erste Menge ist,

◦ wobei die dritte Referenz-Repräsentation den Referenzbereich nach Applikation einer dritten Menge des Kontrastmittels repräsentiert, wobei die dritte Menge verschieden von der zweiten Menge, vorzugsweise größer als die zweite Menge ist,

(120) Bereitstellen eines Modells des maschinellen Lernens, wobei das Modell des maschinellen Lernens zwei Teilmodelle umfasst: ein erstes Teilmodell und ein zweites Teilmodell,

(130) Trainieren des Modells des maschinellen Lernens, wobei das Trainieren umfasst:

(131) Zuführen einer ersten Referenz-Repräsentation und einer zweiten Referenz-Repräsentation eines Referenzbereichs eines Referenzobjekts dem ersten Teilmodell, wobei das erste Teilmodell konfiguriert ist, auf Basis der ersten Referenz-Repräsentation, der zweiten Referenz-Repräsentation und Modellparametern mindestens einen Modellparameter für das zweite Teilmodell zu ermitteln,

(132) Empfangen des von dem ersten Teilmodell ermittelten mindestens einen Modellparameters,

(133) Zuführen des ermittelten mindestens einen Modellparameters und der ersten Referenz-Repräsentation und/oder der zweiten Referenz-Repräsentation dem zweiten Teilmodell, wobei das zweite Teilmodell konfiguriert ist, auf Basis der ersten Referenz-Repräsentation und/oder der zweiten Referenz-Repräsentation und des von dem ersten Teilmodell ermittelten Modellparameters eine synthetische dritte Referenz-Repräsentation zu erzeugen,

(134) Empfangen der von dem zweiten Teilmodell erzeugten synthetischen dritten Referenz-Repräsentation,

(135) Quantifizieren von Abweichungen zwischen der synthetischen dritten Referenz-Repräsentation und der dritten Referenz-Repräsentation der Trainingsdaten,

(136) Reduzieren der Abweichungen durch Modifizieren von Modellparametern des ersten Teilmodells,

(140) Speichern des trainierten Modells des maschinellen Lernens und/oder Verwenden des trainierten Modells des maschinellen Lernens zum Erzeugen

einer synthetischen Repräsentation eines Untersuchungsbereichs eines Untersuchungsobjekts.

Fig. 8 zeigt eine Ausführungsform für das Erzeugen einer synthetischen Repräsentation eines Untersuchungsbereichs eines Untersuchungsobjekts (Vorhersageverfahren) in Form eines Ablaufschemas.

Das Vorhersageverfahren (200) umfasst die Schritte:

(210) Bereitstellen eines trainierten Modells des maschinellen Lernens,

(220) Bereitstellen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,

(230) Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,

(240) Zuführen der ersten Repräsentation und der zweiten Repräsentation dem trainierten Modell des maschinellen Lernens, wobei das trainierte Modell des maschinellen Lernens zwei Teilmodelle umfasst: ein erstes Teilmodell und ein zweites Teilmodell, wobei das erste Teilmodell konfiguriert ist und trainiert wurde, auf Basis der ersten Repräsentation und der zweiten Repräsentation mindestens einen Modellparameter für das zweite Teilmodell zu ermitteln, wobei das zweite Teilmodell konfiguriert ist, auf Basis der ersten Repräsentation und/oder der zweiten Repräsentation und des von dem ersten Teilmodell ermittelten mindestens einen Modellparameters eine synthetische dritte Repräsentation zu erzeugen,

(250) Empfangen einer synthetischen dritten Repräsentation des Untersuchungsbereichs des Untersuchungsobjekts von dem trainierten Modell des maschinellen Lernens, wobei die synthetische dritte Repräsentation den Referenzbereich nach Applikation einer dritten Menge des Kontrastmittels repräsentiert, wobei die dritte Menge verschieden von der zweiten Menge, vorzugsweise größer als die zweite Menge ist,

(260) Ausgeben und/oder Speichern der synthetischen dritten Repräsentation und/oder Übermitteln der synthetischen dritten Repräsentation an ein separates Computersystem.

Der Begriff "Bereitstellen" kann beispielsweise "Empfangen" oder "Erzeugen" bedeuten.

Der Begriff "Empfangen" umfasst sowohl das Abrufen als auch das Entgegennehmen von Gegenständen (z.B. von Repräsentationen und/oder einem (trainierten) Modell des maschinellen Lernens)), die z.B. an das Computersystem der vorliegenden Offenbarung übermittelt werden. Gegenstände können aus einem oder mehreren Datenspeichern ausgelesen und/oder von einem separaten Computersystem übermittelt werden. Repräsentation können beispielsweise von einem Computertomographen, von einem Magnetresonanztomographen oder von einem Ultraschall-Scanner empfangen werden.

Der Begriff "Erzeugen" bedeutet vorzugsweise, dass eine Repräsentation auf Basis einer anderen (z.B. einer empfangenen) Repräsentation oder auf Basis von mehreren anderen (z.B. empfangenen) Repräsentationen erzeugt wird. So kann beispielsweise eine empfangene Repräsentation eine Repräsentation eines Untersuchungsbereichs eines Untersuchungsobjekts im Ortsraum sein. Auf Basis dieser Ortsraum-Repräsentation kann beispielsweise eine Repräsentation des Untersuchungsbereichs des Untersuchungsobjekts im Frequenzraum durch eine Transformation (z.B. eine Fourier-Transformation) erzeugt werden. Weitere Möglichkeiten eine Repräsentation auf Basis einer oder mehrerer anderer Repräsentationen zu erzeugen, sind in dieser Beschreibung beschrieben.

Fig. 9 zeigt beispielhaft und schematisch ein Computersystem gemäß der vorliegenden Offenbarung.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 9 gezeigte Computersystem (1) umfasst eine Empfangseinheit (11), eine Steuer- und Recheneinheit (12) und eine Ausgabeeinheit (13).

Die Steuer- und Recheneinheit (12) dient der Steuerung des Computersystems (1), der Koordinierung der Datenflüsse zwischen den Einheiten des Computersystems (1) und der Durchführung von Berechnungen.

Die Steuer- und Recheneinheit (12) ist konfiguriert:

- eine erste Repräsentation zu erzeugen oder die Empfangseinheit (11) zu veranlassen, eine erste Repräsentation zu empfangen, wobei die erste Repräsentation einen Untersuchungsbereich eines Untersuchungsobjekts ohne Kontrastmittel oder nach Applikation einer ersten Menge eines Kontrastmittels repräsentiert,

- eine zweite Repräsentation zu erzeugen oder die Empfangseinheit (11) zu veranlassen, eine zweite Repräsentation zu empfangen, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,

- die erste Repräsentation und die zweite Repräsentation einem trainierten Modell des maschinellen Lernens zuzuführen

    ◦ wobei das trainierte Modell des maschinellen Lernens anhand von Trainingsdaten trainiert wurde,

    ◦ wobei die Trainingsdaten für jedes Referenzobjekt einer Vielzahl an Referenzobjekten (i) eine erste Referenz-Repräsentation eines Referenzbereichs des Referenzobjekts und eine zweite Referenz-Repräsentation des Referenzbereichs des Referenzobjekts als Eingabedaten und (ii) eine dritte Referenz-Repräsentation des Referenzbereichs des Referenzobjekts als Zieldaten umfassen,

    ◦ wobei die erste Referenz-Repräsentation den Referenzbereich ohne Kontrastmittel oder nach Applikation einer ersten Menge eines Kontrastmittels repräsentiert,

    ◦ wobei die zweite Referenz-Repräsentation den Referenzbereich nach Applikation einer zweiten Menge des Kontrastmittels repräsentiert, wobei die zweite Menge größer als die erste Menge ist,

    ◦ wobei die dritte Referenz-Repräsentation den Referenzbereich nach Applikation einer dritten Menge des Kontrastmittels repräsentiert, wobei die dritte Menge verschieden von der zweiten Menge, vorzugsweise größer als die zweite Menge ist,

    ◦ wobei das trainierte Modell des maschinellen Lernens zwei Teilmodelle umfasst: ein erstes Teilmodell und ein zweites Teilmodell, wobei das erste Teilmodell konfiguriert ist und trainiert wurde, auf Basis der ersten Referenz-Repräsentation und der zweiten Referenz-Repräsentation eines Referenzobjekts mindestens einen Modellparameter für das zweite Teilmodell zu ermitteln, wobei das zweite Teilmodell konfiguriert ist, auf Basis der ersten Referenz-Repräsentation und/oder der zweiten Referenz-Repräsentation und des von dem ersten Teilmodell ermittelten mindestens einen Modellparameters eine synthetische dritte Referenz-Repräsentation zu erzeugen,

- von dem trainierten Modell des maschinellen Lernens eine synthetische dritte Repräsentation des Untersuchungsbereichs des Untersuchungsobjekts zu empfangen, wobei die synthetische dritte Repräsentation den Referenzbereich nach Applikation einer dritten Menge des Kontrastmittels repräsentiert, wobei die dritte Menge verschieden von der zweiten Menge, vorzugsweise größer als die zweite Menge ist,

- die Ausgabeeinheit (13) zu veranlassen die synthetische dritte Repräsentation auszugeben und/oder in einem Datenspeicher zu speichern und/oder an ein separates Computersystem zu übermitteln.

Fig. 10 zeigt beispielhaft und schematisch eine weitere Ausführungsform des Computersystems. Das Computersystem (1) umfasst eine Verarbeitungseinheit (21), die mit einem Speicher (22) verbunden ist. Die Verarbeitungseinheit (21) und der Speicher (22) bilden eine Steuer- und Recheneinheit, wie sie in Fig. 9 gezeigt ist.

Die Verarbeitungseinheit (21) (engl.: *processing unit*) kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband

oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (11, 32, 33) und/oder eine oder mehrere Benutzerschnittstellen (12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

Das Computersystem der vorliegenden Offenbarung kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

Gegenstand der vorliegenden Erfindung ist auch ein Computerprogrammprodukt. Ein solches Computerprogrammprodukt umfasst einen nichtflüchtigen Datenträger wie beispielsweise eine CD, eine DVD, ein USB-Stick oder ein anderes Medium zum Speichern von Daten. Auf dem Datenträger ist ein Computerprogramm gespeichert. Das Computerprogramm kann in einen Arbeitsspeicher eines Computersystems (insbesondere in einen Arbeitsspeicher eines Computersystems der vorliegenden Offenbarung) geladen werden und dort das Computersystem dazu veranlassen, folgende Schritte ausführen:

- Empfangen oder Erzeugen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,

- Empfangen oder Erzeugen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,

- Zuführen der ersten Repräsentation und der zweiten Repräsentation einem trainierten Modell des maschinellen Lernens, wobei das trainierte Modell des maschinellen Lernens anhand von Trainingsdaten trainiert wurde,

    ◦ wobei die Trainingsdaten für jedes Referenzobjekt einer Vielzahl an Referenzobjekten (i) eine erste Referenz-Repräsentation eines Referenzbereichs des Referenzobjekts und eine zweite Referenz-Repräsentation des Referenzbereichs des Referenzobjekts als Eingabedaten und (ii) eine dritte Referenz-Repräsentation des Referenzbereichs des Referenzobjekts als Zieldaten umfassen,

◦ wobei die erste Referenz-Repräsentation den Referenzbereich ohne Kontrastmittel oder nach Applikation einer ersten Menge eines Kontrastmittels repräsentiert,

◦ wobei die zweite Referenz-Repräsentation den Referenzbereich nach Applikation einer zweiten Menge des Kontrastmittels repräsentiert, wobei die zweite Menge größer als die erste Menge ist,

◦ wobei die dritte Referenz-Repräsentation den Referenzbereich nach Applikation einer dritten Menge des Kontrastmittels repräsentiert, wobei die dritte Menge verschieden von der zweiten Menge, vorzugsweise größer als die zweite Menge ist,

◦ wobei das trainierte Modell des maschinellen Lernens zwei Teilmodelle umfasst: ein erstes Teilmodell und ein zweites Teilmodell, wobei das erste Teilmodell konfiguriert ist und trainiert wurde, auf Basis der ersten Referenz-Repräsentation und der zweiten Referenz-Repräsentation eines Referenzobjekts mindestens einen Modellparameter für das zweite Teilmodell zu ermitteln, wobei das zweite Teilmodell konfiguriert ist, auf Basis der ersten Referenz-Repräsentation und/oder der zweiten Referenz-Repräsentation und des von dem ersten Teilmodell ermittelten mindestens einen Modellparameters eine synthetische dritte Referenz-Repräsentation zu erzeugen,

- Empfangen einer synthetischen dritten Repräsentation des Untersuchungsbereichs des Untersuchungsobjekts von dem trainierten Modell des maschinellen Lernens, wobei die synthetische dritte Repräsentation den Referenzbereich nach Applikation einer dritten Menge des Kontrastmittels repräsentiert, wobei die dritte Menge verschieden von der zweiten Menge, vorzugsweise größer als die zweite Menge ist,

- Ausgeben und/oder Speichern der synthetischen dritten Repräsentation und/oder Übermitteln der synthetischen dritten Repräsentation an ein separates Computersystem.

Das Computerprogrammprodukt kann auch in Kombination (in einer Zusammenstellung) mit dem Kontrastmittel vermarktet werden. Eine solche Zusammenstellung wird auch als Kit bezeichnet. Ein solches Kit umfasst das Kontrastmittel und das Computerprogrammprodukt. Es ist auch möglich, dass ein solches Kit das Kontrastmittel und Mittel umfasst, die es einen Käufer erlauben, das Computerprogramm zu beziehen, z.B. von einer Internetseite herunterzuladen. Diese Mittel können einen Link, d.h. eine Adresse der Internetseite umfassen, auf der das Computerprogramm bezogen, z.B. von der das Computerprogramm auf ein mit dem Internet verbundenes Computersystem heruntergeladen werden kann. Diese Mittel können einen Code (z.B. eine alphanumerische Zeichenkette oder einen QR-Code, oder einen DataMatrix-Code oder einen Barcode oder eine anderen optisch und/oder elektronisch lesbaren Code) umfassen, mit dem der Käufer Zugang zu dem Computerprogramm erhält. Ein solcher Link und/oder Code kann beispielsweise auf einer Verpackung des Kontrastmittels aufgedruckt und/oder auf einem Beipackzettel zum Kontrastmittel aufgedruckt sein. Ein Kit ist somit ein Kombinationsprodukt umfassend ein Kontrastmittel und ein Computerprogramm (z.B. in Form eines Zugangs zu dem Computerprogramm oder in Form von ausführbarem Programmcode auf einem Datenträger), das zusammen zum Kauf angeboten wird.

Die vorliegende Erfindung kann für verschiedene Zwecke verwendet werden. Einige Anwendungsbeispiele werden nachfolgend beschrieben, ohne die Erfindung auf diese Anwendungsbeispiele beschränken zu wollen.

Ein erstes Anwendungsbeispiel betrifft magnetresonanztomographische Untersuchungen zur Abgrenzung von intraaxialen Tumoren wie intrazerebralen Metastasen und malignen Gliomen. Wegen des infiltrativen Wachstums dieser Tumoren ist eine genaue Abgrenzung zwischen Tumor und gesundem Gewebe schwierig. Die Bestimmung der Ausdehnung eines Tumors ist jedoch für eine operative Entfernung entscheidend. Die Unterscheidung zwischen Tumoren und gesundem Gewebe wird durch Applikation eines extrazellulären erleichtert; nach intravenöser Gabe einer Standarddosis von 0,1 mmol/kg Körpergewicht des extrazellulären MRT-Kontrastmittels Gadobutrol lassen sich intraaxiale Tumoren deutlich besser abgrenzen. Bei höheren Dosen wird der Kontrast zwischen Läsion und gesundem Hirngewebe weiter erhöht; die Nachweisrate von Hirnmetastasen steigt linear mit der Dosis des Kontrastmittels an (siehe z.B. M. Hartmann et al.: Does the administration of a high dose of a paramagnetic contrast medium (Gadovist) improve the diagnostic value of magnetic resonance tomography in glioblastomas? doi: 10.1055/s-2007-1015623).

Dabei können eine einzelne Dreifachdosis oder eine zweite Folgedosis bis zu einer Gesamtdosis von 0,3 mmol/kg Körpergewicht verabreicht werden. Dadurch werden der Patient und die Umgebung zusätzlichem Gadolinium ausgesetzt, und im Falle eines zweiten Scans entstehen weitere zusätzliche Kosten.

Die vorliegende Erfindung kann eingesetzt werden, um eine Kontrastmitteldosis, die über die Standardmenge hinausgeht, zu vermeiden. Es können eine erste MRT-Aufnahme ohne Kontrastmittel oder mit einer geringeren Menge als die Standardmenge und eine zweite MRT-Aufnahme mit der Standardmenge erzeugt werden. Auf Basis dieser erzeugten MRT-Aufnahmen kann

wie in dieser Offenbarung beschrieben, eine synthetische MRT-Aufnahme erzeugt werden, bei der sich der Kontrast zwischen Läsionen und gesundem Gewebe durch Verändern des Verstärkungsfaktors $\alpha$ in weiten Grenzen variieren lässt. Dabei können Kontraste erreicht werden, die sonst nur durch Applikation einer Menge an Kontrastmittel erzielt werden können, die höher als die Standardmenge ist.

Ein weiteres Anwendungsbeispiel betrifft die Reduzierung der Menge an MRT-Kontrastmittel in einer magnetresonanztomographischen Untersuchung. Gadolinium-haltige Kontrastmittel wie Gadobutrol werden für eine Vielzahl an Untersuchungen eingesetzt. Sie dienen der Kontrastverstärkung bei Untersuchungen des Schädels, der Wirbelsäule, der Brust oder anderen Untersuchungen. Im zentralen Nervensystem hebt Gadobutrol Bereiche mit einer gestörten Blut-Hirn-Schranke und/oder abnormalen Gefäßen hervor. Im Brustgewebe macht Gadobutrol das Vorhandensein und Ausmaß einer bösartigen Brusterkrankung sichtbar. Gadobutrol wird auch in der kontrastverstärkten Magnetresonanzangiographie zur Diagnose von Schlaganfällen, zum Nachweis der Tumordurchblutung und zum Nachweis einer fokalen zerebralen Ischämie eingesetzt.

Aufgrund der zunehmenden Umweltbelastung, der Kostenbelastung des Gesundheitssystems und der Befürchtung akuter Nebenwirkungen und möglicher langfristiger Gesundheitsrisiken, insbesondere bei wiederholter und langfristiger Exposition, wird eine Dosisreduzierung von Gadolinium-haltigen Kontrastmitteln angestrebt. Dies kann durch die vorliegende Erfindung erreicht werden.

Es können eine erste MRT-Aufnahme ohne Kontrastmittel und eine zweite MRT-Aufnahme mit einer Kontrastmittelmenge erzeugt werden, die geringer als die Standardmenge ist. Auf Basis dieser erzeugten MRT-Aufnahmen kann wie in dieser Offenbarung beschrieben, eine synthetische MRT-Aufnahme erzeugt werden, bei der sich der Kontrast durch Verändern des Verstärkungsfaktors $\alpha$ in weiten Grenzen variieren lässt. Dabei kann mit einer geringeren Kontrastmittelmenge als der Standardmenge ein Kontrast erreicht werden, der dem Kontrast nach der Applikation der Standardmenge entspricht.

Ein weiteres Anwendungsbeispiel betrifft die Detektion, Identifizierung und/oder Charakterisierung von Läsionen in der Leber mit Hilfe eines hepatobiliären Kontrastmittels wie beispielsweise Primovist®.

Primovist® wird intravenös (i.v.) in einer Standarddosis von 0,025 mmol/kg Körpergewicht verabreicht. Diese Standarddosis ist geringer als die Standarddosis von 0,1 mmol/kg Körpergewicht bei extrazellulären MR-Kontrastmitteln. Im Vergleich zur kontrastverstärkten MRT mit extrazellulären Gadolinium-haltigen Kontrastmitteln ermöglicht Primovist® eine dynamische T1w-Mehrphasenbildgebung. Aufgrund der niedrigeren Dosis von Primovist® und der Beobachtung vorübergehender Bewegungsartefakte, die kurz nach der intravenösen Verabreichung auftreten können, wird die Kontrastverstärkung von Primovist® in der arteriellen Phase jedoch von Radiologen als geringer wahrgenommen als die die Kontrastverstärkung von extrazellulären MRT-Kontrastmitteln. Die Beurteilung der Kontrastverstärkung in der arteriellen Phase und der Vaskularität von fokalen Leberläsionen ist für die genaue Charakterisierung der Läsion aber von entscheidender Bedeutung.

Mit Hilfe der vorliegenden Erfindung kann der Kontrast insbesondere in der arteriellen Phase erhöht werden, ohne dass eine höhere Dosis verabreicht werden muss.

Es können eine erste MRT-Aufnahme ohne Kontrastmittel und eine zweite MRT-Aufnahme während der arteriellen Phase nach der Applikation einer Kontrastmittelmenge erzeugt werden, die der Standardmenge entspricht. Auf Basis dieser erzeugten MRT-Aufnahmen kann wie in dieser Offenbarung beschrieben, eine synthetische MRT-Aufnahme erzeugt werden, bei der sich der Kontrast in der arteriellen Phase durch Verändern des Verstärkungsfaktors $\alpha$ in weiten Grenzen variieren lässt. Dabei können Kontraste erreicht werden, die sonst nur durch Applikation einer Menge an Kontrastmittel erzielt werden können, die höher als die Standardmenge ist.

Ein weiteres Anwendungsbeispiel betrifft die Verwendung von MRT-Kontrastmitteln in computertomographischen Untersuchungen.

MRT-Kontrastmittel entfalten üblicherweise in einer CT-Untersuchung eine geringere kontraststeigernde Wirkung als CT-Kontrastmittel. Dennoch kann es vorteilhaft sein, ein MRT-Kontrastmittel in einer CT-Untersuchung einzusetzen. Als Beispiel sei eine minimalinvasive Intervention in der Leber eines Patienten aufgeführt, bei der ein Chirurg den Eingriff mittels eines CT-Scanners verfolgt. Die Computertomographie (CT) hat gegenüber der Magnetresonanztomographie den Vorteil, dass während der Erzeugung von CT-Aufnahmen eines Untersuchungsbereichs eines Untersuchungsobjekts chirurgische Eingriffe in dem Untersuchungsbereich in größerem Umfang möglich sind. Es gibt hingegen nur wenige interventionelle Bestecke und chirurgische Geräte, die MRTtauglich sind. Zudem ist der Zugang zum Patienten durch die in der MRT verwendeten Magneten eingeschränkt. Während ein Chirurg also einen Eingriff in dem Untersuchungsbereich vornimmt, kann er den Untersuchungsbereich mit Hilfe der CT bildhaft darstellen und den Eingriff auf einem Monitor verfolgen.

Möchte ein Chirurg beispielsweise einen Eingriff in der Leber eines Patienten vornehmen, um z.B. eine Biopsie an einer Leberläsion durchzuführen oder einen Tumor zu entfernen, so ist in einer CT-Aufnahme der Leber der Kontrast zwischen einer Leberläsion oder dem Tumor und gesundem Lebergewebe nicht so ausgeprägt wie in einer MRT-Aufnahme nach der Applikation eines hepatobiliären Kontrastmittels. In der CT sind derzeit keine hepatobiliären CT-spezifischen Kontrastmittel bekannt und/oder zugelassen. Die Verwendung eines

MRT-Kontrastmittels, insbesondere eines hepatobiliären MRT-Kontrastmittels in der Computertomographie kombiniert also die Möglichkeit der Differenzierung zwischen gesundem und erkranktem Lebergewebe und die Möglichkeit der Durchführung eines Eingriffs bei gleichzeitiger Darstellung der Leber.

Dabei kann die vergleichsweise geringe, durch das MRT-Kontrastmittel erzielte Kontrastverstärkung mit Hilfe der vorliegenden Erfindung erhöht werden, ohne dass eine höhere Dosis als die Standarddosis verabreicht werden muss.

Es können eine erste CT-Aufnahme ohne MRT-Kontrastmittel und eine zweite CT-Aufnahme nach der Applikation eines MRT-Kontrastmittels erzeugt werden, dessen Menge der Standardmenge entspricht. Auf Basis dieser erzeugten CT-Aufnahmen kann wie in dieser Offenbarung beschrieben, eine synthetische CT-Aufnahme erzeugt werden, bei der sich der durch das MRT-Kontrastmittel hervorgerufene Kontrast durch Verändern des Verstärkungsfaktors $\alpha$ in weiten Grenzen variieren lässt. Dabei können Kontraste erreicht werden, die sonst nur durch Applikation einer Menge an MRT-Kontrastmittel erzielt werden können, die höher als die Standardmenge ist.

## Patentansprüche

**1.** Computer-implementiertes Verfahren umfassend:

- Bereitstellen eines trainierten Modells ($M^T$) des maschinellen Lernens,

  ◦ wobei das trainierte Modell ($M^T$) des maschinellen Lernens anhand von Trainingsdaten (TD) trainiert wurde,
  ◦ wobei die Trainingsdaten (TD) für jedes Referenzobjekt einer Vielzahl an Referenzobjekten (i) eine erste Referenz-Repräsentation (RR1) eines Referenzbereichs des Referenzobjekts und eine zweite Referenz-Repräsentation (RR2) des Referenzbereichs des Referenzobjekts als Eingabedaten und (ii) eine dritte Referenz-Repräsentation (RR3) des Referenzbereichs des Referenzobjekts als Zieldaten umfassen,
  ◦ wobei die erste Referenz- Repräsentation (RR1) den Referenzbereich ohne Kontrastmittel oder nach Applikation einer ersten Menge eines Kontrastmittels repräsentiert,
  ◦ wobei die zweite Referenz-Repräsentation (RR2) den Referenzbereich nach Applikation einer zweiten Menge des Kontrastmittels repräsentiert, wobei die zweite Menge größer als die erste Menge ist,
  ◦ wobei die dritte Referenz-Repräsentation (RR3) den Referenzbereich nach Applikation einer dritten Menge des Kontrastmittels repräsentiert, wobei die dritte Menge verschieden von der zweiten Menge, vorzugsweise größer als die zweite Menge ist,
  ◦ wobei das trainierte Modell ($M^T$) des maschinellen Lernens zwei Teilmodelle umfasst: ein erstes Teilmodell ($TM1^T$) und ein zweites Teilmodell (TM2), wobei das erste Teilmodell ($TM1^T$) konfiguriert ist und trainiert wurde, auf Basis der ersten Referenz-Repräsentation (RR1) und der zweiten Referenz-Repräsentation (RR2) des Untersuchungsbereichs eines Referenzobjekts mindestens einen Modellparameter (MP) für das zweite Teilmodell (TM2) zu ermitteln, wobei das zweite Teilmodell (TM2) konfiguriert ist, auf Basis der ersten Referenz-Repräsentation (RR1) und der zweiten Referenz-Repräsentation (RR2) und des von dem ersten Teilmodell ($TM1^T$) ermittelten mindestens einen Modellparameters (MP) eine synthetische dritte Referenz-Repräsentation (RR3*) zu erzeugen,

- Bereitstellen einer ersten Repräsentation (R1), wobei die erste Repräsentation (R1) einen Untersuchungsbereich eines Untersuchungsobjekts ohne Kontrastmittel oder nach Applikation einer ersten Menge eines Kontrastmittels repräsentiert,
- Bereitstellen einer zweiten Repräsentation (R2), wobei die zweite Repräsentation (R2) den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation (R1) und der zweiten Repräsentation (R2) dem trainierten Modell ($M^T$) des maschinellen Lernens,
- Empfangen einer synthetischen dritten Repräsentation (R3*) des Untersuchungsbereichs des Untersuchungsobjekts von dem trainierten Modell ($M^T$) des maschinellen Lernens, wobei die synthetische dritte Repräsentation (R3*) den Referenzbereich nach Applikation einer dritten Menge des Kontrastmittels repräsentiert, wobei die dritte Menge verschieden von der zweiten Menge, vorzugsweise größer als die zweite Menge ist,
- Ausgeben und/oder Speichern der synthetischen dritten Repräsentation (R3*) und/oder Übermitteln der synthetischen dritten Repräsentation (R3*) an ein separates Computersystem.

**dadurch gekennzeichnet, dass** das zweite Teilmodell (TM2) konfiguriert ist,

- die erste Repräsentation (R1) des Untersuchungsbereichs des Untersuchungsobjekts von der zweiten Repräsentation (R2) des Untersuchungsbereichs des Untersuchungsobjekts zu subtrahieren, wobei eine Differenz (R2-R1) gebildet wird,
- die Differenz (R2-R1) mit einem Verstärkungsfaktor ($\alpha$) zu multiplizieren, wobei der Verstärkungsfaktor ($\alpha$) eine positive oder negative reelle Zahl ist, wobei der mindestens eine von dem ersten Teilmodell ($TM1^T$) ermittelte Modellparameter (MP) den Verstärkungsfaktor ($\alpha$) umfasst,
- die mit dem Verstärkungsfaktor ($\alpha$) multiplizierte Differenz (R2-R1) auf die erste Repräsentation (R1) oder die zweite Repräsentation (R2) zu addieren.

**2.** Verfahren gemäß Anspruch 1, wobei die erste Repräsentation (R1) und die zweite Repräsentation (R2) Repräsentationen ($R1^F$, $R2^F$) des Untersuchungsbereichs des Untersuchungsobjekts im Frequenzraum sind, wobei das zweite Teilmodell (TM2) konfiguriert ist,

- die Differenz ($R2^F$-$R1^F$) mit einer frequenzabhängigen Gewichtsfunktion (WF) zu multiplizieren, wobei eine gewichtete Differenz ($R2^F$-$R1^F$)$^W$ gebildet wird, wobei der mindestens eine von dem ersten Teilmodell ($TM1^T$) ermittelte Modellparameter (MP) einen oder mehrere Parameter der frequenzabhängigen Gewichtsfunktion (WF) umfasst,
- die gewichtete Differenz ($R2^F$-$R1^F$)$^W$ mit dem Verstärkungsfaktor ($\alpha$) zu multiplizieren,
- die mit dem Verstärkungsfaktor ($\alpha$) multiplizierte gewichtete Differenz ($R2^F$-$R1^F$)$^W$ auf die erste Repräsentation ($R1^F$) oder die zweite Repräsentation ($R2^F$) zu addieren.

**3.** Verfahren gemäß einem der Ansprüche 1 bis 2, wobei das erste Teilmodell ($TM1^T$) ein künstliches neuronales Netz ist oder umfasst.

**4.** Verfahren gemäß einem der Ansprüche 1 bis 3, wobei das zweite Teilmodell (TM2) ein mechanistisches Modell ist.

**5.** Verfahren gemäß einem der Ansprüche 1 bis 4, wobei Modellparameter des zweiten Teilmodells (TM2) keine trainierbaren Parameter sind.

**6.** Verfahren gemäß einem der Ansprüche 1 bis 5, wobei das Modell ($M^T$) des maschinellen Lernens differenzierbar ist und das Trainieren Ende-zu-Ende erfolgt ist.

**7.** Verfahren gemäß einem der Ansprüche 1 bis 6, wobei das Modell ($M^T$) des maschinellen Lernens ein drittes Teilmodell ($TM3^T$) umfasst, wobei das dritte Teilmodell ($TM3^T$) konfiguriert ist und trainiert wurde, auf Basis der von dem zweiten Teilmodell (TM2) erzeugten synthetischen dritten Repräsentation (R3*) eine korrigierte dritte Repräsentation ($R3^{*C}$) zu erzeugen, wobei der Schritt Empfangen einer synthetischen dritten Repräsentation (R3*) des Untersuchungsbereichs des Untersuchungsobjekts von dem trainierten Modell ($M^T$) des maschinellen Lernens umfasst: Empfangen der korrigierten dritten Repräsentation ($R3^{*C}$) des Untersuchungsbereichs des Untersuchungsobjekts von dem trainierten Modell ($M^T$) des maschinellen Lernens, wobei die korrigierte dritte Repräsentation ($R3^{*C}$) den Referenzbereich nach Applikation der dritten Menge des Kontrastmittels repräsentiert, wobei der Schritt Ausgeben und/oder Speichern der synthetischen dritten Repräsentation (R3*) und/oder Übermitteln der synthetischen dritten Repräsentation (R3*) an ein separates Computersystem umfasst: Ausgeben und/oder Speichern der korrigierten dritten Repräsentation ($R3^{*C}$) und/oder Übermitteln der korrigierten dritten Repräsentation ($R3^{*C}$) an ein separates Computersystem.

**8.** Verfahren gemäß Anspruch 7, wobei das dritte Teilmodell ($TM3^T$) ein trainiertes Modell des maschinellen Lernens ist, wobei das dritte Teilmodell ($TM3^T$) vorzugsweise ein künstliches neuronales Netzwerk ist oder umfasst.

**9.** Verfahren gemäß einem der Ansprüche 1 bis 8, wobei das trainierte Modell ($M^T$) des maschinellen Lernens trainiert wurde, wobei das Trainieren des trainierten Modells ($M^T$) des maschinellen Lernens umfasst:

- Bereitstellen der Trainingsdaten (TD),
- Bereitstellen eines Modells (M) des maschinellen Lernens,
- Trainieren des Modells (M) des maschinellen Lernens, wobei das Trainieren umfasst:

    ◦ Zuführen einer ersten Referenz-Repräsentation (RR1) und einer zweiten Referenz-Repräsentation (RR2) eines Referenzbereichs eines Referenzobjekts dem ersten Teilmodell (TM1), wobei das erste Teilmodell (TM1) konfiguriert ist, auf Basis der ersten Referenz-Repräsentation (RR1), der zweiten Referenz-Repräsentation (RR2) und Modellparametern mindestens einen Modellparameter (MP) für das zweite Teilmodell (TM2) zu ermitteln,
    ◦ Empfangen des von dem ersten Teilmodell (TM1) ermittelten mindestens einen Modellparameters (MP),
    ◦ Zuführen des ermittelten mindestens einen Modellparameters (MP) und der ersten

Referenz-Repräsentation (RR1) und/oder der zweiten Referenz-Repräsentation (RR2) dem zweiten Teilmodell (TM2), wobei das zweite Teilmodell (TM2) konfiguriert ist, auf Basis der ersten Referenz-Repräsentation (RR1) und/oder der zweiten Referenz-Repräsentation (RR2) und des von dem ersten Teilmodell (TM1) ermittelten Modellparameters (MP) eine synthetische dritte Referenz-Repräsentation (RR3*) zu erzeugen,

◦ Empfangen der von dem zweiten Teilmodell (TM2) erzeugten synthetischen dritten Referenz-Repräsentation (RR3*),

◦ Quantifizieren von Abweichungen zwischen der synthetischen dritten Referenz-Repräsentation (RR3*) und der dritten Referenz-Repräsentation (RR3) der Trainingsdaten,

◦ Reduzieren der Abweichungen durch Modifizieren von Modellparametern des ersten Teilmodells (TM1),

- Speichern des trainierten Modells ($M^T$) des maschinellen Lernens und/oder Verwenden des trainierten Modells ($M^T$) des maschinellen Lernens zum Erzeugen einer synthetischen Repräsentation (R3*) eines Untersuchungsbereichs eines Untersuchungsobjekts.

**10.** Verfahren gemäß einem der Ansprüche 2 bis 9, wobei die in Anspruch 2 genannte frequenzabhängige Gewichtsfunktion (WF) eine Hann-Funktion, eine Poisson-Funktion oder eine Gauß-Funktion ist.

**11.** Verfahren gemäß einem der Ansprüche 1 bis 10, wobei das Untersuchungsobjekt ein Mensch ist und der Untersuchungsbereich ein Teil des Menschen ist, wobei jedes Referenzobjekt ein Mensch ist und der Referenzbereich eines jeden Referenzobjekts ein Teil des Referenzobjekts ist, wobei der Referenzbereich eines jeden Referenzobjekts und der Untersuchungsbereich der jeweils gleiche Teil des Menschen ist.

**12.** Verfahren gemäß einem der Ansprüche 1 bis 11, wobei die erste Repräsentation (R1) und die zweite Repräsentation (R2) sowie jede Referenz-Repräsentation (RR1, RR2, RR3) eines jeden Referenzobjekts das Ergebnis einer radiologischen Untersuchung ist, wobei die radiologische Untersuchung vorzugsweise eine MRT-Untersuchung oder eine CT-Untersuchung ist und das Kontrastmittel ein MRT-Kontrastmittel oder ein CT-Kontrastmittel ist.

**13.** Verfahren gemäß einem der Ansprüche 1 bis 12, wobei die erste Menge gleich Null ist, die zweite Menge geringer als die Standardmenge des Kontrastmittels ist oder gleich der Standardmenge des Kontrastmittels ist und die dritte Menge größer als die Standardmenge des Kontrastmittels ist.

**14.** Verfahren nach einem der Ansprüche 1 bis 13, wobei das Kontrastmittel

- einen $Gd^{3+}$-Komplex einer Verbindung der Formel (I)

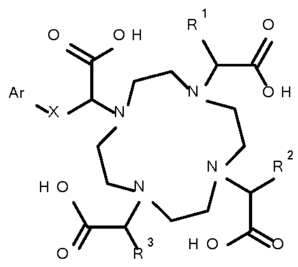

(I) ,

umfasst, wobei
Ar eine Gruppe ausgewählt aus

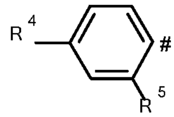

und

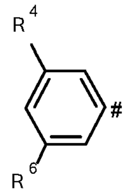

darstellt,
wobei # die Anknüpfung zu X darstellt,
X eine Gruppe darstellt, die aus $CH_2$, $(CH_2)_2$, $(CH_2)_3$, $(CH_2)_4$ und $*-(CH_2)_2-O-CH_2-^{\#}$ ausgewählt wird,
wobei * die Anknüpfung zu Ar darstellt und # die Anknüpfung zum Essigsäurerest darstellt, $R^1$, $R^2$ and $R^3$ unabhängig voneinander ein Wasserstoffatom oder eine Gruppe ausgewählt aus $C_1$-$C_3$-Alkyl, $-CH_2OH$, $-(CH_2)_2OH$ und $-CH_2OCH_3$ darstellen,
$R^4$ eine Gruppe ausgewählt aus $C_2$-$C_4$-Alkoxy, $(H_3C-CH_2)-O-(CH_2)_2-O-$, $(H_3C-CH_2)-O-(CH_2)_2-O-(CH_2)_2-O-$ und $(H_3C-CH_2)-O-(CH_2)_2-O-(CH_2)_2-O-(CH_2)_2-O-$ darstellt,
$R^5$ ein Wasserstoffatom darstellt,
und
$R^6$ ein Wasserstoffatom darstellt,
oder ein Stereoisomer, Tautomer, Hydrat, Solvat oder Salz davon, oder eine Mischung davon, oder

- einen $Gd^{3+}$-Komplex einer Verbindung der Formel (II)

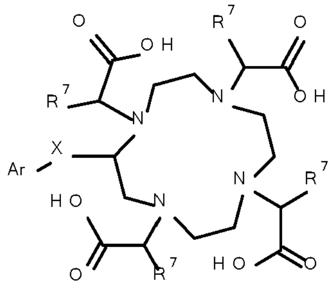

(II) ,

umfasst, wobei
Ar eine Gruppe ausgewählt aus

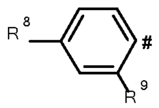 and 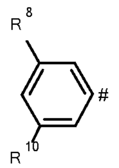

darstellt,
wobei # die Anknüpfung zu X darstellt,
X eine Gruppe darstellt, die aus $CH_2$, $(CH_2)_2$, $(CH_2)_3$, $(CH_2)_4$ and $*-(CH_2)_2-O-CH_2-^{\#}$ ausgewählt wird, wobei * die Anknüpfung zu Ar darstellt und # die Anknüpfung zum Essigsäurerest darstellt,
$R^7$ ein Wasserstoffatom oder eine Gruppe ausgewählt aus $C_1$-$C_3$-Alkyl, $-CH_2OH$, $-(CH_2)_2OH$ und $-CH_2OCH_3$ darstellt;
$R^8$ eine Gruppe ausgewählt aus $C_2$-$C_4$-Alkoxy, $(H_3C-CH_2O)-(CH_2)_2-O-$, $(H_3C-CH_2O)-(CH_2)_2-O-(CH_2)_2-O-$ und $(H_3C-CH_2O)-(CH_2)_2-O-(CH_2)_2-O-(CH_2)_2-O-$ darstellt;
$R^9$ und $R^{10}$ unabhängig voneinander ein Wasserstoffatom darstellen;
oder ein Stereoisomer, Tautomer, Hydrat, Solvat oder Salz davon, oder eine Mischung davon, oder das Kontrastmittel eine der folgenden Substanzen umfasst:

- Gadolinium(III) 2-[4,7,10-tris(carboxymethyl)-1,4,7,10-tetrazacyclododec-1-yl]essigsäure,
- Gadolinium(III) Ethoxybenzyl-diethylenetriaminepentaessigsäure,
- Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat,
- Dihydrogen[(±)-4-carboxy-5,8,11-tris(carboxymethyl)-1-phenyl-2-oxa-5,8,11-triazatridecan-13-oato(5-)]gadolinat(2-),
- Tetragadolinium-[4,10-bis(carboxylatomethyl)-7-13,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat,
- 2,2',2"-(10-{1-carboxy-2-[2-(4-ethoxyphenyl) ethoxy]ethyl}-1,4,7,10-tetraazacyclododecan-1,4,7-triyl)triacetat,
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2"-{10-[(1R)-1-carboxy-2-{4-[2-(2-ethoxyethoxy)ethoxy]phenyl} ethyl]-1,4,7,10-tetraazacyclododecan-1,4,7-triyl}triacetat,
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat)
- Gadolinium 2,2',2"-{10-[(1S)-4-(4-butoxyphenyl)-1-carboxybutyl]-1,4,7,10-tetraazacyclododecan-1,4,7-triyl}triacetat,
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl]acetat,
- Gadolinium(III) 2,2',2"-(10-((2R,3S)-1,3,4-trihydroxybutan-2-yl)-1,4,7,10-tetraazacyclododecan-1,4,7-triyl)triacetat,
- Gadolinium-2,2',2"-{(2S)-10-(carboxymethyl)-2-[4-(2-ethoxyethoxy)benzyl]-1,4,7,10-tetraazacyclododecane-1,4,7-triyl}triacetat,
- Gadolinium-2,2',2"-[10-(carboxymethyl)-2-(4-ethoxybenzyl)-1,4,7,10-tetraazacyclododecane-1,4,7-triyl]triacetat.

**15.** Computersystem (1) umfassend

• eine Empfangseinheit (11),
• eine Steuer- und Recheneinheit (12), und
• eine Ausgabeeinheit (13),

wobei die Steuer- und Recheneinheit (12) konfiguriert ist,

- eine erste Repräsentation (R1) zu erzeugen oder die Empfangseinheit (11) zu veranlassen, eine erste Repräsentation (R1) zu empfangen, wobei die erste Repräsentation (R1) einen Untersuchungsbereich eines Untersuchungsobjekts ohne Kontrastmittel oder nach Applikation einer ersten Menge eines Kontrastmittels repräsentiert,

- eine zweite Repräsentation (R2) zu erzeugen oder die Empfangseinheit (11) zu veranlassen, eine zweite Repräsentation (R2) zu empfangen, wobei die zweite Repräsentation (R2) den Untersuchungsbereich des Untersuchungsobjekts nach Applikation einer zweiten Menge des Kontrastmittels repräsentiert, wobei die zweite Menge größer als die erste Menge ist,
- die erste Repräsentation (R1) und die zweite Repräsentation (R2) einem trainierten Modell ($M^T$) des maschinellen Lernens zuzuführen

◦ wobei das trainierte Modell ($M^T$) des maschinellen Lernens anhand von Trainingsdaten (TD) trainiert wurde,
◦ wobei die Trainingsdaten (TD) für jedes Referenzobjekt einer Vielzahl an Referenzobjekten (i) eine erste Referenz-Repräsentation (RR1) eines Referenzbereichs des Referenzobjekts und eine zweite Referenz-Repräsentation (RR2) des Referenzbereichs des Referenzobjekts als Eingabedaten und (ii) eine dritte Referenz-Repräsentation (RR3) des Referenzbereichs des Referenzobjekts als Zieldaten umfassen,
◦ wobei die erste Referenz-Repräsentation (RR1) den Referenzbereich ohne Kontrastmittel oder nach Applikation einer ersten Menge eines Kontrastmittels repräsentiert,
◦ wobei die zweite Referenz-Repräsentation (RR2) den Referenzbereich nach Applikation einer zweiten Menge des Kontrastmittels repräsentiert, wobei die zweite Menge größer als die erste Menge ist,
◦ wobei die dritte Referenz-Repräsentation (RR3) den Referenzbereich nach Applikation einer dritten Menge des Kontrastmittels repräsentiert, wobei die dritte Menge verschieden von der zweiten Menge, vorzugsweise größer als die zweite Menge ist,
◦ wobei das trainierte Modell ($M^T$) des maschinellen Lernens zwei Teilmodelle umfasst: ein erstes Teilmodell ($TM1^T$) und ein zweites Teilmodell (TM2), wobei das erste Teilmodell ($TM1^T$) konfiguriert ist und trainiert wurde, auf Basis der ersten Referenz-Repräsentation (RR1) und der zweiten Referenz-Repräsentation (RR2) eines jeden Referenzobjekts mindestens einen Modellparameter (MP) für das zweite Teilmodell (TM2) zu ermitteln, wobei das zweite Teilmodell (TM2) konfiguriert ist, auf Basis der ersten Referenz-Repräsentation (RR1) und der zweiten Referenz-Repräsentation (RR2) und des von dem ersten Teilmodell ($TM1^T$) ermittelten mindestens einen Modellparameters (MP) eine synthetische dritte Referenz-Repräsentation (RR3*) zu erzeugen,

- von dem trainierten Modell ($M^T$) des maschinellen Lernens eine synthetische dritte Repräsentation (R3*) des Untersuchungsbereichs des Untersuchungsobjekts zu empfangen, wobei die synthetische dritte Repräsentation (R3*) den Referenzbereich nach Applikation einer dritten Menge des Kontrastmittels repräsentiert, wobei die dritte Menge verschieden von der zweiten Menge, vorzugsweise größer als die zweite Menge ist,
- die Ausgabeeinheit (13) zu veranlassen die synthetische dritte Repräsentation (R3*) auszugeben und/oder in einem Datenspeicher zu speichern und/oder an ein separates Computersystem zu übermitteln,

**dadurch gekennzeichnet, dass** das zweite Teilmodell (TM2) konfiguriert ist,

- die erste Repräsentation (R1) des Untersuchungsbereichs des Untersuchungsobjekts von der zweiten Repräsentation (R2) des Untersuchungsbereichs des Untersuchungsobjekts zu subtrahieren, wobei eine Differenz (R2-R1) gebildet wird,
- die Differenz (R2-R1) mit einem Verstärkungsfaktor ($\alpha$) zu multiplizieren, wobei der Verstärkungsfaktor ($\alpha$) eine positive oder negative reelle Zahl ist, wobei der mindestens eine von dem ersten Teilmodell ($TM1^T$) ermittelte Modellparameter (MP) den Verstärkungsfaktor ($\alpha$) umfasst,
- die mit dem Verstärkungsfaktor ($\alpha$) multiplizierte Differenz (R2-R1) auf die erste Repräsentation (R1) oder die zweite Repräsentation (R2) zu addieren.

**16.** Computerprogrammprodukt umfassend einen Datenträger, auf dem ein Computerprogramm (40) gespeichert ist, wobei das Computerprogramm (40) in einen Arbeitsspeicher (22) eines Computersystems (1) geladen werden kann und dort das Computersystem (1) dazu veranlasst, folgende Schritte ausführen:

- Bereitstellen eines trainierten Modells ($M^T$) des maschinellen Lernens,

◦ wobei das trainierte Modell ($M^T$) des maschinellen Lernens anhand von Trainingsdaten (TD) trainiert wurde,

◦ wobei die Trainingsdaten (TD) für jedes Referenzobjekt einer Vielzahl an Referenzobjekten (i) eine erste Referenz-Repräsentation (RR1) eines Referenzbereichs des Referenzobjekts und eine zweite Referenz-Repräsentation (RR2) des Referenzbereichs des Referenzobjekts als Eingabedaten und (ii) eine dritte Referenz-Repräsentation (RR3) des Referenzbereichs des Referenzobjekts als Zieldaten umfassen,

◦ wobei die erste Referenz- Repräsentation (RR1) den Referenzbereich ohne Kontrastmittel oder nach Applikation einer ersten Menge eines Kontrastmittels repräsentiert,

◦ wobei die zweite Referenz-Repräsentation (RR2) den Referenzbereich nach Applikation einer zweiten Menge des Kontrastmittels repräsentiert, wobei die zweite Menge größer als die erste Menge ist,

◦ wobei die dritte Referenz-Repräsentation (RR3) den Referenzbereich nach Applikation einer dritten Menge des Kontrastmittels repräsentiert, wobei die dritte Menge verschieden von der zweiten Menge, vorzugsweise größer als die zweite Menge ist,

◦ wobei das trainierte Modell ($M^T$) des maschinellen Lernens zwei Teilmodelle umfasst: ein erstes Teilmodell ($TM1^T$) und ein zweites Teilmodell (TM2), wobei das erste Teilmodell ($TM1^T$) konfiguriert ist und trainiert wurde, auf Basis der ersten Referenz-Repräsentation (RR1) und der zweiten Referenz-Repräsentation (RR2) des Untersuchungsbereichs eines Referenzobjekts mindestens einen Modellparameter (MP) für das zweite Teilmodell (TM2) zu ermitteln, wobei das zweite Teilmodell (TM2) konfiguriert ist, auf Basis der ersten Referenz-Repräsentation (RR1) und der zweiten Referenz-Repräsentation (RR2) und des von dem ersten Teilmodell ($TM1^T$) ermittelten mindestens einen Modellparameters (MP) eine synthetische dritte Referenz-Repräsentation (RR3*) zu erzeugen,

- Bereitstellen einer ersten Repräsentation (R1), wobei die erste Repräsentation (R1) einen Untersuchungsbereich eines Untersuchungsobjekts ohne Kontrastmittel oder nach Applikation einer ersten Menge eines Kontrastmittels repräsentiert,
- Bereitstellen einer zweiten Repräsentation (R2), wobei die zweite Repräsentation (R2) den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation (R1) und der zweiten Repräsentation (R2) dem trainierten Modell ($M^T$) des maschinellen Lernens,
- Empfangen einer synthetischen dritten Repräsentation (R3*) des Untersuchungsbereichs des Untersuchungsobjekts von dem trainierten Modell ($M^T$) des maschinellen Lernens, wobei die synthetische dritte Repräsentation (R3*) den Referenzbereich nach Applikation einer dritten Menge des Kontrastmittels repräsentiert, wobei die dritte Menge verschieden von der zweiten Menge, vorzugsweise größer als die zweite Menge ist,
- Ausgeben und/oder Speichern der synthetischen dritten Repräsentation (R3*) und/oder Übermitteln der synthetischen dritten Repräsentation (R3*) an ein separates Computersystem,

**dadurch gekennzeichnet, dass** das zweite Teilmodell (TM2) konfiguriert ist,

- die erste Repräsentation (R1) des Untersuchungsbereichs des Untersuchungsobjekts von der zweiten Repräsentation (R2) des Untersuchungsbereichs des Untersuchungsobjekts zu subtrahieren, wobei eine Differenz (R2-R1) gebildet wird,
- die Differenz (R2-R1) mit einem Verstärkungsfaktor ($\alpha$) zu multiplizieren, wobei der Verstärkungsfaktor ($\alpha$) eine positive oder negative reelle Zahl ist, wobei der mindestens eine von dem ersten Teilmodell ($TM1^T$) ermittelte Modellparameter (MP) den Verstärkungsfaktor ($\alpha$) umfasst,
- die mit dem Verstärkungsfaktor ($\alpha$) multiplizierte Differenz (R2-R1) auf die erste Repräsentation (R1) oder die zweite Repräsentation (R2) zu addieren.

**17.** Verwendung eines Kontrastmittels in einem radiologischen Untersuchungsverfahren umfassend:

- Bereitstellen eines trainierten Modells ($M^T$) des maschinellen Lernens,

◦ wobei das trainierte Modell ($M^T$) des maschinellen Lernens anhand von Trainingsdaten (TD) trainiert wurde,

◦ wobei die Trainingsdaten (TD) für jedes Referenzobjekt einer Vielzahl an Referenzobjekten (i) eine erste Referenz-Repräsentation (RR1) eines Referenzbereichs des Referenzobjekts und eine zweite Referenz-Repräsentation (RR2) des Referenzbereichs des Referenzobjekts als Eingabedaten und (ii) eine dritte Referenz-Repräsentation

sentation (RR3) des Referenzbereichs des Referenzobjekts als Zieldaten umfassen,

◦ wobei die erste Referenz- Repräsentation (RR1) den Referenzbereich ohne Kontrastmittel oder nach Applikation einer ersten Menge des Kontrastmittels repräsentiert,

◦ wobei die zweite Referenz-Repräsentation (RR2) den Referenzbereich nach Applikation einer zweiten Menge des Kontrastmittels repräsentiert, wobei die zweite Menge größer als die erste Menge ist,

◦ wobei die dritte Referenz-Repräsentation (RR3) den Referenzbereich nach Applikation einer dritten Menge des Kontrastmittels repräsentiert, wobei die dritte Menge verschieden von der zweiten Menge, vorzugsweise größer als die zweite Menge ist,

◦ wobei das trainierte Modell ($M^T$) des maschinellen Lernens zwei Teilmodelle umfasst: ein erstes Teilmodell ($TM1^T$) und ein zweites Teilmodell (TM2), wobei das erste Teilmodell ($TM1^T$) konfiguriert ist und trainiert wurde, auf Basis der ersten Referenz-Repräsentation (RR1) und der zweiten Referenz-Repräsentation (RR2) des Untersuchungsbereichs eines Referenzobjekts mindestens einen Modellparameter (MP) für das zweite Teilmodell (TM2) zu ermitteln, wobei das zweite Teilmodell (TM2) konfiguriert ist, auf Basis der ersten Referenz-Repräsentation (RR1) und der zweiten Referenz-Repräsentation (RR2) und des von dem ersten Teilmodell ($TM1^T$) ermittelten mindestens einen Modellparameters (MP) eine synthetische dritte Referenz-Repräsentation (RR3*) zu erzeugen,

- Bereitstellen einer ersten Repräsentation (R1), wobei die erste Repräsentation (R1) einen Untersuchungsbereich eines Untersuchungsobjekts ohne Kontrastmittel oder nach Applikation einer ersten Menge des Kontrastmittels repräsentiert,
- Bereitstellen einer zweiten Repräsentation (R2), wobei die zweite Repräsentation (R2) den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation (R1) und der zweiten Repräsentation (R2) dem trainierten Modell ($M^T$) des maschinellen Lernens,
- Empfangen einer synthetischen dritten Repräsentation (R3*) des Untersuchungsbereichs des Untersuchungsobjekts von dem trainierten Modell ($M^T$) des maschinellen Lernens, wobei die synthetische dritte Repräsentation (R3*) den Referenzbereich nach Applikation einer dritten Menge des Kontrastmittels repräsentiert, wobei die dritte Menge verschieden von der zweiten Menge, vorzugsweise größer als die zweite Menge ist,
- Ausgeben und/oder Speichern der synthetischen dritten Repräsentation (R3*) und/oder Übermitteln der synthetischen dritten Repräsentation (R3*) an ein separates Computersystem,

**dadurch gekennzeichnet, dass** das zweite Teilmodell (TM2) konfiguriert ist,

- die erste Repräsentation (R1) des Untersuchungsbereichs des Untersuchungsobjekts von der zweiten Repräsentation (R2) des Untersuchungsbereichs des Untersuchungsobjekts zu subtrahieren, wobei eine Differenz (R2-R1) gebildet wird,
- die Differenz (R2-R1) mit einem Verstärkungsfaktor ($\alpha$) zu multiplizieren, wobei der Verstärkungsfaktor ($\alpha$) eine positive oder negative reelle Zahl ist, wobei der mindestens eine von dem ersten Teilmodell ($TM1^T$) ermittelte Modellparameter (MP) den Verstärkungsfaktor ($\alpha$) umfasst,
- die mit dem Verstärkungsfaktor ($\alpha$) multiplizierte Differenz (R2-R1) auf die erste Repräsentation (R1) oder die zweite Repräsentation (R2) zu addieren.

**18.** Kit umfassend ein Computerprogrammprodukt gemäß Anspruch 16 und ein Kontrastmittel, wobei das Kontrastmittel vorzugsweise

- einen $Gd^{3+}$-Komplex einer Verbindung der Formel (I)

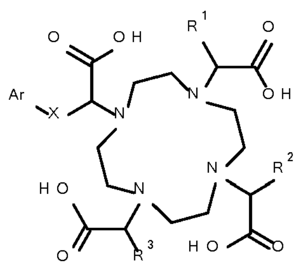

(I) ,

umfasst, wobei
Ar eine Gruppe ausgewählt aus

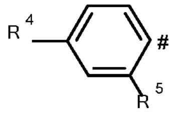 und 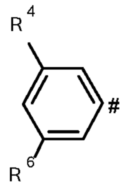

darstellt,
wobei # die Anknüpfung zu X darstellt,
X eine Gruppe darstellt, die aus $CH_2$, $(CH_2)_2$, $(CH_2)_3$, $(CH_2)_4$ und $*-(CH_2)_2-O-CH_2-\#$ ausgewählt wird,
wobei * die Anknüpfung zu Ar darstellt und # die Anknüpfung zum Essigsäurerest darstellt,
$R^1$, $R^2$ and $R^3$ unabhängig voneinander ein Wasserstoffatom oder eine Gruppe ausgewählt aus $C_1$-$C_3$-Alkyl, $-CH_2OH$, $-(CH_2)_2OH$ und $-CH_2OCH_3$ darstellen,
$R^4$ eine Gruppe ausgewählt aus $C_2$-$C_4$-Alkoxy, $(H_3C-CH_2)-O-(CH_2)_2-O-$, $(H_3C-CH_2)-O-(CH_2)_2-O-(CH_2)_2-O-$ und $(H_3C-CH_2)-O-(CH_2)_2-O-(CH_2)_2-O-(CH_2)_2-O-$ darstellt,
$R^5$ ein Wasserstoffatom darstellt,
und
$R^6$ ein Wasserstoffatom darstellt,
oder ein Stereoisomer, Tautomer, Hydrat, Solvat oder Salz davon, oder eine Mischung davon, oder

- einen $Gd^{3+}$-Komplex einer Verbindung der Formel (II)

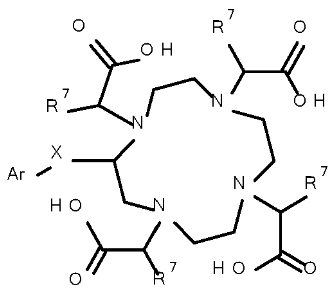

(II) ,

umfasst, wobei
Ar eine Gruppe ausgewählt aus

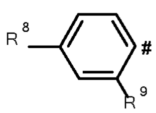 and 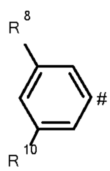

darstellt,
wobei # die Anknüpfung zu X darstellt,
X eine Gruppe darstellt, die aus $CH_2$, $(CH_2)_2$, $(CH_2)_3$, $(CH_2)_4$ and $*-(CH_2)_2-O-CH_2-\#$ ausgewählt wird, wobei * die Anknüpfung zu Ar darstellt und # die Anknüpfung zum Essigsäurerest darstellt,
$R^7$ ein Wasserstoffatom oder eine Gruppe ausgewählt aus $C_1$-$C_3$-Alkyl, $-CH_2OH$, $-(CH_2)_2OH$ und $-CH_2OCH_3$ darstellt;
$R^8$ eine Gruppe ausgewählt aus $C_2$-$C_4$-Alkoxy, $(H_3C-CH_2O)-(CH_2)_2-O-$, $(H_3C-CH_2O)-(CH_2)_2-O-(CH_2)_2-O-$ und $(H_3C-CH_2O)-(CH_2)_2-O-(CH_2)_2-O-(CH_2)_2-O-$ darstellt;
$R^9$ und $R^{10}$ unabhängig voneinander ein Wasserstoffatom darstellen;
oder ein Stereoisomer, Tautomer, Hydrat, Solvat oder Salz davon, oder eine Mischung davon, oder

- das Kontrastmittel eine der folgenden Substanzen umfasst:

- Gadolinium(III) 2-[4,7,10-tris(carboxymethyl)-1,4,7,10-tetrazacyclododec-1-yl]essigsäure,
- Gadolinium(III) Ethoxybenzyl-diethylenetriaminepentaessigsäure,
- Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat,
- Dihydrogen[(±)-4-carboxy-5,8,11-tris(carboxymethyl)-1-phenyl-2-oxa-5,8,11-triazatridecan-13-oato(5-)]gadolinat(2-),
- Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazaheptadecan-2-yl}-1,4,7,10-tetraazacyclododecan-1-yl]acetat,
- 2,2',2''-(10-{1-carboxy-2-[2-(4-ethoxyphenyl)ethoxy]ethyl}-1,4,7,10-tetraazacyclododecan-1,4,7-triyl)triacetat,
- Gadolinium 2,2',2''-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2''-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
- Gadolinium

(2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat)
- Gadolinium 2,2',2"-{10-[(1S)-4-(4-butoxyphenyl)-1-carboxybutyl]-1,4,7,10-tetraazacyclododecan-1,4,7-triyl}triacetat,
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl]acetat,
- Gadolinium(III) 2,2',2"-(10-((2R,3S)-1,3,4-trihydroxybutan-2-yl)-1,4,7,10-tetraazacyclododecan-1,4,7-triyl)triacetat,
- Gadolinium-2,2',2"-{(2S)-10-(carboxymethyl)-2-[4-(2-ethoxyethoxy)benzyl]-1,4,7,10-tetraazacyclododecane-1,4,7-triyl}triacetat,
- Gadolinium-2,2',2"-[10-(carboxymethyl)-2-(4-ethoxybenzyl)-1,4,7,10-tetraazacyclododecane-1,4,7-triyl]triacetat.

## Claims

**1.** Computer-implemented method comprising the steps of:

- providing a trained machine-learning model ($M^T$),

o where the trained machine-learning model ($M^T$) has been trained on the basis of training data (TD),
o where the training data (TD) for each reference object of a multiplicity of reference objects comprise (i) a first reference representation (RR1) of a reference region of the reference object and a second reference representation (RR2) of the reference region of the reference object as input data and (ii) a third reference representation (RR3) of the reference region of the reference object as target data,
o where the first reference representation (RR1) represents the reference region without contrast agent or after administration of a first amount of a contrast agent,
o where the second reference representation (RR2) represents the reference region after administration of a second amount of the contrast agent, the second amount being larger than the first amount,
o where the third reference representation (RR3) represents the reference region after administration of a third amount of the contrast agent, the third amount being different from the second amount, preferably larger than the second amount,
o where the trained machine-learning model ($M^T$) comprises two submodels: a first submodel ($TM1^T$) and a second submodel (TM2), where the first submodel ($TM1^T$) is configured and has been trained to determine, based on the first reference representation (RR1) and the second reference representation (RR2) of the examination region of a reference object, at least one model parameter (MP) for the second submodel (TM2), where the second submodel (TM2) is configured to generate, based on the first reference representation (RR1) and the second reference representation (RR2) and on the at least one model parameter (MP) determined by the first submodel ($TM1^T$), a synthetic third reference representation (RR3*),

- providing a first representation (R1), where the first representation (R1) represents an examination region of an examination object without contrast agent or after administration of a first amount of a contrast agent,
- providing a second representation (R2), where the second representation (R2) represents the examination region of the examination object after administration of a second amount of the contrast agent, the second amount being larger than the first amount,
- feeding the first representation (R1) and the second representation (R2) to the trained machine-learning model ($M^T$),
- receiving from the trained machine-learning model ($M^T$) a synthetic third representation (R3*) of the examination region of the examination object, where the synthetic third representation (R3*) represents the reference region after administration of a third amount of the contrast agent, the third amount being different from the second amount, preferably larger than the second amount,
- outputting and/or storing the synthetic third representation (R3*) and/or transmitting the synthetic third representation (R3*) to a separate computer system, **characterized in that** the second submodel (TM2) is configured
- to subtract the first representation (R1) of the examination region of the examination object from the second representation (R2) of the examination region of the examination object, wherein a difference (R2 - R1) is formed,
- to multiply the difference (R2 - R1) by a gain

factor ($\alpha$), where the gain factor ($\alpha$) is a positive or negative real number, where the at least one model parameter (MP) determined by the first submodel ($TM1^T$) includes the gain factor ($\alpha$),
- to add the difference (R2 - R1) multiplied by the gain factor ($\alpha$) to the first representation (R1) or the second representation (R2).

**2.** Method according to Claim 1, wherein the first representation (R1) and the second representation (R2) are representations ($R1^F$, $R2^F$) of the examination region of the examination object in frequency space, where the second submodel (TM2) is configured

- to multiply the difference ($R2^F$ - $R1^F$) by a frequency-dependent weight function (WF), wherein a weighted difference $(R2^F - R1^F)^W$ is formed, where the at least one model parameter (MP) determined by the first submodel ($TM1^T$) includes one or more parameters of the frequency-dependent weight function (WF),
- to multiply the weighted difference $(R2^F - R1^F)^W$ by the gain factor ($\alpha$),
- to add the weighted difference $(R2^F - R1^F)^W$ multiplied by the gain factor ($\alpha$) to the first representation ($R1^F$) or the second representation ($R2^F$).

**3.** Method according to either of Claims 1 and 2, wherein the first submodel ($TM1^T$) is an artificial neural network or includes such a network.

**4.** Method according to any of Claims 1 to 3, wherein the second submodel (TM2) is a mechanistic model.

**5.** Method according to any of Claims 1 to 4, wherein model parameters of the second submodel (TM2) are not trainable parameters.

**6.** Method according to any of Claims 1 to 5, wherein the machine-learning model ($M^T$) is differentiable and the training takes place in an end-to-end manner.

**7.** Method according to any of Claims 1 to 6, wherein the machine-learning model ($M^T$) includes a third submodel ($TM3^T$), where the third submodel ($TM3^T$) is configured and has been trained to generate, based on the synthetic third representation (R3*) generated by the second submodel (TM2), a corrected third representation ($R3^{*C}$), where the step of receiving from the trained machine-learning model ($M^T$) a synthetic third representation (R3*) of the examination region of the examination object comprises: receiving from the trained machine-learning model ($M^T$) the corrected third representation ($R3^{*C}$) of the examination region of the examination object, where the corrected third representation ($R3^{*C}$) represents the reference region after administration of the third amount of the contrast agent, where the step of outputting and/or storing the synthetic third representation (R3*) and/or transmitting the synthetic third representation (R3*) to a separate computer system comprises: outputting and/or storing the corrected third representation ($R3^{*C}$) and/or transmitting the corrected third representation ($R3^{*C}$) to a separate computer system.

**8.** Method according to Claim 7, wherein the third submodel ($TM3^T$) is a trained machine-learning model, where the third submodel ($TM3^T$) is preferably an artificial neural network or includes such a network.

**9.** Method according to any of Claims 1 to 8, wherein the trained machine-learning model ($M^T$) has been trained, where the training of the trained machine-learning model ($M^T$) comprises the steps of:

- providing the training data (TD),
- providing a machine-learning model (M),
- training the machine-learning model (M), where the training comprises the steps of:

o feeding a first reference representation (RR1) and a second reference representation (RR2) of a reference region of a reference object to the first submodel (TM1), where the first submodel (TM1) is configured to determine, based on the first reference representation (RR1), on the second reference representation (RR2) and on model parameters, at least one model parameter (MP) for the second submodel (TM2),
o receiving the at least one model parameter (MP) determined by the first submodel (TM1),
o feeding the at least one model parameter (MP) that has been determined and the first reference representation (RR1) and/or the second reference representation (RR2) to the second submodel (TM2), where the second submodel (TM2) is configured to generate, based on the first reference representation (RR1) and/or the second reference representation (RR2) and on the model parameter (MP) determined by the first submodel (TM1), a synthetic third reference representation (RR3*),
o receiving the synthetic third reference representation (RR3*) generated by the second submodel (TM2),
o quantifying differences between the synthetic third reference representation (RR3*) and the third reference representation (RR3) of the training data,

o reducing the differences by modifying model parameters of the first submodel (TM1),

- storing the trained machine-learning model ($M^T$) and/or using the trained machine-learning model ($M^T$) to generate a synthetic representation (R3*) of an examination region of an examination object.

**10.** Method according to any of Claims 2 to 9, wherein the frequency-dependent weight function (WF) mentioned in Claim 2 is a Hann function, a Poisson function or a Gaussian function.

**11.** Method according to any of Claims 1 to 10, wherein the examination object is a human and the examination region is a part of the human, wherein each reference object is a human and the reference region of each such reference object is a part of the reference object, wherein the reference region of each such reference object and the examination region are the same part of the human.

**12.** Method according to any of Claims 1 to 11, wherein the first representation (R1) and the second representation (R2) and also each reference representation (RR1, RR2, RR3) of each such reference object is the result of a radiological examination, where the radiological examination is preferably an MRI examination or a CT examination and the contrast agent is an MRI contrast agent or a CT contrast agent.

**13.** Method according to any of Claims 1 to 12, wherein the first amount is equal to zero, the second amount is smaller than the standard amount of the contrast agent or equal to the standard amount of the contrast agent and the third amount is larger than the standard amount of the contrast agent.

**14.** Method according to any of Claims 1 to 13, wherein the contrast agent comprises

- a $Gd^{3+}$ complex of a compound of the formula (I)

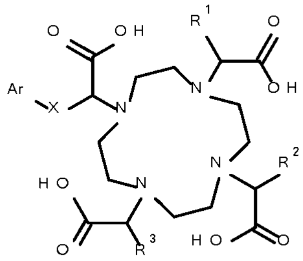

(I),

where

Ar is a group selected from

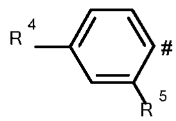

and

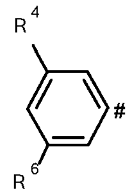

,

where # is the linkage to X,
X is a group selected from
$CH_2$, $(CH_2)_2$, $(CH_2)_3$, $(CH_2)_4$ and *-$(CH_2)_2$-O-$CH_2$-#,
where * is the linkage to Ar and # is the linkage to the acetic acid residue,
$R^1$, $R^2$ and $R^3$ are independently a hydrogen atom or a group selected from $C_1$-$C_3$ alkyl, -$CH_2OH$, -$(CH_2)_2OH$ and -$CH_2OCH_3$,
$R^4$ is a group selected from $C_2$-$C_4$ alkoxy, ($H_3C$-$CH_2$)-O-$(CH_2)_2$-O-, ($H_3C$-$CH_2$)-O-$(CH_2)_2$-O-$(CH_2)_2$-O-and ($H_3C$-$CH_2$)-O-$(CH_2)_2$-O-$(CH_2)_2$-O-$(CH_2)_2$-O-,
$R^5$ is a hydrogen atom,
and
$R^6$ is a hydrogen atom,
or a stereoisomer, tautomer, hydrate, solvate or salt thereof, or a mixture thereof, or

- a $Gd^{3+}$ complex of a compound of the formula (II)

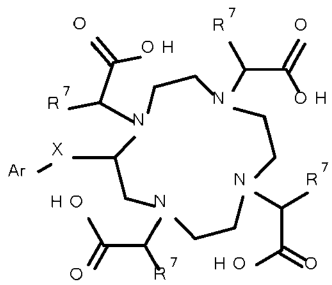

(II),

where

Ar is a group selected from

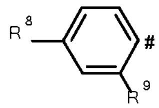

and

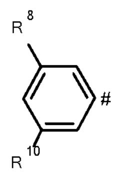

,

where # is the linkage to X,
X is a group selected from $CH_2$, $(CH_2)_2$, $(CH_2)_3$, $(CH_2)_4$ and *- $(CH_2)_2$-O-$CH_2$-#, where * is the linkage to Ar and # is the linkage to the acetic acid residue,
$R^7$ is a hydrogen atom or a group selected from $C_1$-$C_3$ alkyl, -$CH_2OH$, -$(CH_2)_2OH$ and -$CH_2OCH_3$;
$R^8$ is a group selected from $C_2$-$C_4$ alkoxy, $(H_3C$-$CH_2O)$- $(CH_2)_2$-O-, $(H_3C$-$CH_2O)$-$(CH_2)_2$-O-$(CH_2)_2$-O-and $(H_3C$-$CH_2O)$- $(CH_2)_2$-O-$(CH_2)_2$-O-$(CH_2)_2$-O-;
$R^9$ and $R^{10}$ are independently a hydrogen atom;
or a stereoisomer, tautomer, hydrate, solvate or salt thereof, or a mixture thereof, or the contrast agent comprises one of the following substances:

- gadolinium(III) 2-[4,7,10-tris(carboxymethyl)-1,4,7,10-tetrazacyclododec-1-yl]acetic acid,
- gadolinium(III) ethoxybenzyldiethylenetriaminepentaacetic acid,
- gadolinium(III) 2-[3,9-bis[1-carboxylato-4-(2,3-dihydroxypropylamino)-4-oxobutyl]-3,6,9,15-tetrazabicyclo [9.3.1]pentadeca-1(15),11,13-trien-6-yl]-5-(2,3-dihydroxypropylamino)-5-oxopentanoate,
- dihydrogen [(±)-4-carboxy-5,8,11-tris(carboxymethyl)-1-phenyl-2-oxa-5,8,11-triazatridecan-13-oato(5-)] gadolinate(2-),
- tetragadolinium [4,10-bis(carboxylatomethyl)-7-{3,6,12,15-tetraoxo-16-[4,7,10-tris(carboxylatomethyl)-1,4,7,10-tetraazacyclododecan-1-yl]-9,9-bis({[({2-[4,7,10-tris(carboxylatomethyl)-1,4,7,10-tetraazacyclododecan-1-yl]propanoyl}amino) acetyl]amino}methyl)-4,7,11,14-tetraazaheptadecan-2-yl}-1,4,7,10-tetraazacyclododecan-1-yl]acetate,
- 2,2',2"-(10-{1-carboxy-2-[2-(4-ethoxyphenyl)ethoxy]ethyl}-1,4,7,10-tetraazacyclododecane-1,4,7-triyl)triacetate,
- gadolinium 2,2',2"-{10-[1-carboxy-2-{4-[2-(2-ethoxyethoxy)ethoxy] phenyl}ethyl]-1,4,7,10-tetraazacyclododecane-1,4,7-triyl}triacetate,
- gadolinium 2,2',2"-{10-[(1R)-1-carboxy-2-{4-[2-(2-ethoxyethoxy)ethoxy] phenyl}ethyl]-1,4,7,10-tetraazacyclododecane-1,4,7-triyl}triacetate,
- gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
- gadolinium 2,2',2"-{10-[(1S)-4-(4-butoxyphenyl)-1-carboxybutyl]-1,4,7,10-tetraazacyclododecane-1,4,7-triyl}triacetate,
- gadolinium(III) 5,8-bis(carboxylatomethyl)-2-[2-(methylamino)-2-oxoethyl]-10-oxo-2,5,8,11-tetraazadodecane-1-carboxylate hydrate,
- gadolinium(III) 2-[4-(2-hydroxypropyl)-7,10-bis(2-oxido-2-oxoethyl)-1,4,7,10-tetrazacyclododec-1-yl]acetate,
- gadolinium (III) 2,2',2"-(10-((2R,3S)-1,3,4-trihydroxybutan-2-yl)-1,4,7,10-tetraazacyclododecane-1,4,7-triyl)triacetate,
- gadolinium-2,2',2"-{ (2S)-10-(carboxymethyl)-2-[4-(2-ethoxyethoxy)benzyl]-1,4,7,10-tetraazacyclododecane-1,4,7-triyl}triacetate,
- gadolinium-2,2',2"-[10-(carboxymethyl)-2-(4-ethoxybenzyl)-1,4,7,10-tetraazacyclododecane-1,4,7-triyl]triacetate.

**15.** Computer system (1) comprising

• a receiving unit (11),
• a control and calculation unit (12), and
• an output unit (13),
wherein the control and calculation unit (12) is configured

- to generate a first representation (R1) or cause the receiving unit (11) to receive a first representation (R1), where the first representation (R1) represents an examination region of an examination object without contrast agent or after administration of a first amount of a contrast agent,
- to generate a second representation (R2) or to cause the receiving unit (11) to receive a second representation (R2), where the second representation (R2) represents the examination region of the examination object after administration of a second amount of the contrast agent, the second amount being larger than the first amount,
- to feed the first representation (R1) and the second representation (R2) to a trained machine-learning model ($M^T$),

o where the trained machine-learning model ($M^T$) has been trained on the

basis of training data (TD),

o where the training data (TD) for each reference object of a multiplicity of reference objects comprise (i) a first reference representation (RR1) of a reference region of the reference object and a second reference representation (RR2) of the reference region of the reference object as input data and (ii) a third reference representation (RR3) of the reference region of the reference object as target data,

o where the first reference representation (RR1) represents the reference region without contrast agent or after administration of a first amount of a contrast agent,

o where the second reference representation (RR2) represents the reference region after administration of a second amount of the contrast agent, the second amount being larger than the first amount,

o where the third reference representation (RR3) represents the reference region after administration of a third amount of the contrast agent, the third amount being different from the second amount, preferably larger than the second amount,

o where the trained machine-learning model ($M^T$) comprises two submodels: a first submodel ($TM1^T$) and a second submodel (TM2), where the first submodel ($TM1^T$) is configured and has been trained to determine, based on the first reference representation (RR1) and the second reference representation (RR2) of each such reference object, at least one model parameter (MP) for the second submodel (TM2), where the second submodel (TM2) is configured to generate, based on the first reference representation (RR1) and the second reference representation (RR2) and on the at least one model parameter (MP) determined by the first submodel ($TM1^T$), a synthetic third reference representation (RR3*),

- to receive from the trained machine-learning model ($M^T$) a synthetic third representation (R3*) of the examination region of the examination object, where the synthetic third representation (R3*) represents the reference region after administration of a third amount of the contrast agent, the third amount being different from the second amount, preferably larger than the second amount,

- to cause the output unit (13) to output the synthetic third representation (R3*) and/or to store it in a data storage medium and/or to transmit it to a separate computer system,

**characterized in that** the second submodel (TM2) is configured

- to subtract the first representation (R1) of the examination region of the examination object from the second representation (R2) of the examination region of the examination object, wherein a difference (R2 - R1) is formed,

- to multiply the difference (R2 - R1) by a gain factor ($\alpha$), where the gain factor ($\alpha$) is a positive or negative real number, where the at least one model parameter (MP) determined by the first submodel ($TM1^T$) includes the gain factor ($\alpha$),

- to add the difference (R2 - R1) multiplied by the gain factor ($\alpha$) to the first representation (R1) or the second representation (R2).

**16.** Computer program product comprising a data carrier on which there is stored a computer program (40), where the computer program (40) can be loaded into a working memory (22) of a computer system (1), where it causes the computer system (1) to execute the following steps:

- providing a trained machine-learning model ($M^T$),

o where the trained machine-learning model ($M^T$) has been trained on the basis of training data (TD),

o where the training data (TD) for each reference object of a multiplicity of reference objects comprise (i) a first reference representation (RR1) of a reference region of the reference object and a second reference representation (RR2) of the reference region of the reference object as input data and (ii) a third reference representation (RR3) of the reference region of the reference object as target data,

o where the first reference representation (RR1) represents the reference region without contrast agent or after administration of a first amount of a contrast agent,

o where the second reference representation (RR2) represents the reference region after administration of a second amount of the contrast agent, the second amount being larger than the first amount,

o where the third reference representation (RR3) represents the reference region after administration of a third amount of the contrast agent, the third amount being different from the second amount, preferably larger than the second amount,

o where the trained machine-learning model ($M^T$) comprises two submodels: a first submodel ($TM1^T$) and a second submodel (TM2), where the first submodel ($TM1^T$) is configured and has been trained to determine, based on the first reference representation (RR1) and the second reference representation (RR2) of the examination region of a reference object, at least one model parameter (MP) for the second submodel (TM2), where the second submodel (TM2) is configured to generate, based on the first reference representation (RR1) and the second reference representation (RR2) and on the at least one model parameter (MP) determined by the first submodel ($TM1^T$), a synthetic third reference representation (RR3*),

- providing a first representation (R1), where the first representation (R1) represents an examination region of an examination object without contrast agent or after administration of a first amount of a contrast agent,
- providing a second representation (R2), where the second representation (R2) represents the examination region of the examination object after administration of a second amount of the contrast agent, the second amount being larger than the first amount,
- feeding the first representation (R1) and the second representation (R2) to the trained machine-learning model ($M^T$),
- receiving from the trained machine-learning model ($M^T$) a synthetic third representation (R3*) of the examination region of the examination object, where the synthetic third representation (R3*) represents the reference region after administration of a third amount of the contrast agent, the third amount being different from the second amount, preferably larger than the second amount,
- outputting and/or storing the synthetic third representation (R3*) and/or transmitting the synthetic third representation (R3*) to a separate computer system, **characterized in that** the second submodel (TM2) is configured
- to subtract the first representation (R1) of the examination region of the examination object from the second representation (R2) of the examination region of the examination object, wherein a difference (R2 - R1) is formed,
- to multiply the difference (R2 - R1) by a gain factor ($\alpha$), where the gain factor ($\alpha$) is a positive or negative real number, where the at least one model parameter (MP) determined by the first submodel ($TM1^T$) includes the gain factor ($\alpha$),
- to add the difference (R2 - R1) multiplied by the gain factor ($\alpha$) to the first representation (R1) or the second representation (R2).

**17.** Use of a contrast agent in a radiological examination method comprising the steps of:

- providing a trained machine-learning model ($M^T$),

o where the trained machine-learning model ($M^T$) has been trained on the basis of training data (TD),

o where the training data (TD) for each reference object of a multiplicity of reference objects comprise (i) a first reference representation (RR1) of a reference region of the reference object and a second reference representation (RR2) of the reference region of the reference object as input data and (ii) a third reference representation (RR3) of the reference region of the reference object as target data,

o where the first reference representation (RR1) represents the reference region without contrast agent or after administration of a first amount of the contrast agent,

o where the second reference representation (RR2) represents the reference region after administration of a second amount of the contrast agent, the second amount being larger than the first amount,

o where the third reference representation (RR3) represents the reference region after administration of a third amount of the contrast agent, the third amount being different from the second amount, preferably larger than the second amount,

o where the trained machine-learning model ($M^T$) comprises two submodels: a first submodel ($TM1^T$) and a second submodel (TM2), where the first submodel ($TM1^T$) is configured and has been trained to determine, based on the first reference representation (RR1) and the second reference representation (RR2) of the examination region of a reference object, at least one model parameter (MP) for the second submodel (TM2), where the second submodel (TM2) is configured to generate, based on the first reference representation (RR1) and the second reference representation (RR2) and on the at least one model parameter

(MP) determined by the first submodel ($TM1^T$), a synthetic third reference representation (RR3*),

- providing a first representation (R1), where the first representation (R1) represents an examination region of an examination object without contrast agent or after administration of a first amount of the contrast agent,
- providing a second representation (R2), where the second representation (R2) represents the examination region of the examination object after administration of a second amount of the contrast agent, the second amount being larger than the first amount,
- feeding the first representation (R1) and the second representation (R2) to the trained machine-learning model ($M^T$),
- receiving from the trained machine-learning model ($M^T$) a synthetic third representation (R3*) of the examination region of the examination object, where the synthetic third representation (R3*) represents the reference region after administration of a third amount of the contrast agent, the third amount being different from the second amount, preferably larger than the second amount,
- outputting and/or storing the synthetic third representation (R3*) and/or transmitting the synthetic third representation (R3*) to a separate computer system, **characterized in that** the second submodel (TM2) is configured
- to subtract the first representation (R1) of the examination region of the examination object from the second representation (R2) of the examination region of the examination object, wherein a difference (R2 - R1) is formed,
- to multiply the difference (R2 - R1) by a gain factor ($\alpha$), where the gain factor ($\alpha$) is a positive or negative real number, where the at least one model parameter (MP) determined by the first submodel ($TM1^T$) includes the gain factor ($\alpha$),
- to add the difference (R2 - R1) multiplied by the gain factor ($\alpha$) to the first representation (R1) or the second representation (R2).

**18.** Kit comprising a computer program product according to Claim 16 and a contrast agent, wherein the contrast agent preferably comprises

- a $Gd^{3+}$ complex of a compound of the formula (I)

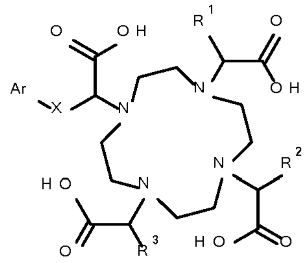

(I),

where

Ar is a group selected from

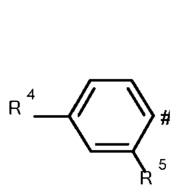

and

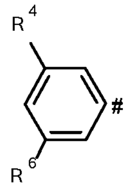

,

where # is the linkage to X,
X is a group selected from $CH_2$, $(CH_2)_2$, $(CH_2)_3$, $(CH_2)_4$ and *-$(CH_2)_2$-O-$CH_2$-#,
where * is the linkage to Ar and # is the linkage to the acetic acid residue,
$R^1$, $R^2$ and $R^3$ are independently a hydrogen atom or a group selected from $C_1$-$C_3$ alkyl, -$CH_2OH$, -$(CH_2)_2OH$ and -$CH_2OCH_3$,
$R^4$ is a group selected from $C_2$-$C_4$ alkoxy, ($H_3C$-$CH_2$)-O-$(CH_2)_2$-O-, ($H_3C$-$CH_2$)-O-$(CH_2)_2$-O-$(CH_2)_2$-O-and ($H_3C$-$CH_2$)-O-$(CH_2)_2$-O-$(CH_2)_2$-O-$(CH_2)_2$-O-,
$R^5$ is a hydrogen atom,
and
$R^6$ is a hydrogen atom,
or a stereoisomer, tautomer, hydrate, solvate or salt thereof, or a mixture thereof, or

- a $Gd^{3+}$ complex of a compound of the formula (II)

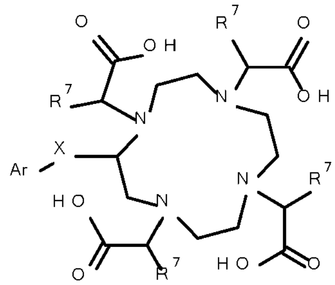

(II),

where

Ar is a group selected from

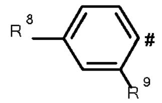 and 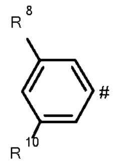 ,

where # is the linkage to X,
X is a group selected from $CH_2$, $(CH_2)_2$, $(CH_2)_3$, $(CH_2)_4$ and *-$(CH_2)_2$-O-$CH_2$-#, where * is the linkage to Ar and # is the linkage to the acetic acid residue,
$R^7$ is a hydrogen atom or a group selected from $C_1$-$C_3$ alkyl, -$CH_2OH$, -$(CH_2)_2OH$ and -$CH_2OCH_3$;
$R^8$ is a group selected from $C_2$-$C_4$ alkoxy, ($H_3C$-$CH_2O$)-$(CH_2)_2$-O-, ($H_3C$-$CH_2O$)-$(CH_2)_2$-O-$(CH_2)_2$-O- and ($H_3C$-$CH_2O$)- $(CH_2)_2$-O-$(CH_2)_2$-O-$(CH_2)_2$-O-;
$R^9$ and $R^{10}$ are independently a hydrogen atom;
or a stereoisomer, tautomer, hydrate, solvate or salt thereof, or a mixture thereof, or

- the contrast agent comprises one of the following substances:
- gadolinium(III) 2-[4,7,10-tris(carboxymethyl)-1,4,7,10-tetrazacyclododec-1-yl]acetic acid,
- gadolinium(III) ethoxybenzyldiethylenetriaminepentaacetic acid,
- gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoate,
- dihydrogen [(±)-4-carboxy-5,8,11-tris(carboxymethyl)-1-phenyl-2-oxa-5,8,11-triazatridecan-13-oato(5-)]gadolinate(2-),
- tetragadolinium [4,10-bis(carboxylatomethyl)-7-{3,6,12,15-tetraoxo-16-[4,7,10-tris(carboxylatomethyl)-1,4,7,10-tetraazacyclododecan-1-yl]-9,9-bis({[({2-[4,7,10-tris(carboxylatomethyl)-1,4,7,10-tetraazacyclododecan-1-yl]propanoyl}amino)acetyl]amino}methyl)-4,7,11,14-tetraazaheptadecan-2-yl}-1,4,7,10-tetraazacyclododecan-1-yl]acetate,
- 2,2',2''-(10-{1-carboxy-2-[2-(4-ethoxyphenyl)ethoxy]ethyl}-1,4,7,10-tetraazacyclododecane-1,4,7-triyl)triacetate,
- gadolinium 2,2',2''-{10-[1-carboxy-2-{4-[2-(2-ethoxyethoxy)ethoxy]phenyl}ethyl]-1,4,7,10-tetraazacyclododecane-1,4,7-triyl}triacetate,
- gadolinium 2,2',2''-{10-[(1R)-1-carboxy-2-{4-[2-(2-ethoxyethoxy)ethoxy]phenyl}ethyl]-1,4,7,10-tetraazacyclododecane-1,4,7-triyl}triacetate,
- gadolinium (2S,2'S,2''S)-2,2',2''-{10-[(1S)-1-carboxy-4-{4-[2-(2-ethoxyethoxy)ethoxy]phenyl}butyl]-1,4,7,10-tetraazacyclododecane-1,4,7-triyl}tris(3-hydroxypropanoate),
- gadolinium 2,2',2''-{10-[(1S)-4-(4-butoxyphenyl)-1-carboxybutyl]-1,4,7,10-tetraazacyclododecane-1,4,7-triyl}triacetate,
- gadolinium(III) 5,8-bis(carboxylatomethyl)-2-[2-(methylamino)-2-oxoethyl]-10-oxo-2,5,8,11-tetraazadodecane-1-carboxylate hydrate,
- gadolinium(III) 2-[4-(2-hydroxypropyl)-7,10-bis(2-oxido-2-oxoethyl)-1,4,7,10-tetrazacyclododec-1-yl]acetate,
- gadolinium (III) 2,2',2''-(10-((2R,3S)-1,3,4-trihydroxybutan-2-yl)-1,4,7,10-tetraazacyclododecane-1,4,7-triyl)triacetate,
- gadolinium-2,2',2''-{(2S)-10-(carboxymethyl)-2-[4-(2-ethoxyethoxy)benzyl]-1,4,7,10-tetraazacyclododecane-1,4,7-triyl}triacetate,
- gadolinium-2,2',2''-[10-(carboxymethyl)-2-(4-ethoxybenzyl)-1,4,7,10-tetraazacyclododecane-1,4,7-triyl]triacetate.

## Revendications

**1.** Procédé mis en œuvre par ordinateur, comprenant les étapes consistant à :

- fournir un modèle ($M^T$) d'apprentissage automatique entraîné ,

o le modèle ($M^T$) d'apprentissage automatique entraîné ayant été entraîné sur la base de données d'apprentissage (TD),
o les données d'apprentissage (TD), pour chaque objet de référence d'une pluralité d'objets de référence comprenant (i) une première représentation de référence (RR1) d'une zone de référence de l'objet de référence et une deuxième représentation de référence (RR2) de la zone de référence de l'objet de référence en tant que données d'entrée et (ii) une troisième représentation de référence (RR3) de la zone de référence de l'objet de référence en tant que données cibles,
o la première représentation de référence (RR1) représentant la zone de référence sans agent de contraste ou après l'application d'une première quantité d'un agent de contraste,
o la deuxième représentation de référence

(RR2) représentant la zone de référence après l'application d'une deuxième quantité de l'agent de contraste, la deuxième quantité étant supérieure à la première quantité,
o la troisième représentation de référence (RR3) représentant la zone de référence après l'application d'une troisième quantité de l'agent de contraste, la troisième quantité étant différente de la deuxième quantité, de préférence supérieure à la deuxième quantité,
o le modèle ($M^T$) d'apprentissage automatique entraîné comprenant deux modèles partiels : un premier modèle partiel ($TM1^T$) et un deuxième modèle partiel (TM2), le premier modèle partiel ($TM1^T$) étant configuré et ayant été entraîné pour déterminer, sur la base de la première représentation de référence (RR1) et de la deuxième représentation de référence (RR2) de la zone d'examen d'un objet de référence, au moins un paramètre de modèle (MP) pour le deuxième modèle partiel (TM2), le deuxième modèle partiel (TM2) étant configuré pour générer, sur la base de la première représentation de référence (RR1) et de la deuxième représentation de référence (RR2) et dudit au moins un paramètre de modèle (MP) déterminé par le premier modèle partiel ($TM1^T$), une troisième représentation de référence synthétique (RR3*),

- fournir une première représentation (R1), la première représentation (R1) représentant une zone d'examen d'un objet d'examen sans agent de contraste ou après l'application d'une première quantité d'un agent de contraste,
- fournir une deuxième représentation (R2), la deuxième représentation (R2) représentant la zone d'examen de l'objet d'examen après l'application d'une deuxième quantité de l'agent de contraste, la deuxième quantité étant supérieure à la première quantité,
- appliquer la première représentation (R1) et la deuxième représentation (R2) au modèle ($M^T$) d'apprentissage automatique entraîné,
- recevoir une troisième représentation synthétique (R3*) de la zone d'examen de l'objet d'examen en provenance du modèle ($M^T$) d'apprentissage automatique entraîné, la troisième représentation synthétique (R3*) représentant la zone de référence après l'application d'une troisième quantité du produit de contraste, la troisième quantité étant différente de la deuxième quantité, de préférence supérieure à la deuxième quantité,
- délivrer et/ou stocker la troisième représentation synthétique (R3*) et/ou transférer la troisième représentation corrigée (R3*) à un système informatique séparé,

**caractérisé en ce que** le deuxième modèle partiel (TM2) est configuré pour

- soustraire la première représentation (R1) de la zone d'examen de l'objet examen à la deuxième représentation (R2) de la zone d'examen de l'objet examen, une différence (R2-R1) étant calculée,
- multiplier la différence (R2-R1) par un facteur d'amplification ($\alpha$), le facteur d'amplification ($\alpha$) étant un nombre réel positif ou négatif, ledit au moins un paramètre de modèle (MP) déterminé par le premier modèle partiel ($TM1^T$) comprenant le facteur d'amplification ($\alpha$),
- additionner la différence (R2-R1) multipliée par le facteur d'amplification ($\alpha$) à la première représentation (R1) ou à la deuxième représentation (R2).

**2.** Procédé selon la revendication 1, dans lequel la première représentation (R1) et la deuxième représentation (R2) sont des représentations ($R1^F$, $R2^F$) dans l'espace fréquentiel de la zone d'examen de l'objet examen, le deuxième modèle partiel (TM2) étant configuré pour

- multiplier la différence ($R2^F$-$R1^F$) par une fonction de pondération (WF) dépendant de la fréquence, une différence pondérée $(R2^F-R1^F)^W$ étant calculée, ledit au moins un paramètre de modèle (MP) déterminé par le premier modèle partiel ($TM1^T$) comprenant un ou plusieurs paramètres de la fonction de pondération dépendant de la fréquence (WF),
- multiplier la différence pondérée $(R2^F-R1^F)^W$ par le facteur d'amplification ($\alpha$),
- additionner la différence pondérée $(R2^F-R1^F)^W$ multipliée par le facteur d'amplification ($\alpha$) à la première représentation ($R1^F$) ou à la deuxième représentation ($R2^F$).

**3.** Procédé selon l'une quelconque des revendications 1 à 2, dans lequel le modèle partiel ($TM1^T$) est ou comprend un réseau neuronal artificiel.

**4.** Procédé selon l'une quelconque des revendications 1 à 3, dans lequel le deuxième modèle partiel (TM2) est un modèle mécaniste.

**5.** Procédé selon l'une quelconque des revendications 1 à 4, dans lequel les paramètres de modèle du deuxième modèle partiel (TM2) ne sont pas des paramètres pouvant être entraînés.

**6.** Procédé selon l'une quelconque des revendications

1 à 5, dans lequel le modèle ($M^T$) d'apprentissage automatique est différentiable et l'entraînement a été effectué de bout en bout.

**7.** Procédé selon l'une quelconque des revendications 1 à 6, dans lequel le modèle ($M^T$) d'apprentissage automatique comprend un troisième modèle partiel ($TM3^T$), le troisième modèle partiel ($TM3^T$) étant configuré et ayant été entraîné pour générer, sur la base de la troisième représentation synthétique (R3*) générée par le deuxième modèle partiel (TM2), une troisième représentation corrigée ($R3^{*C}$), l'étape de réception d'une troisième représentation synthétique (R3*) de la zone d'examen de l'objet d'examen en provenance du modèle ($M^T$) d'apprentissage automatique entraîné comprenant les étapes consistant à : recevoir la troisième représentation corrigée ($R3^{*C}$) de la zone d'examen de l'objet d'examen en provenance du modèle ($M^T$) d'apprentissage automatique entraîné, la troisième représentation corrigée ($R3^{*C}$) représentant la zone de référence après l'application de la troisième quantité d'un agent de contraste, l'étape consistant à délivrer et/ou stocker la troisième représentation synthétique (R3*) et/ou transférer la troisième représentation synthétique (R3*) à un système informatique séparé consistant à : - délivrer et/ou stocker la troisième représentation corrigée ($R3^{*C}$) et/ou transférer la troisième représentation corrigée ($R3^{*C}$) à un système informatique séparé.

**8.** Procédé selon la revendication 7, dans lequel le troisième modèle partiel ($TM3^T$) est un modèle d'apprentissage automatique entraîné, le troisième modèle partiel ($TM3^T$) étant ou comprenant de préférence un réseau neuronal artificiel.

**9.** Procédé selon l'une quelconque des revendications 1 à 8, dans lequel le modèle ($M^T$) d'apprentissage automatique entraîné a été entraîné, l'entraînement du modèle ($M^T$) d'apprentissage automatique entraîné comprenant les étapes consistant à :

- fournir les données d'apprentissage (TD),
- fournir un modèle (M) d'apprentissage automatique,
- entraîner le modèle (M) d'apprentissage automatique, l'entraînement comprenant les étapes consistant à :

o appliquer une première représentation de référence (RR1) et une deuxième représentation de référence (RR2) d'une zone de référence d'un objet de référence au premier modèle partiel (TM1), le premier modèle partiel (TM1) étant configuré pour déterminer, sur la base de la première représentation de référence (RR1), de la deuxième représentation de référence (RR2) et des paramètres de modèle, au moins un paramètre de modèle (MP) pour le deuxième modèle partiel (TM2),
o recevoir ledit au moins un paramètre de modèle (MP) déterminé par le premier modèle partiel (TM1),
o appliquer ledit au moins un paramètre de modèle déterminé (MP) et la première représentation de référence (RR1) et/ou la deuxième représentation de référence (RR2) au deuxième modèle partiel (TM2), le deuxième modèle partiel (TM2) étant configuré pour générer, sur la base de la première représentation de référence (RR1) et/ou de la deuxième représentation de référence (RR2) et du paramètre de modèle (MP) déterminé par le premier modèle partiel (TM1), une troisième représentation de référence synthétique (RR3*),
o recevoir la troisième représentation de référence synthétique (RR3*) générée par le deuxième modèle partiel (TM2),
o quantifier les écarts entre la troisième représentation de référence synthétique (RR3*) et la troisième représentation de référence (RR3) des données d'apprentissage,
o réduire les écarts en modifiant des paramètres du premier modèle partiel (TM1),

- stocker le modèle ($M^T$) d'apprentissage automatique entraîné et/ou utiliser le modèle ($M^T$) d'apprentissage automatique entraîné pour générer une représentation synthétique (R3*) d'une zone d'examen d'un objet d'examen.

**10.** Procédé selon l'une quelconque des revendications 2 à 9, dans lequel la fonction de pondération (WF) dépendant de la fréquence mentionnée dans la revendication 2 est une fonction de Hann, une fonction de poisson ou une fonction gaussienne.

**11.** Procédé selon l'une quelconque des revendications 1 à 10, dans lequel l'objet d'examen est un humain et la zone d'examen est une partie de l'humain, chaque objet de référence étant un humain et la zone de référence de chaque objet de référence étant une partie de l'objet de référence, la zone de référence de chaque objet de référence et la zone d'examen étant respectivement la même partie de l'humain dans chaque cas.

**12.** Procédé selon l'une quelconque des revendications 1 à 11, dans lequel la première représentation (R1) et la deuxième représentation (R2) et chaque représentation de référence (RR1, RR2, RR3) de chaque objet de référence sont le résultat d'un examen

radiologique, l'examen radiologique est de préférence un examen IRM ou un examen CT et l'agent de contraste étant un agent de contraste IRM ou un agent de contraste CT.

**13.** Procédé selon l'une quelconque des revendications 1 à 12, dans lequel la première quantité est égale à zéro, la deuxième quantité est inférieure à la quantité standard de l'agent de contraste ou égale à la quantité standard de l'agent de contraste, et la troisième quantité est supérieure à la quantité standard de l'agent de contraste.

**14.** Procédé selon l'une quelconque des revendications 1 à 13, dans lequel l'agent de contraste comprend

- un complexe de $Gd^{3+}$ d'un composé de formule (I),

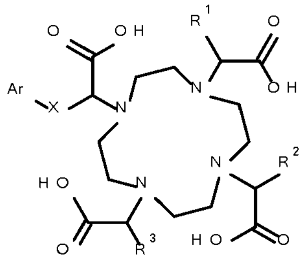

(I),

dans lequel

Ar représente un groupe choisi parmi

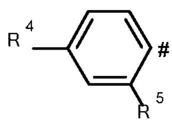

et

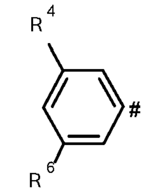

,

où # représente la liaison à X,
X représente un groupe choisi parmi $CH_2$, $(CH_2)_2$, $(CH_2)_3$, $(CH_2)_4$ et *-$(CH_2)_2$-O-$CH_2$-*,
où * représente la liaison à Ar et # représente la liaison au radical d'acide acétique,
$R^1$, $R^2$ et $R^3$ représentent, indépendamment les uns des autres, un atome d'hydrogène ou un groupe choisi parmi un alkyle en $C_1$-$C_3$, -$CH_2OH$, -$(CH_2)_2OH$ et -$CH_2OCH_3$,
$R^4$ représente un groupe choisi parmi un alcoxy en $C_2$-$C_4$, ($H_3C$-$CH_2$)-O-$(CH_2)_2$-O-, ($H_3C$-$CH_2$)-O-$(CH_2)_2$-O-$(CH_2)_2$-O- et ($H_3C$-$CH_2$)-O-$(CH_2)_2$-O-$(CH_2)_2$-O-$(CH_2)_2$-O-,
$R^5$ représente un atome d'hydrogène,
et
$R^6$ représente un atome d'hydrogène,
ou un stéréoisomère, un tautomère, un hydrate, un solvate ou un sel de celui-ci, ou un mélange de ceux-ci, ou

- un complexe de $Gd^{3+}$ d'un composé de formule (II),

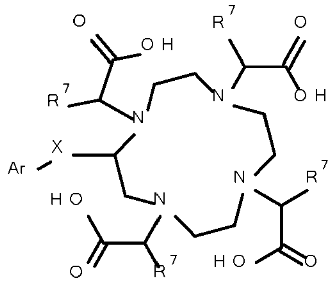

(II),

dans lequel

Ar représente un groupe choisi parmi

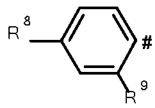

et

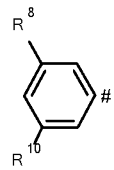

,

où # représente la liaison à X,
X représente un groupe choisi parmi $CH_2$, $(CH_2)_2$, $(CH_2)_3$, $(CH_2)_4$ et *-$(CH_2)_2$-O-$CH_2$-#, où * représente la liaison à Ar et # représente la liaison au radical d'acide acétique, $R^7$ représente un atome d'hydrogène ou un groupe choisi parmi un alkyle en $C_1$-$C_3$, -$CH_2OH$, -$(CH_2)_2OH$ et -$CH_2OCH_3$ ;
$R^8$ représente un groupe choisi parmi un alcoxy en $C_2$-$C_4$, ($H_3C$-$CH_2O$)-$(CH_2)_2$-O-, ($H_3C$-$CH_2O$)-$(CH_2)_2$-O-$(CH_2)_2$-O- et ($H_3C$-$CH_2O$)-$(CH_2)_2$-O-$(CH_2)_2$-O-$(CH_2)_2$-O- ;
$R^9$ et $R^{10}$ représentent, indépendamment l'un de l'autre, un atome d'hydrogène ;
ou un stéréoisomère, un tautomère, un hydrate, un solvate ou un sel de celui-ci, ou un mélange de ceux-ci, ou l'agent de contraste comprend l'une des substances suivantes :

- acide gadolinium(III) 2-[4,7,10-tris(carboxyméthyl)-1,4,7,10-tétrazacyclododéc-1-yl]acétique,
- acide gadolinium(III) éthoxybenzyl-diéthylènetriaminepentaacétique,

- 2-[3,9-bis[1-carboxylato-4-(2,3-dihydroxypropylamino)-4-oxobutyl]-3,6,9,15-tétrazabicyclo[9.3.1]pentadéca-1(15),11,13-trién-6-yl]-5-(2,3-dihydroxypropylamino)-5-oxopentanoate de gadolinium(III),
- dihydrogène[(±)-4-carboxy-5,8,11-tris(carboxyméthyl)-1-phényl-2-oxa-5,8,11-triazatridécan-13-oato(5-)] gadolinate(2-),
- acétate de tétragadolinium-[4,10-bis(carboxylatométhyl)-7-{3,6,12,15-tétraoxo-16-[4,7,10-tris-(carboxylatométhyl)-1,4,7,10-tétraazacyclododécan-1-yl]-9,9-bis({[({2-[4,7,10-tris-(carboxylatométhyl)-1,4,7,10-tétraazacyclododécan-1-yl]propanoyl}amino) acétyl]-amino}méthyl)-4,7,11,14-tétraazahepta-décan-2-yl}-1,4,7,10-tétraazacyclododécan-1-yle],
- triacétate de 2,2',2"-(10-{1-carboxy-2-[2-(4-éthoxyphényl)éthoxy] éthyl}-1,4,7,10-tétraazacyclododécan-1,4,7-triyle),
- 2,2',2"-{10-[1-carboxy-2-{4-[2-(2-éthoxyéthoxy)éthoxy]phényl} éthyl]-1,4,7,10-tétraazacyclododécan-1,4,7-triyl}triacétate de gadolinium,
- 2,2',2"-{10-[(1R)-1-carboxy-2-{4-[2-(2-éthoxyéthoxy)éthoxy] phényl}éthyl]-1,4,7,10-tétraazacyclododécan-1,4,7-triyl}triacétate de gadolinium,
- (2S,2'S,2"S)-2,2',2"-{10-[(1S)-1-carboxy-4-{4-[2-(2-éthoxyéthoxy)éthoxy] phényl}butyl]-1,4,7,10-tétraazacyclododécan-1,4,7-triyl}tris(3-hydroxypropanoate) de gadolinium,
- 2,2',2"-{10-[(1S)-4-(4-butoxyphényl)-1-carboxybutyl]-1,4,7,10-tétraazacyclododécan-1,4,7-triyl}triacétate de gadolinium,
- 5,8-bis(carboxylatométhyl)-2-[2-(méthylamino)-2-oxoéthyl]-10-oxo-2,5,8,11-tétraazadodécan-1-carboxylate-hydrate de gadolinium (III),
- 2-[4-(2-hydroxypropyl)-7,10-bis(2-oxido-2-oxoéthyl)-1,4,7,10-tétrazacyclododéc-1-yl]acétate de gadolinium (III),
- 2,2',2"-(10-((2R,3S)-1,3,4-trihydroxybutan-2-yl)-1,4,7,10-tétraazacyclododécan-1,4,7-triyl)triacétate de gadolinium (III),
- triacétate de gadolinium-2,2',2"-{(2S)-10-(carboxyméthyl)-2-[4-(2-éthoxyéthoxy)benzyl]-1,4,7,10-tétraazacyclododécane-1,4,7-triyle},
- triacétate de gadolinium-2,2',2"-[10-(carboxyméthyl)-2-(4-éthoxybenzyl)-1,4,7,10-tétraazacyclododécane-1,4,7-triyle].

**15.** Système informatique (1) comprenant

• une unité de réception (11),
• une unité de commande et de calcul (12), et
• une unité de sortie (13),

l'unité de commande et de calcul (12) étant configurée pour

- générer une première représentation (R1) ou amener l'unité de réception (11) à recevoir une première représentation (R1), la première représentation (R1) représentant une zone d'examen d'un objet d'examen sans agent de contraste ou après l'application d'une première quantité d'un agent de contraste,
- générer une deuxième représentation (R2) ou amener l'unité de réception (11) à recevoir la deuxième représentation (R2), la deuxième représentation (R2) représentant la zone d'examen de l'objet d'examen après l'application d'une deuxième quantité de l'agent de contraste, la deuxième quantité étant supérieure à la première quantité,
- appliquer la première représentation (R1) et la deuxième représentation (R2) à un modèle ($M^T$) d'apprentissage automatique entraîné

o le modèle ($M^T$) d'apprentissage automatique entraîné ayant été entraîné sur la base de données d'apprentissage (TD),
o les données d'apprentissage (TD), pour chaque objet de référence d'une pluralité d'objets de référence comprenant (i) une première représentation de référence (RR1) d'une zone de référence de l'objet de référence et une deuxième représentation de référence (RR2) de la zone de référence de l'objet de référence en tant que données d'entrée et (ii) une troisième représentation de référence (RR3) de la zone de référence de l'objet de référence en tant que données cibles,
o la première représentation de référence (RR1) représentant la zone de référence sans agent de contraste ou après l'application d'une première quantité d'un agent de contraste,

o la deuxième représentation de référence (RR2) représentant la zone de référence après l'application d'une deuxième quantité de l'agent de contraste, la deuxième quantité étant supérieure à la première quantité,
o la troisième représentation de référence (RR3) représentant la zone de référence après l'application d'une troisième quantité de l'agent de contraste, la troisième quantité étant différente de la deuxième quantité, de préférence supérieure à la deuxième quantité,
o le modèle ($M^T$) d'apprentissage automatique entraîné comprenant deux modèles partiels : un premier modèle partiel ($TM1^T$) et un deuxième modèle partiel (TM2), le premier modèle partiel ($TM1^T$) étant configuré et ayant été entraîné pour déterminer, sur la base de la première représentation de référence (RR1) et de la deuxième représentation de référence (RR2) de chaque objet de référence, au moins un paramètre de modèle (MP) pour le deuxième modèle partiel (TM2), le deuxième modèle partiel (TM2) étant configuré pour générer, sur la base de la première représentation de référence (RR1) et de la deuxième représentation de référence (RR2) et dudit au moins un paramètre de modèle (MP) déterminé par le premier modèle partiel ($TM1^T$), une troisième représentation de référence synthétique (RR3*),

- recevoir une troisième représentation synthétique (R3*) de la zone d'examen de l'objet d'examen en provenance du modèle ($M^T$) d'apprentissage automatique entraîné, la troisième représentation synthétique (R3*) représentant la zone de référence après l'application d'une troisième quantité du produit de contraste, la troisième quantité étant différente de la deuxième quantité, de préférence supérieure à la deuxième quantité,
- amener l'unité de sortie (13) à délivrer la troisième représentation synthétique (R3*) et/ou à la stocker dans une mémoire de données et/ou à la transférer à un système informatique séparé.

**caractérisé en ce que** le deuxième modèle partiel (TM2) est configuré pour

- soustraire la première représentation (R1) de la zone d'examen de l'objet examen à la deuxième représentation (R2) de la zone d'examen de l'objet examen, une différence (R2-R1) étant calculée,
- multiplier la différence (R2-R1) par un facteur d'amplification ($\alpha$), le facteur d'amplification ($\alpha$) étant un nombre réel positif ou négatif, ledit au moins un paramètre de modèle (MP) déterminé par le premier modèle partiel ($TM1^T$) comprenant le facteur d'amplification ($\alpha$),
- additionner la différence (R2-R1) multipliée par le facteur d'amplification ($\alpha$) à la première représentation (R1) ou à la deuxième représentation (R2).

**16.** Produit de programme informatique, comprenant un support de données sur lequel est stocké un programme informatique (40), le programme informatique (40) pouvant être chargé dans une mémoire vive (22) d'un système informatique (1) et y amenant le système informatique (1) à exécuter les étapes suivantes consistant à :

- fournir un modèle ($M^T$) d'apprentissage automatique entraîné ,

o le modèle ($M^T$) d'apprentissage automatique entraîné ayant été entraîné sur la base de données d'apprentissage (TD),
o les données d'apprentissage (TD), pour chaque objet de référence d'une pluralité d'objets de référence comprenant (i) une première représentation de référence (RR1) d'une zone de référence de l'objet de référence et une deuxième représentation de référence (RR2) de la zone de référence de l'objet de référence en tant que données d'entrée et (ii) une troisième représentation de référence (RR3) de la zone de référence de l'objet de référence en tant que données cibles,
o la première représentation de référence (RR1) représentant la zone de référence sans agent de contraste ou après l'application d'une première quantité d'un agent de contraste,
o la deuxième représentation de référence (RR2) représentant la zone de référence après l'application d'une deuxième quantité de l'agent de contraste, la deuxième quantité étant supérieure à la première quantité,
o la troisième représentation de référence (RR3) représentant la zone de référence après l'application d'une troisième quantité de l'agent de contraste, la troisième quantité étant différente de la deuxième quantité, de préférence supérieure à la deuxième quantité,

o le modèle ($M^T$) d'apprentissage automatique entraîné comprenant deux modèles partiels : un premier modèle partiel ($TM1^T$) et un deuxième modèle partiel (TM2), le premier modèle partiel ($TM1^T$) étant configuré et ayant été entraîné pour déterminer, sur la base de la première représentation de référence (RR1) et de la deuxième représentation de référence (RR2) de la zone d'examen d'un objet de référence, au moins un paramètre de modèle (MP) pour le deuxième modèle partiel (TM2), le deuxième modèle partiel (TM2) étant configuré pour générer, sur la base de la première représentation de référence (RR1) et de la deuxième représentation de référence (RR2) et dudit au moins un paramètre de modèle (MP) déterminé par le premier modèle partiel ($TM1^T$), une troisième représentation de référence synthétique (RR3*),

- fournir une première représentation (R1), la première représentation (R1) représentant une zone d'examen d'un objet d'examen sans agent de contraste ou après l'application d'une première quantité d'un agent de contraste,
- fournir une deuxième représentation (R2), la deuxième représentation (R2) représentant la zone d'examen de l'objet d'examen après l'application d'une deuxième quantité de l'agent de contraste, la deuxième quantité étant supérieure à la première quantité,
- appliquer la première représentation (R1) et la deuxième représentation (R2) au modèle ($M^T$) d'apprentissage automatique entraîné,
- recevoir une troisième représentation synthétique (R3*) de la zone d'examen de l'objet d'examen en provenance du modèle ($M^T$) d'apprentissage automatique entraîné, la troisième représentation synthétique (R3*) représentant la zone de référence après l'application d'une troisième quantité du produit de contraste, la troisième quantité étant différente de la deuxième quantité, de préférence supérieure à la deuxième quantité,
- délivrer et/ou stocker la troisième représentation synthétique (R3*) et/ou transférer la troisième représentation synthétique (R3*) à un système informatique séparé,

**caractérisé en ce que** le deuxième modèle partiel (TM2) est configuré pour

- soustraire la première représentation (R1) de la zone d'examen de l'objet examen à la deuxième représentation (R2) de la zone d'examen de l'objet examen, une différence (R2-R1) étant calculée,
- multiplier la différence (R2-R1) par un facteur d'amplification ($\alpha$), le facteur d'amplification ($\alpha$) étant un nombre réel positif ou négatif, ledit au moins un paramètre de modèle (MP) déterminé par le premier modèle partiel ($TM1^T$) comprenant le facteur d'amplification ($\alpha$),
- additionner la différence (R2-R1) multipliée par le facteur d'amplification ($\alpha$) à la première représentation (R1) ou à la deuxième représentation (R2).

**17.** Utilisation d'un agent de contraste dans un procédé d'examen radiologique comprenant les étapes consistant à :

- fournir un modèle ($M^T$) d'apprentissage automatique entraîné ,

o le modèle ($M^T$) d'apprentissage automatique entraîné ayant été entraîné sur la base de données d'apprentissage (TD),
o les données d'apprentissage (TD), pour chaque objet de référence d'une pluralité d'objets de référence comprenant (i) une première représentation de référence (RR1) d'une zone de référence de l'objet de référence et une deuxième représentation de référence (RR2) de la zone de référence de l'objet de référence en tant que données d'entrée et (ii) une troisième représentation de référence (RR3) de la zone de référence de l'objet de référence en tant que données cibles,
o la première représentation de référence (RR1) représentant la zone de référence sans agent de contraste ou après l'application d'une première quantité de l'agent de contraste,
o la deuxième représentation de référence (RR2) représentant la zone de référence après l'application d'une deuxième quantité de l'agent de contraste, la deuxième quantité étant supérieure à la première quantité,
o la troisième représentation de référence (RR3) représentant la zone de référence après l'application d'une troisième quantité de l'agent de contraste, la troisième quantité étant différente de la deuxième quantité, de préférence supérieure à la deuxième quantité,
o le modèle ($M^T$) d'apprentissage automatique entraîné comprenant deux modèles partiels : un premier modèle partiel ($TM1^T$) et un deuxième modèle partiel (TM2), le premier modèle partiel ($TM1^T$) étant configuré et ayant été entraîné pour déterminer, sur la base de la première représentation de référence (RR1) et de la deuxième représentation de référence (RR2) de la zone d'examen d'un objet de référence, au moins un paramètre de modèle (MP) pour le deuxième modèle partiel (TM2), le deuxième modèle partiel (TM2) étant configuré pour générer, sur la base de la première représentation de référence (RR1) et de la deuxième représentation de référence (RR2) et dudit au moins un paramètre de modèle (MP) déterminé par le premier modèle partiel ($TM1^T$), une troisième représentation de référence synthétique (RR3*),

- fournir une première représentation (R1), la première représentation (R1) représentant une zone d'examen d'un objet d'examen sans agent de contraste ou après l'application d'une première quantité d'un agent de contraste,
- fournir une deuxième représentation (R2), la deuxième représentation (R2) représentant la zone d'examen de l'objet d'examen après l'application d'une deuxième quantité de l'agent de contraste, la deuxième quantité étant supérieure à la première quantité,
- appliquer la première représentation (R1) et la deuxième représentation (R2) au modèle ($M^T$) d'apprentissage automatique entraîné,
- recevoir une troisième représentation synthétique (R3*) de la zone d'examen de l'objet d'examen en provenance du modèle ($M^T$) d'apprentissage automatique entraîné, la troisième représentation synthétique (R3*) représentant la zone de référence après l'application d'une troisième quantité du produit de contraste, la troisième quantité étant différente de la deuxième quantité, de préférence supérieure à la deuxième quantité,
- délivrer et/ou stocker la troisième représentation synthétique (R3*) et/ou transférer la troisième représentation synthétique (R3*) à un système informatique séparé,

**caractérisé en ce que** le deuxième modèle partiel (TM2) est configuré pour

- soustraire la première représentation (R1) de la zone d'examen de l'objet examen à la deuxième représentation (R2) de la zone d'examen de l'objet examen, une différence (R2-R1) étant calculée,
- multiplier la différence (R2-R1) par un facteur d'amplification ($\alpha$), le facteur d'amplification ($\alpha$) étant un nombre réel positif ou négatif, ledit au moins un paramètre de modèle (MP) déterminé par le premier modèle partiel ($TM1^T$) comprenant le facteur d'amplification ($\alpha$),
- additionner la différence (R2-R1) multipliée par le facteur d'amplification ($\alpha$) à la première représentation (R1) ou à la deuxième représentation (R2).

**18.** Kit, comprenant un produit de programme informatique selon la revendication 16 et un agent de contraste, l'agent de contraste comprenant de préférence

- un complexe de $Gd^{3+}$ d'un composé de formule (I),

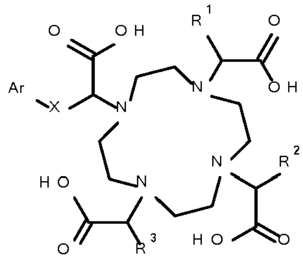

(I),

dans lequel

Ar représente un groupe choisi parmi

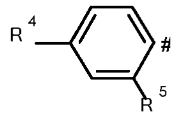

et

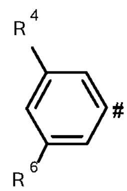

,

où # représente la liaison à X,
X représente un groupe choisi parmi $CH_2$, $(CH_2)_2$, $(CH_2)_3$, $(CH_2)_4$ et *-$(CH_2)_2$-O-$CH_2$-#,
où * représente la liaison à Ar et # représente la liaison au radical d'acide acétique,
$R^1$, $R^2$ et $R^3$ représentent, indépendamment les uns des autres, un atome d'hydrogène ou un groupe choisi parmi un alkyle en $C_1$-$C_3$, -$CH_2OH$, -$(CH_2)_2OH$ et -$CH_2OCH_3$,
$R^4$ représente un groupe choisi parmi un alcoxy en $C_2$-$C_4$, $(H_3C\text{-}CH_2)$-O-$(CH_2)_2$-O-, $(H_3C\text{-}CH_2)$-O-$(CH_2)_2$-O-$(CH_2)_2$-O- et $(H_3C\text{-}CH_2)$-O-$(CH_2)_2$-O-$(CH_2)_2$-O-$(CH_2)_2$-O-,
$R^5$ représente un atome d'hydrogène,
et
$R^6$ représente un atome d'hydrogène,
ou un stéréoisomère, un tautomère, un hydrate, un solvate ou un sel de celui-ci, ou un mélange de ceux-ci, ou

- un complexe de $Gd^{3+}$ d'un composé de formule (II),

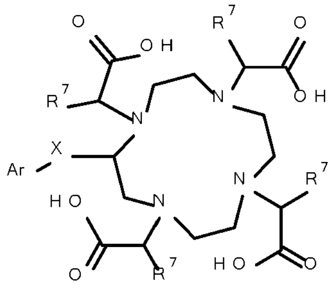

(II),

dans lequel

Ar représente un groupe choisi parmi

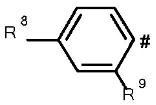

et

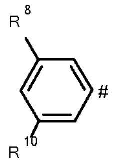

,

où # représente la liaison à X,
X représente un groupe choisi parmi $CH_2$, $(CH_2)_2$, $(CH_2)_3$, $(CH_2)_4$ et $*-(CH_2)_2-O-CH_2-^{\#}$, où * représente la liaison à Ar et # représente la liaison au radical d'acide acétique, $R^7$ représente un atome d'hydrogène ou un groupe choisi parmi un alkyle en $C_1$-$C_3$, $-CH_2OH$, $-(CH_2)_2OH$ et $-CH_2OCH_3$ ;
$R^8$ représente un groupe choisi parmi un alcoxy en $C_2$-$C_4$, $(H_3C-CH_2O)-(CH_2)_2-O-$, $(H_3C-CH_2O)-(CH_2)_2-O-(CH_2)_2-O-$ et $(H_3C-CH_2O)-(CH_2)_2-O-(CH_2)_2-O-(CH_2)_2-O-$ ;
$R^9$ et $R^{10}$ représentent, indépendamment l'un de l'autre, un atome d'hydrogène ;
ou un stéréoisomère, un tautomère, un hydrate, un solvate ou un sel de celui-ci, ou un mélange de ceux-ci, ou

- l'agent de contraste comprend l'une des substances suivantes :
- acide gadolinium(III) 2-[4,7,10-tris(carboxyméthyl)-1,4,7,10-tétrazacyclododéc-1-yl]acétique,
- acide gadolinium(III) éthoxybenzyl-diéthylènetriaminepentaacétique,
- 2-[3,9-bis[1-carboxylato-4-(2,3-dihydroxypropylamino)-4-oxobutyl]-3,6,9,15-tétrazabicyclo [9.3.1]pentadéca-1(15),11,13-trién-6-yl]-5-(2,3-dihydroxypropylamino)-5-oxopentanoate de gadolinium(III),
- dihydrogène[(±)-4-carboxy-5,8,11-tris(carboxyméthyl)-1-phényl-2-oxa-5,8,11-triazatridécan-13-oato(5-)]gadolinate(2-),
- acétate de tétragadolinium-[4,10-bis(carboxylatométhyl)-7-{3,6,12,15-tétraoxo-16-[4,7,10-tris-(carboxylatométhyl)-1,4,7,10-tétraazacyclododécan-1-yl]-9,9-bis({[({2-[4,7,10-tris-(carboxylatométhyl)-1,4,7,10-tétraazacyclododécan-1-yl]propanoyl}amino)acétyl]-amino}méthyl)-4,7,11,14-tétraazahepta-décan-2-yl}-1,4,7,10-tétraazacyclododécan-1-yle],
- triacétate de 2,2',2"-(10-{1-carboxy-2-[2-(4-éthoxyphényl)éthoxy]éthyl}-1,4,7,10-tétraazacyclododécan-1,4,7-triyle),
- 2,2',2"-{10-[1-carboxy-2-{4-[2-(2-éthoxyéthoxy)éthoxy]phényl}éthyl]-1,4,7,10-tétraazacyclododécan-1,4,7-triyl}triacétate de gadolinium,
- 2,2',2"-{10-[(1R)-1-carboxy-2-{4-[2-(2-éthoxyéthoxy)éthoxy]phényl}éthyl]-1,4,7,10-tétraazacyclododécan-1,4,7-triyl}triacétate de gadolinium,
- (2S,2'S,2"S)-2,2',2"-{10-[(1S)-1-carboxy-4-{4-[2-(2-éthoxyéthoxy)éthoxy] phényl}butyl]-1,4,7,10-tétraazacyclododécan-1,4,7-triyl} tris(3-hydroxypropanoate) de gadolinium,
- 2,2',2"-{10-[(1S)-4-(4-butoxyphenyl)-1-carboxybutyl]-1,4,7,10-tétraazacyclododécan-1,4,7-triyl}triacétate de gadolinium,
- 5,8-bis(carboxylatométhyl)-2-[2-(méthylamino)-2-oxoéthyl]-10-oxo-2,5,8,11-tétraazadodécan-1-carboxylate-hydrate de gadolinium (III),
- 2-[4-(2-hydroxypropyl)-7,10-bis(2-oxido-2-oxoéthyl)-1,4,7,10-tétrazacyclododéc-1-yl]acétate de gadolinium (III),
- 2,2',2"-(10-((2R,3S)-1,3,4-trihydroxybutan-2-yl)-1,4,7,10-tétraazacyclododécan-1,4,7-triyl) triacétate de gadolinium (III),
- triacétate de gadolinium-2,2',2"-{(2S)-10-(carboxyméthyl)-2-[4-(2-éthoxyéthoxy)benzyl]-1,4,7,10-tétraazacyclododécane-1,4,7-triyle},
- triacétate de gadolinium-2,2',2"-[10-(carboxyméthyl)-2-(4-éthoxybenzyl)-1,4,7,10-tétraazacyclododécane-1,4,7-triyle].

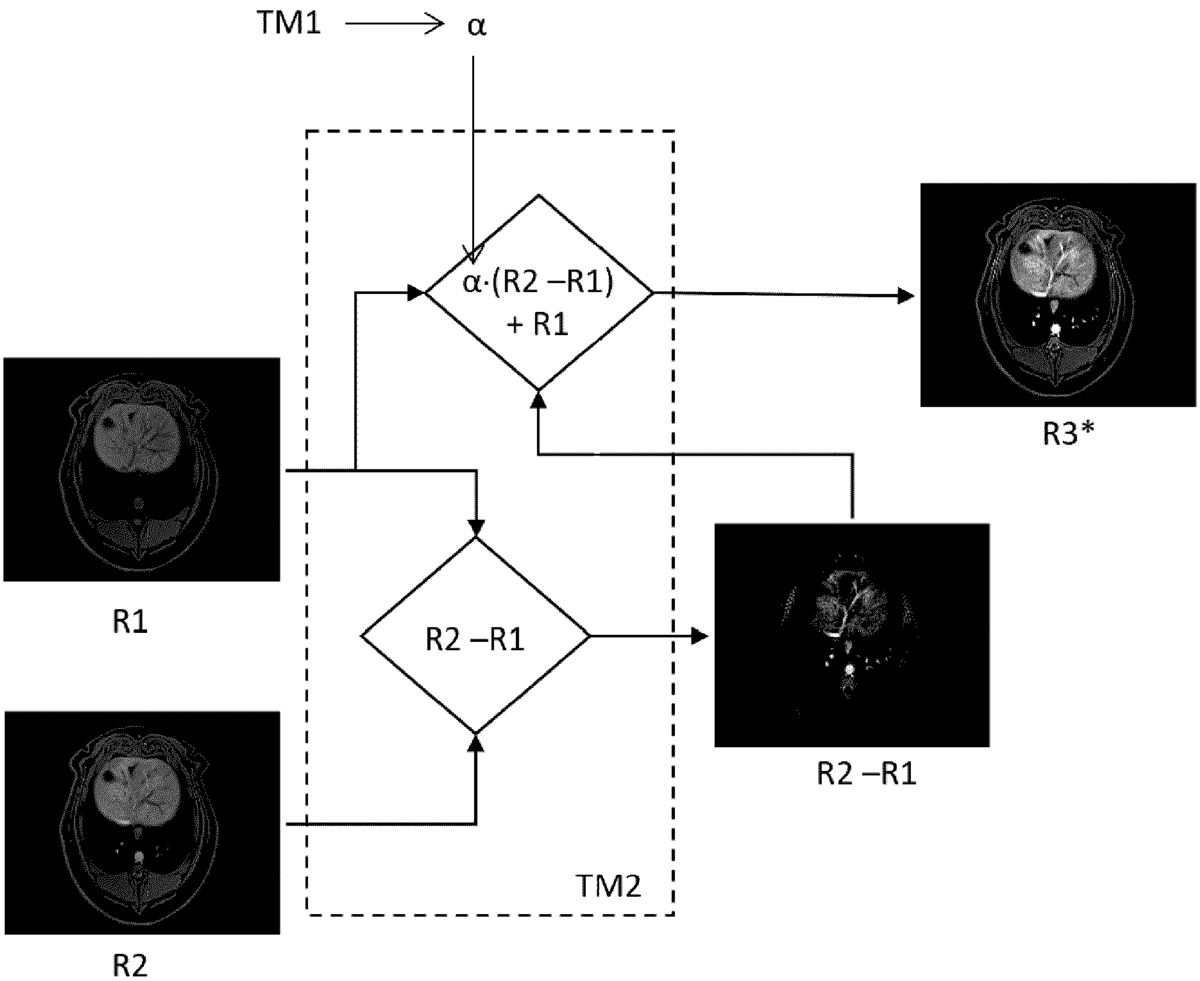


Fig. 1

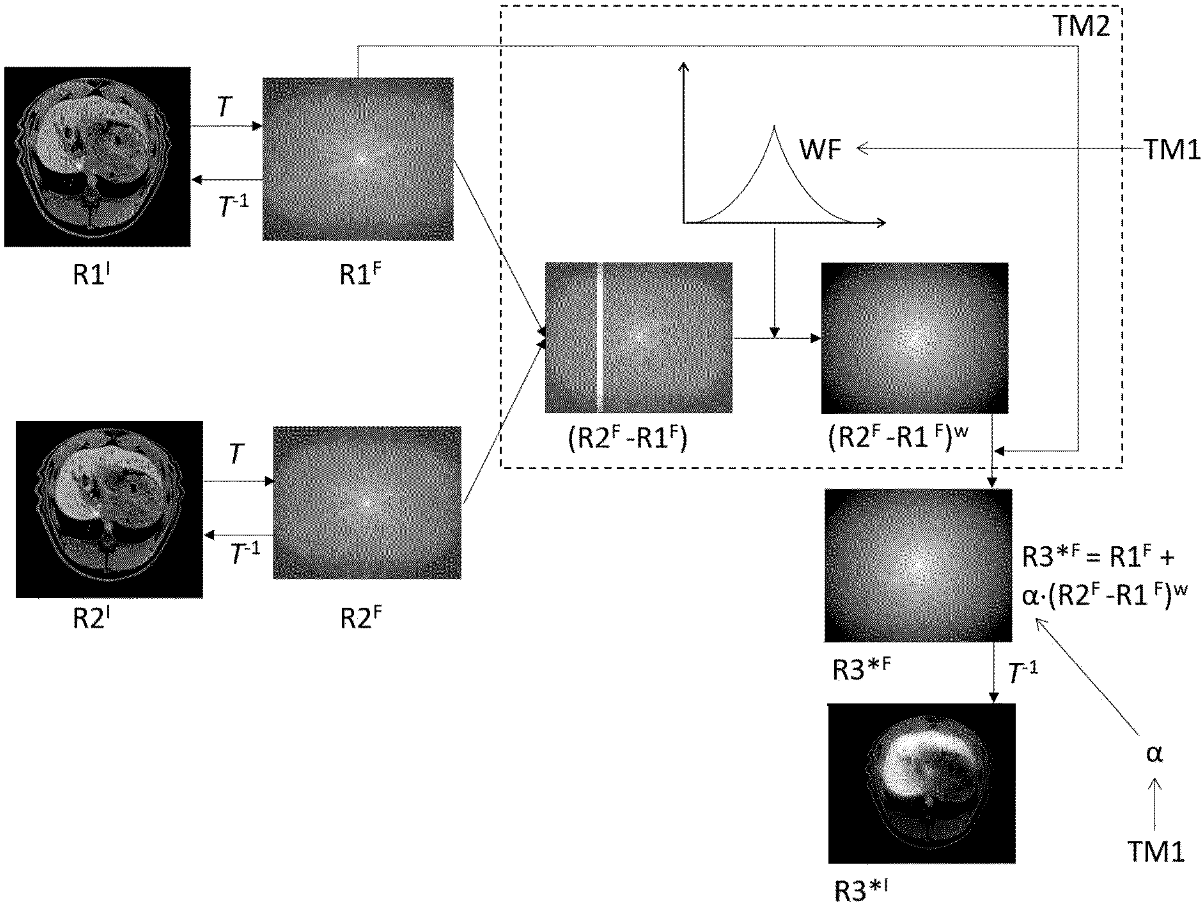
TM2
T
$T^{-1}$
WF
TM1
$R1^I$
$R1^F$
$(R2^F - R1^F)$
$(R2^F - R1^F)^w$
T
$T^{-1}$
$R2^I$
$R2^F$
$R3^{*F} = R1^F + \alpha \cdot (R2^F - R1^F)^w$
$R3^{*F}$
$T^{-1}$
α
TM1
$R3^{*I}$

Fig. 2

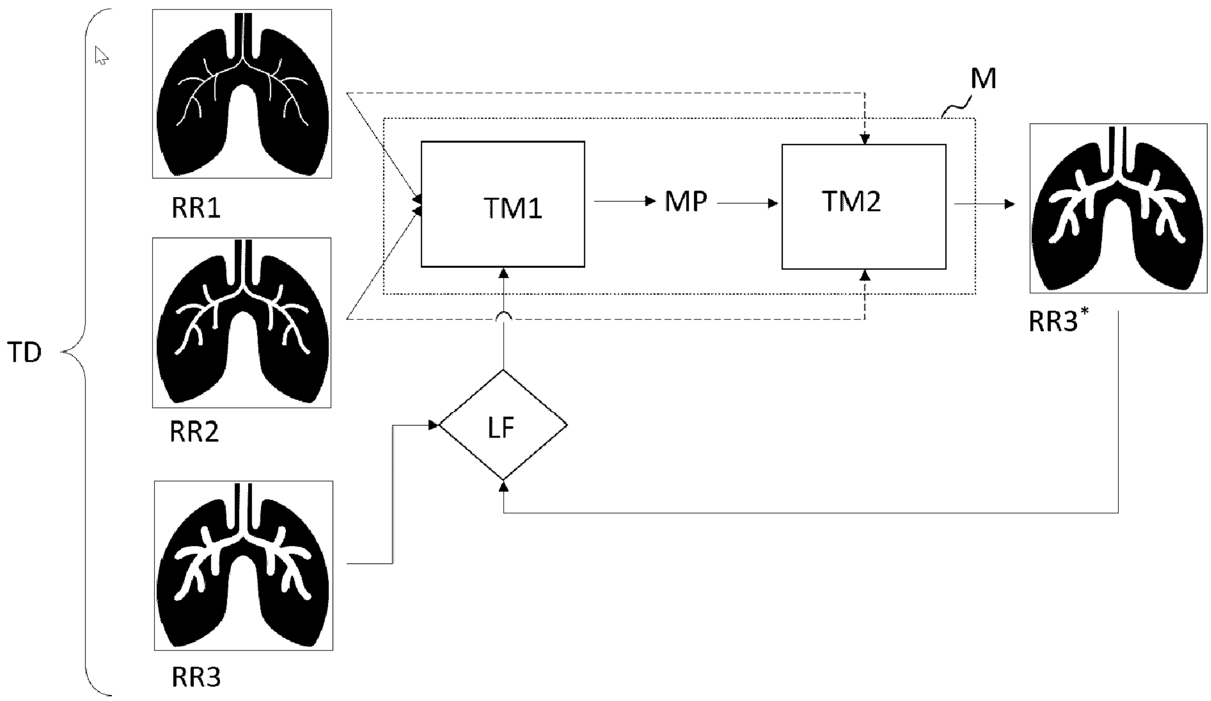
M
RR1
TM1
MP
TM2
RR3*
TD
RR2
LF
RR3

Fig. 3

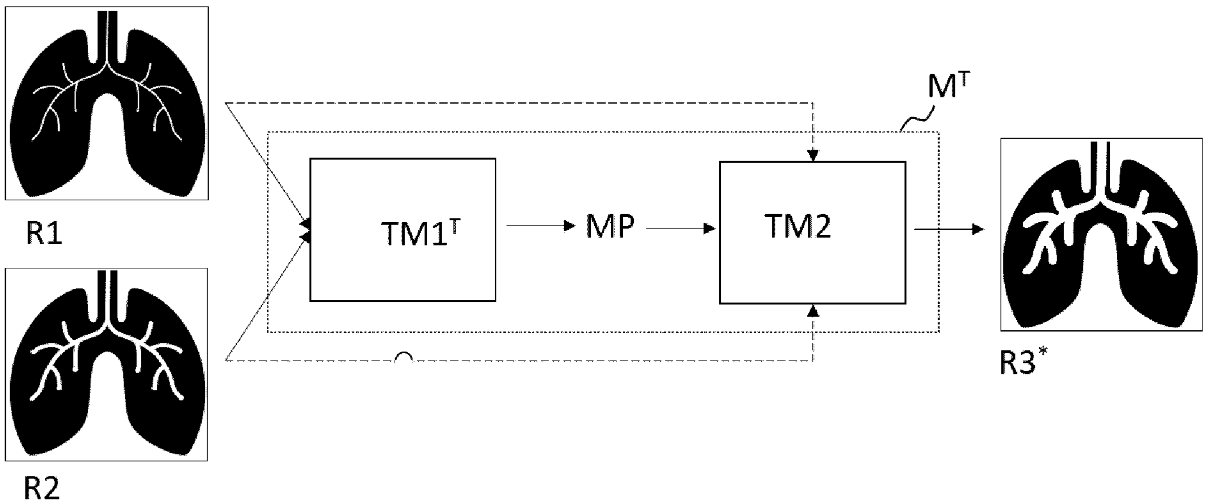


Fig. 4

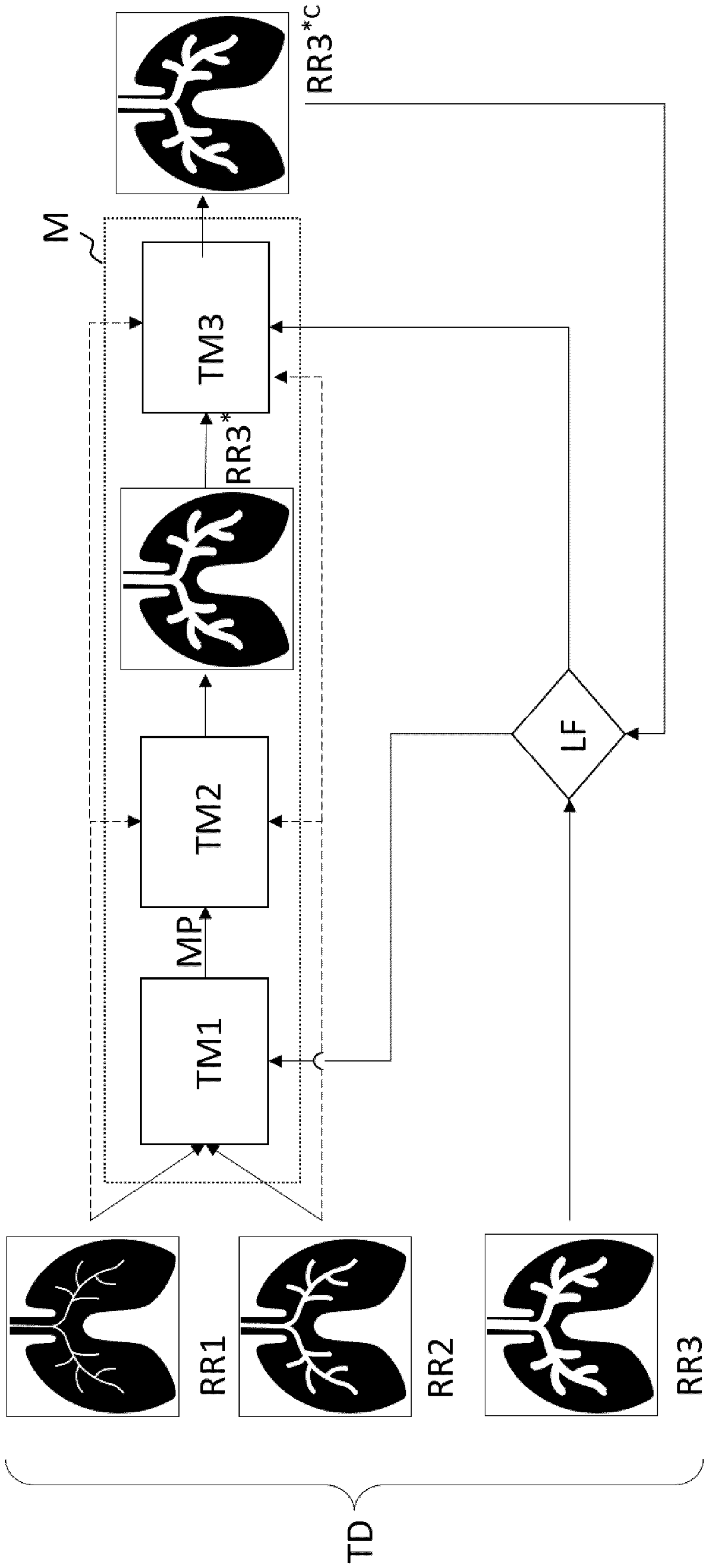


Fig. 5

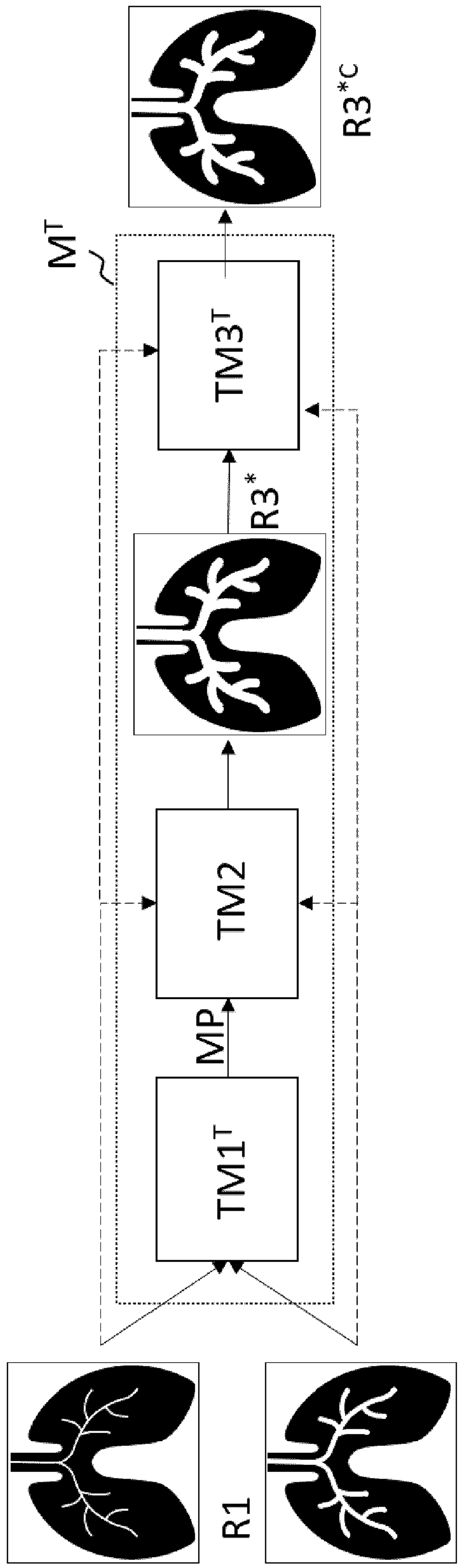


Fig. 6

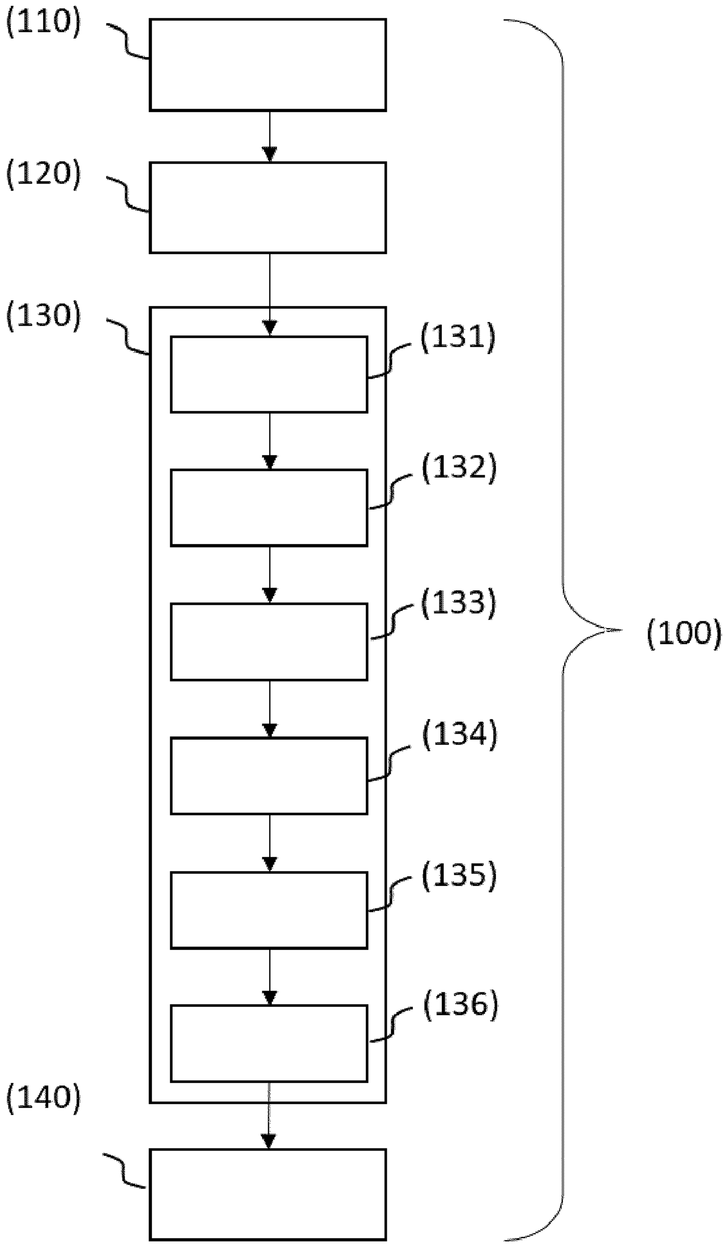


Fig. 7

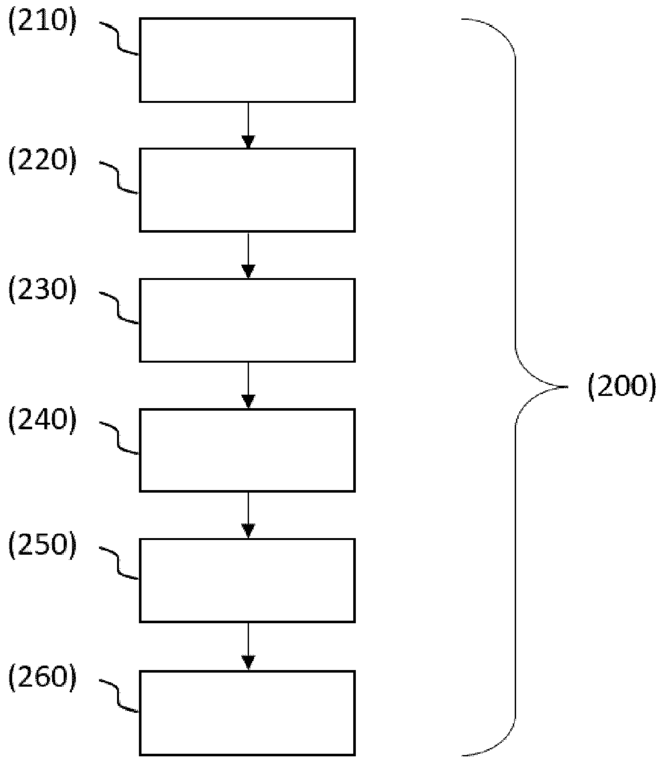


Fig. 8

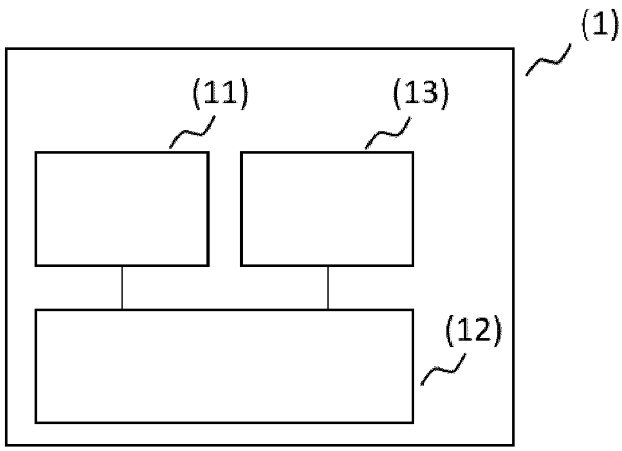


Fig. 9

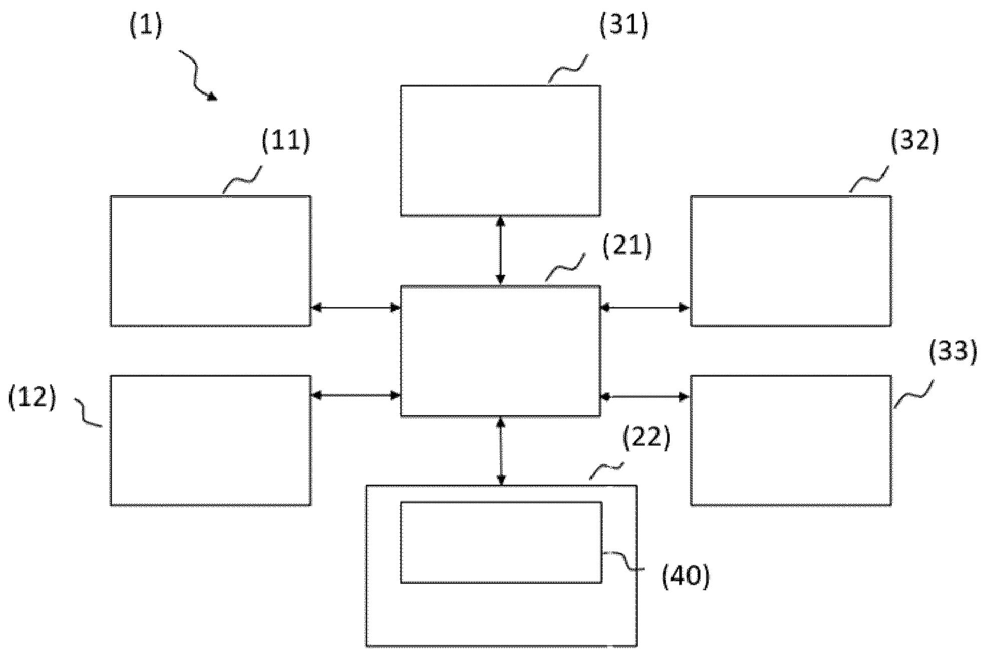


Fig. 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2019074938 A1
- US 20190108634 A1
- WO 2007042504 A
- WO 2020030618 A
- WO 2022013454 A
- WO 2016193190 A
- WO 2022194777 A
- US 6039931 A
- EP 22207079
- EP 22207080
- EP 23168725


### In der Beschreibung aufgeführte Nicht-Patentliteratur


- **JASCINTH et al.** ontrast Agents in computed tomography: A Review. *Journal of Applied Dental and Medical Sciences*, 2016, vol. 2 (2), 143-149
- **H. LUSIC et al.** X-ray-Computed Tomography Contrast Agents. *Chem. Rev.*, 2013, vol. 113 (3), 1641-1666, https://www.radiology.wisc.edu/wp-content/uploads/2017/10/contrast-agents-tutorial.pdf
- **M. R. NOUH et al.** Radiographie and magnetic resonances contrast agents: Essentials and tips for safe practices. *World J Radiol*, 28 September 2017, vol. 9 (9), 339-349
- **L. C. ABONYI et al.** Intravascular Contrast Media in Radiography: Historical Development & Review of Risk Factors for Adverse Reactions. *South American Journal of Clinical Research*, 2016, vol. 3 (1), 1-10
- Ultrasound contrast agents, Endosc Ultrasound. **A. IGNEE et al.** ACR Manual on Contrast Media, 2020. November 2016, vol. 5, 355-362
- **J. LOHRKE et al.** Preclinical Profile of Gadoquatrane: A Novel Tetramerie, Macrocyclic High Relaxivity Gadolinium-Based Contrast Agent.. *Invest Radiol.*, 2022, vol. 57 (10), 629-638
- **B. K. FANG**. The Radon Transformation and Its Application in Tomography. *Journal of Physics Conference Series*, vol. 1903 (1), 012066
- **A.S. HASSANEIN et al.** A Survey on Hough Transform, Theory, Techniques and Applications. *arXiv:1502.02160v1*
- **F.J. HARRIS et al.** On the Use of Windows for Harmonic Analysis with the Discrete Fourier Transform. *Proceedings oft he IEEE*, vol. 66 (1), 1978, https://docs.scipy.org/doc/scipy/reference/signal.windows.html
- **K. M. M PRABHU**. Window Functions and Their Applications in Signal Processing. CRC Press, 2014
- **S. SKARE**. Rigid Body Image Realignment in Image Space vs. k-Space. *ISMRM SCIENTIFIC WORKSHOP on Motion Correction*, 2014, https://cds.ismrm.org/protected/Motion_14/Program/Syllabus/Skare.pdf
- U-net: Convolutional networks for biomedical image segmentation. **O. RONNEBERGER et al.** International Conference on Medical image computing and computer-assisted intervention. Springer, 2015, 234-241
- **M.-Y. LIU et al.** Generative Adversarial Networks for Image and Video Synthesis: Algorithms and Applications. *arXiv:2008.02793*
- **HENRY et al.** *Pix2Pix GAN for Image-to-Image Translation*
- **LEDIG et al.** Photo-Realistic Single Image Super-Resolution Using a Generative Adversarial Network. *arXiv:1609.04802v5*
- **B. D. KARIMI et al.** Convolution-Free Medical Image Segmentation using Transformers. *arXiv:2102.13645 [eess.IV*
- **M. HARTMANN et al.** *Does the administration of a high dose of a paramagnetic contrast medium (Gadovist) improve the diagnostic value of magnetic resonance tomography in glioblastomas*